(19) Europäisches Patentamt
European Patent Office
Office européen des brevets

(11) **EP 4 775 690 A2**

(12) **EUROPEAN PATENT APPLICATION**

(43) Date of publication:
**15.07.2026 Bulletin 2026/29**

(21) Application number: **26165385.1**

(22) Date of filing: **16.05.2019**

(51) International Patent Classification (IPC):
***C12Q 1/6876*** (2018.01)

(52) Cooperative Patent Classification (CPC):
(C-Sets available)
**C12Q 1/6858; C12Q 1/6876;** C12Q 2600/172
(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2018 US 201862672573 P**
**27.02.2019 US 201962811517 P**

(62) Document number(s) of the earlier application(s) in accordance with Art. 76 EPC:
**19804416.6 / 3 794 120**

(71) Applicant: **Twinstrand Biosciences, Inc.**
**Seattle, WA 98121 (US)**

(72) Inventors:
• **SALK, Jesse, J.**
**Seattle, WA 98125 (US)**
• **VALENTINE, Charles, Clinton, III**
**Seattle, WA 98122 (US)**

• **DANAHER, Patrick**
**Seattle, WA 98115 (US)**
• **LO, Fang, Yin**
**Seattle, WA 98177 (US)**

(74) Representative: **Haseltine Lake Kempner LLP**
**One Portwall Square**
**Portwall Lane**
**Bristol BS1 6BH (GB)**

Remarks:
•This application was filed on 17-03-2026 as a divisional application to the application mentioned under INID code 62.
•Claims filed after the date of filing of the application/ after the date of receipt of the divisional application (Rule 68(4) EPC).

(54) **METHODS AND REAGENTS FOR RESOLVING NUCLEIC ACID MIXTURES AND MIXED CELL POPULATIONS AND ASSOCIATED APPLICATIONS**

(57)    Methods and associated reagents for assessing and resolving nucleic acid mixtures and/or mixed cell populations are disclosed herein. Some embodiments of the technology are directed to utilizing Duplex Sequencing for assessing and resolving nucleic acid mixtures (e.g., multichimeric mixtures, mixtures of nucleic acids from more than one source, etc.) in a sample and associated applications. Other embodiments are directed to detecting and quantifying a donor source of nucleic acid from a mixture.

EP 4 775 690 A2

(52) Cooperative Patent Classification (CPC): (Cont.)

C-Sets
**C12Q 1/6858, C12Q 2525/191, C12Q 2535/122, C12Q 2537/165**

**Description**

**CROSS-REFERENCE TO RELATED APPLICATIONS**

This application claims priority to and the benefit of U.S. Provisional Patent Application No. 62/672,573, filed May 16, 2018, and U.S. Provisional Patent Application No. 62/811,517, filed February 27, 2019, the disclosures of which are hereby incorporated by reference in their entirety.

**BACKGROUND**

Resolving mixed cell populations derived from different clones or individuals, or tracking original sources within nucleic acid mixtures, often requires tracking specific genetic markers that differ between the clones or individuals that contributed to the mixtures. While it is sometimes possible to distinguish cells from different clones or individuals by non-genetic means (i.e. differences in proteins expressed on the cell surface, etc.), this is not always possible or may be experimentally impractical for high-throughput use. Genetic polymorphism can be used as a convenient, predictable and statistically generalizable lineage marker for defining a cell or DNA molecule's origin. In humans, for example, approximately 0.1% of the human genome is polymorphic (e.g., one out of every 1000 nucleotide bases varies in sequence within the human population). Common forms of variation can include single nucleotide polymorphisms/single nucleotide variants (SNPs/SNVs), multinucleotide variations (MNVs), short insertions and deletions (indels), variations in the length of short tandem repeats (STRs), as well as other larger scale structural variations such as inter- or intra-chromosomal rearrangements, duplications, deletions, tandem duplications and inversions, among others.

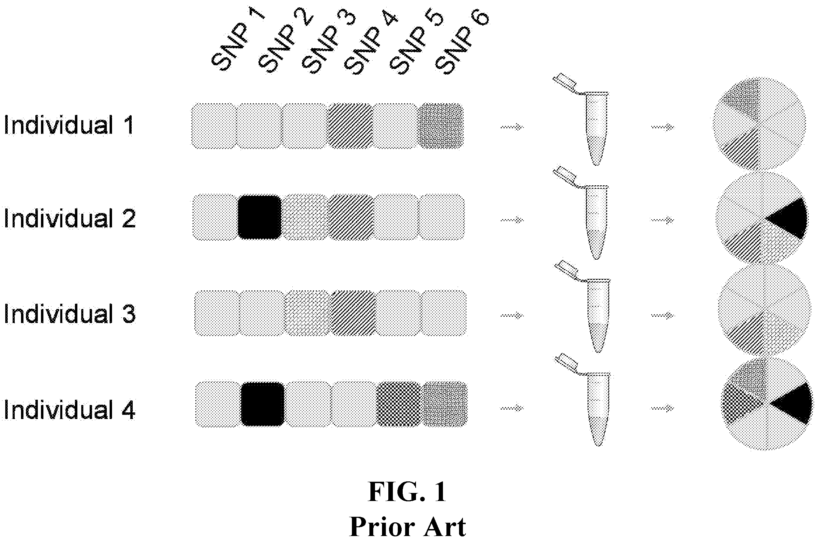

In general, when individuals are genotyped, the respective identities of each individual can be distinguished by resolving these polymorphic differences in genotypes. When using short-read next generation DNA sequencing (NGS) platforms for genotyping, SNPs are among the most abundant and convenient forms of polymorphisms for distinguishing different individuals. The degree of global population variation at a given polymorphic site is commonly described by the minor allele frequency (MAF) which is the frequency of the $2^{nd}$ most common variant in the population (i.e. as determined from a database of recorded variation such as dbSNP). As an example, a MAF of 0.5 generally means there is a 50% abundance of each of allele in a population, and a MAF of 0.05 generally means there is a 5% abundance of one allele and a 95% abundance of the other allele, although a lower frequency allele can also exist (i.e. one variant at 5%, another at 92% and 3% at a third) Generally, the more polymorphic sites that are queried, the more likely it is that two or more individuals can be distinguished from one another (FIG. 1). Because adjacent portions of the genome are commonly co-inherited (i.e. in linkage disequilibrium), assessing multiple polymorphic sites in different regions of the genome (i.e. on different chromosomes), is typically advantageous for maximizing the chance of being able to effectively distinguish two or more individual contributors to a mixed population of cells from different individuals.

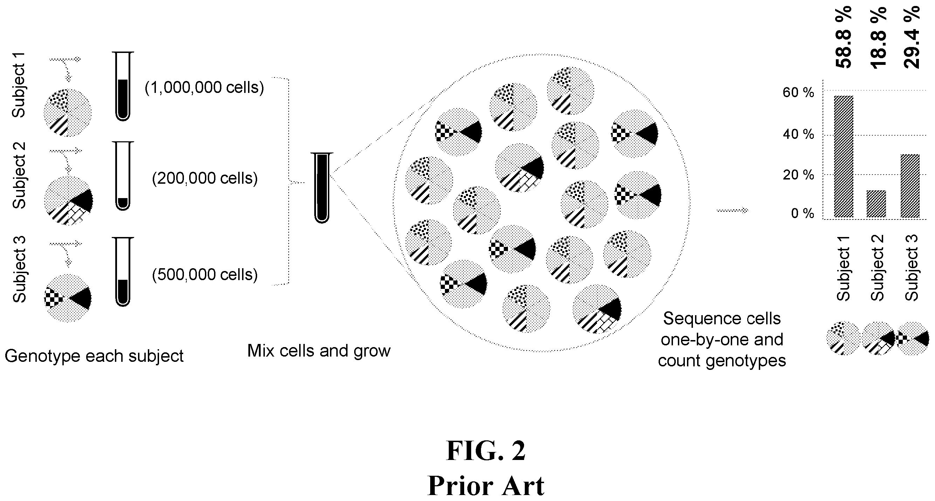

One way that mixtures of cells derived from different individuals have been resolved and quantified is with a single-cell analysis approach (FIG. 2) where individual cells are genotyped (DNA or RNA from each independent cell is sequenced and each unique genotype is counted). This can be achieved by processing each cell as a distinct entity in an individual test tube, plate well, droplet, etc., such that the derivative sequence reads from each cell can be linked back to that same cell (often using some form of single cell barcoding technique, i.e. PMID 28091601, PMID 2954551, PMID 30087104.) This approach is advantageous insofar as the genotypes of many polymorphic markers from a single cell or large DNA molecule can be informatically linked together, however, these approaches are often complex, expensive and frequently require intact cells or other special preparation of material.

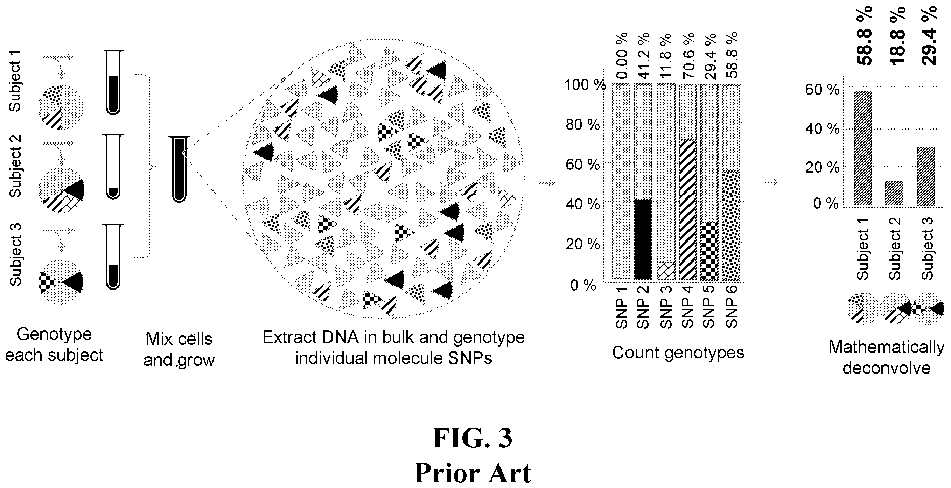

Another approach is single-molecule analysis in which cells mixed and grown together have nucleic acids bulk-extracted and genotyped and the relative abundance of individual polymorphic sites are counted. The results can be computationally deconvolved and compared to known genotypes from each individual source (FIG. 3). Mixtures of DNA molecules that are not contained within cells can be similarly genotyped and deconvolved. This approach is simpler than single-cell genotyping but can require sequencing to higher depth and assessing more polymorphic sites to technically resolve the mixture. This approach also may require far higher sequencing accuracy, which can be limiting with conventional NGS methods, especially as mixtures increase in complexity.

**SUMMARY**

The present technology relates generally to methods and associated reagents for assessing and resolving nucleic acid mixtures and/or mixed cell populations. In particular, some embodiments of the technology are directed to utilizing Duplex Sequencing for assessing and resolving nucleic acid mixtures (e.g., multichimeric mixtures, mixtures of nucleic acids from more than one source, etc.) in a sample and associated applications. For example, various embodiments of the present technology include performing Duplex Sequencing methods that allow direct identification and quantification of personal alleles as well as unique combination of alleles to deconvolve a mixture into proportions of original sources of that mixture. Various aspects of the present technology have many applications in both pre-clinical and clinical cancer (tumor) assessment, forensics (identification, etc.), mixture assessment for cell therapies (e.g., cord blood therapy), mixture assessment from human-derived samples, detection of microchimerism, quality control with cell manufacturing, mixture identification in food supply (e.g. mixtures of strains of staple crops, fish, etc.), contamination assessment in biological industrial processes (e.g. cell-based manufacturing), mixture deconvolution of closely related strains, species, breeds or quasispecies, identification of illegally trafficked animals or animal products, contamination with, or misuse of, proprietary strains of plants or animals, multi-pregnancy deconvolution of fetal DNA, deconvolution of organ-transplant derived DNA, among others.

In some embodiments, the present disclosure provides methods for detecting and/or quantifying a donor source of nucleic acid from a mixture that comprises the steps of providing the mixture comprising target double-stranded DNA molecules from one or more donor sources, wherein the target double-stranded DNA molecules contain one or more genetic polymorphisms, and generating an error-corrected sequence read for each of a plurality of the target double-stranded DNA molecules in the mixture. In certain embodiments, generating an error-corrected sequence read comprises the steps of ligating adapter molecules to the plurality of target double-stranded DNA fragments to generate a plurality of adapter-DNA molecules, generating a set of copies of an original first strand of the adapter-DNA molecule and a set of copies of an original second strand of the adapter-DNA molecule, sequencing one or more copies of the original first and second strands to provide a first strand sequence and a second strand sequence, and comparing the first strand sequence and the second strand sequence to identify one or more correspondences between the first and second strand sequences. The method further comprises identifying a donor source of nucleic acid present in the mixture of nucleic acid by deconvolving the error-corrected sequence reads into individual genotypes.

In some embodiments, the present disclosure also provides methods for detecting and/or quantifying a donor source of nucleic acid from a mixture including the steps of generating duplex sequencing data from raw sequencing data, wherein the raw sequencing data is generated from a mixture comprising target double-stranded DNA molecules from one or more donor sources, and wherein the target double-stranded DNA molecules contain one or more genetic polymorphisms, and identifying a donor source of nucleic acid present in the mixture of nucleic acid by deconvolving the error-corrected sequence reads into individual genotypes.

In some embodiments, the mixture includes one or more unknown individual genotypes, and wherein deconvolving the error-corrected sequence reads into individual genotypes includes the steps of identifying microhaplotype allele combinations present within individual target double-stranded DNA molecules that map to one or more genetic loci in a reference sequence, evaluating all possible mixing proportions against all possible genotypes present at each genetic locus within the one or more genetic loci, and determining a list of all possible individual genotypes that adequately fit the identified microhaplotype allele combinations and all possible mixing proportions evaluated.

In other embodiments, the mixture comprises one or more known individual genotypes, and wherein deconvolving the error-corrected sequence reads into individual genotypes includes the steps of identifying microhaplotype allele combinations present within individual target double-stranded DNA molecules in the mixture, summing total counts of each allele donated from each known individual genotype, and determining a mixing proportion of each known genotype present in the mixture.

In some embodiments, the mixture comprises more than one donor source, and wherein the method further comprises determining the proportion of each donor source from the more than one donor sources present in the mixture by calculating the proportion of each genetic polymorphism or the proportion of a substantially unique combination of genetic polymorphisms present in the error-corrected sequence reads. In some embodiments, the target double-stranded DNA molecules were extracted from one or more cord blood samples. In other embodiments, the target double-stranded DNA molecules were extracted from a forensic sample. In further embodiments, the target double-stranded DNA molecules were extracted from a patient with a stem cell or organ transplant. In still further embodiments, the target double-stranded DNA molecules were extracted from a patient, and wherein identifying the one or more donor sources present in the mixture includes measuring a level of microchimerism in the patient. In yet other embodiments, the target double-stranded DNA molecules were extracted from a tumor sample.

In some embodiments, the method can further comprise quantifying a relative abundance of each individual genotype present in the mixture. In other embodiments, the one or more genetic polymorphisms comprise a microhaplotype. In embodiments that include steps of generating an error-corrected sequence read for each of a plurality of the target double-stranded DNA molecules in the mixture, the method can further comprise selectively enriching one or more targeted genomic regions prior to sequencing. In embodiments that include steps generating duplex sequencing data, the target double-stranded DNA molecules in the mixture may be selectively enriched for one or more targeted genomic regions prior to generating raw sequencing data. In some such embodiments, the one or more targeted genomic regions comprises a microhaplotype site in the genome.

In some embodiments, the method provides for detecting and/or quantifying a donor source of nucleic acid from a mixture, wherein one or more of the donor sources have known genotypes. In other embodiments, the method provides for detecting and/or quantifying a donor source of nucleic acid from a mixture, wherein one or more of the donor sources have unknown genotypes. In various embodiments, the method can include comparing one or more individual genotypes to a database comprising a plurality of known genotypes to identify the one or more donor sources.

In some embodiments, the present disclosure provides for systems, such as for example systems for detecting and/or quantifying a donor source of nucleic acid from a mixture. Various embodiments of systems in accordance with aspects of the present technology include a computer network for transmitting information relating to sequencing data and genotype data, wherein the information includes one or more of raw sequencing data, duplex sequencing data, sample information, and genotype information; a client computer associated with one or more user computing devices and in communication with the computer network; a database connected to the computer network for storing a plurality of genotype profiles and user results records; a duplex sequencing module in communication with the computer network and configured to receive raw sequencing data and requests from the client computer for generating duplex sequencing data, group sequence reads from families representing an original double-stranded nucleic acid molecule and compare representative sequences from individual strands to each other to generate duplex sequencing data; and a genotype module in communication with the computer network and configured to identify microhaplotype alleles and calculate relative abundance of the donor source to generate genotype data. In some embodiments, the genotype profiles comprise microhaplotype and/or single nucleotide polymorphism (SNP) information from a plurality of known donor sources.

In some embodiments, the present disclosure provides for a computer system for performing a method in accordance with aspects of the present technology and, for example, as described herein for detecting and/or quantifying a donor source of nucleic acid from a mixture. The system can include at least one computer with a processor, memory, database, and a non-transitory computer readable storage medium comprising instructions for the processor(s), wherein said processor(s) are configured to execute said instructions to perform operations comprising the methods.

In some embodiments, the present disclosure provides for a non-transitory computer-readable storage medium comprising instructions that, when executed by one or more processors, performs a method in accordance with aspects of the present technology and, for example, as described herein. In certain embodiments, the non-transitory computer-readable storage medium includes instructions for computing a mixing proportion of each identified donor source.

In still further embodiments, the present disclosure provides for a non-transitory computer-readable medium whose contents cause at least one computer to perform a method for providing duplex sequencing data for double-stranded nucleic acid molecules in a sample comprising a mixture of donor source material. For example, some methods include steps of receiving raw sequence data from a user computing device; creating a sample-specific data set comprising a plurality of raw sequence reads derived from a plurality of nucleic acid molecules in the sample; grouping sequence reads from families representing an original double-stranded nucleic acid molecule, wherein the grouping is based on a shared single molecule identifier sequence; comparing a first strand sequence read and a second strand sequence read from an original double-stranded nucleic acid molecule to identify one or more correspondences between the first and second strand sequences reads; providing duplex sequencing data for the double-stranded nucleic acid molecules in the sample; and identifying microhaplotype allele combinations present within individual double-stranded nucleic acid molecules in the sample to identify one or more donor sources in the mixture; and, optionally, computing a mixing proportion of each identified donor source. In some embodiments, such methods may also include the steps of identifying nucleotide positions of non-complementarity between the compared first and second sequence reads, and, in positions of non-complementarity, identifying and eliminating or discounting process errors.

In other embodiments, the present disclosure provides for a non-transitory computer-readable medium whose contents cause at least one computer to perform a method for detecting, identifying and quantifying microhaplotypes present in nucleic acid mixtures to determine known source genotypes a sample, where the method includes steps of identifying microhaplotype allele combinations present within individual DNA molecules in a mixture; summing total counts of each allele donated from each known source genotype; and determining a mixing proportion of each genotype present in the mixture. In some embodiments, the method may also include a step of calculating a mixing proportion that includes using a regression-based model. In further embodiments, the method may also include a step of comparing the determined mixing proportion of each genotype with an original mixing proportion.

In still further embodiments, the present disclosure provides for a non-transitory computer-readable medium whose contents cause at least one computer to perform a method for deconvolving nucleic acid mixtures of unknown genotypes in a sample, where the method includes the steps of identifying microhaplotype allele combinations present within individual DNA molecules in a mixture; evaluating all possible mixing proportions against all possible genotypes present at each genetic locus; and determining a list of all possible genotypes that adequately fit the identified microhaplotype allele combinations and all possible mixing proportions evaluated. In some embodiments, the method may also include the step of comparing the possible genotypes from the unknown genotypes in the sample to a database comprising genotype profiles of known sources to identify a donor source.

Other embodiments and aspects of the present technology are described further in the following detailed description.

## BRIEF DESCRIPTION OF THE DRAWING

Many aspects of the present disclosure can be better understood with reference to the following figures, which together make up the Drawing. These figures are for illustration purposes only, and not for limitation. The components in the figures are not necessarily to scale. Instead, emphasis is placed on illustrating clearly the principles of the present disclosure.

FIG. **1,** FIG. **2,** and FIG. **3** each illustrate aspects of SNP sequencing from the prior art.

FIG. **4A** illustrates a nucleic acid adapter molecule for use with some embodiments of the present technology and a double-stranded adapter-nucleic acid complex resulting from ligation of the adapter molecule to a double-stranded nucleic acid fragment in accordance with an embodiment of the present technology.

FIG. **4B** and FIG. **4C** are conceptual cartoon illustrations of various Duplex Sequencing method steps in accordance with an embodiment of the present technology.

FIG. **5** is a schematic diagram of a network computer system for use with the methods and/or reagents disclosed herein for deconvolution of nucleic acids in a mixture in accordance with an embodiment of the present technology.

FIG. **6** is a flow diagram illustrating a routine for providing Duplex Sequencing consensus sequence data in accordance with an embodiment of the present technology in accordance with an embodiment of the present technology.

FIG. **7** is a flow diagram illustrating a routine for detecting, identifying and quantifying microhaplotypes present in nucleic acid mixtures to determine known source genotypes a sample in accordance with an embodiment of the present technology.

FIG. **8** is a flow diagram illustrating a routine for deconvolving nucleic acid mixtures of unknown genotypes in a sample in accordance with an embodiment of the present technology.

FIG. **9** illustrates one example of genotype data that can be determined using the routine of FIG. **7,** and in accordance with an aspect of the present technology.

FIG. **10** illustrates one example of genotype data that can be determined using the routine of FIG. **8,** and in accordance with an aspect of the present technology.

FIG. **11** illustrates a schematic for a method for analyzing cord blood expansion followed by deconvolution of nucleic acid mixtures in accordance with an aspect of the present technology.

FIG. **12** provides an exemplary global distribution of the SNP panel used in the method described in FIG. **11,** and in accordance with an aspect of the present technology.

FIG. **13** is a bar graph showing exemplary on-target duplex sequencing depth for each sample and in accordance with aspect of the present technology.

FIG. **14** shows a panel identifying 11 specific SNP alleles used to differentiate the donor genotypes in accordance with an aspect of the present technology.

FIGS. **15A-B** and FIGS. **15C-D** each are bar graphs showing the relative abundance of each cord sample in the mixtures, as quantified by Nanodrop sequencing (dark grey bar on the left for each sample) and in accordance with an aspect of the present technology (light grey bar on the right for each sample).

FIG. **15E** is a bar graph showing DNA quantification within each sample by Nanodrop (dark grey bar on the left for each sample) and by Qubit fluorometer (light grey bar on the right for each sample) measurement in accordance with an aspect of the present technology.

FIG. **16** illustrates the fold-difference in quantification for each individual cord blood sample within each mixture in accordance with an aspect of the present technology.

FIG. **17** is a bar graph depicting the flow-cytometry determined CD34+ fraction of cells prior to expansion and the CD34+ fraction of cells (as determined by Duplex Sequencing) following expansion for each individual blood cord sample in accordance with an aspect of the present technology.

FIG. **18A** is a bar graph showing frequency of microhaplotype regions as a function of nucleotide sequence length in accordance with an aspect of the present technology.

FIG. **18B** is an example of allele frequency for one microhaplotype in varied populations in accordance with an aspect of the present technology.

FIG. **19** is a line graph showing results of a simulated deconvolution of Duplex Sequencing data for showing estimates of mixing proportions in accordance with an aspect of the present technology.

FIG. **20** illustrates one example of a linear regression model for determining donor sources within a mixture in accordance with an aspect of the present technology.

FIG. **21,** panels A-D are each bar graphs depicting the true mixing proportions of sources 1-5 in accordance with an aspect of the present technology.

FIG. **22,** panels A-D show true mixing proportions plotted against estimated mixing proportions for each individual source in each of the mixtures when the genotypes for each of the five potential sources was known in advance, and in accordance with an aspect of the present technology.

FIG. **23A,** FIG. **23B,** FIG. **23C,** and FIG. **23D** are each heat map graphs demonstrating the likelihood and abundance of each unknown genotype source determined in the sample using Duplex sequencing, and in accordance with an aspect of the present technology.

FIG. **24,** panels A-D show results of microhaplotype alleles that could be determined for multiple sources even when the genotypes of the sources were not previously known, and in accordance with an aspect of the present technology.

FIG. **25** is a scatter plot comparing actual mixing proportions of samples against the estimates of abundance of the samples in a mixture in accordance with an aspect of the present technology.

FIG. **26** is a line graph plotting proportions of genotypes present in a mixture that are detected (solid line) and proportion of reads with at least one false positive (dashed line) in accordance with an aspect of the present technology.

FIG. **27,** panels A-C are line plots showing estimated versus true mixing proportions in 3 different simulated mixtures of 50 genotypes and in accordance with an aspect of the present technology.

## DETAILED DESCRIPTION

Specific details of several embodiments of the technology are described below with reference to FIGS. **1-27.** The embodiments can include, for example, methods for deconvolving mixtures and source identification and associated reagents, kits and software for use in such methods. Some embodiments of the technology are directed to utilizing Duplex Sequencing for assessing and resolving a mixture (e.g., cell mixture, tissue mixture, multichimeric organism or tissue, fetal DNA, transplant tissue, multichimeric cell cultures, a forensic sample, nucleic acid mixtures, etc.). Other embodiments of the technology are directed to utilizing Duplex Sequencing for determining a genotype signature (e.g., combination of unique polymorphisms) associated with an individual or group of individuals. Additional embodiments of the technology are directed to identifying more than one source of genetic material contributing to a mixture and relative proportions of each source based on, for example, individuals' genotype signatures.

Although many of the embodiments are described herein with respect to Duplex Sequencing other sequencing modalities capable of generating error-corrected sequencing reads in addition to those described herein are within the scope of the present technology. Additionally, other embodiments of the present technology can have different configurations, components, or procedures than those described herein. A person of ordinary skill in the art, therefore, will accordingly understand that the technology can have other embodiments with additional elements and that the technology can have other embodiments without several of the features shown and described below with reference to FIGS. 1-27.

I. Certain Definitions

In order for the present disclosure to be more readily understood, certain terms are first defined below. Additional definitions for the following terms and other terms are set forth throughout the specification.

In this application, unless otherwise clear from context, the term "a" may be understood to mean "at least one." As used in this application, the term "or" may be understood to mean "and/or." In this application, the terms "comprising" and "including" may be understood to encompass itemized components or steps whether presented by themselves or together with one or more additional components or steps. Where ranges are provided herein, the endpoints are included. As used in this application, the term "comprise" and variations of the term, such as "comprising" and "comprises," are not intended to exclude other additives, components, integers or steps.

*About:* The term "about", when used herein in reference to a value, refers to a value that is similar, in context to the referenced value. In general, those skilled in the art, familiar with the context, will appreciate the relevant degree of variance encompassed by "about" in that context. For example, in some embodiments, the term "about" may encompass a range of values that within 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value. For variances of single digit integer values where a single numerical value step in either the positive or negative direction would exceed 25% of the value, "about" is generally accepted by those skilled in the art to include, at least 1, 2, 3, 4, or 5 integer values in either the positive or negative direction, which may or may not cross zero depending on the circumstances. A non-limiting example of this is the supposition that 3 cents can be considered about 5 cents in some situations that would be apparent to one skilled in that art.

*Analog:* As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, e.g., by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance.

*Biological Sample:* As used herein, the term "biological sample" or "sample" typically refers to a sample obtained or derived from one or more biological sources (e.g., a tissue or organism or cell culture) of interest, as described herein. In some embodiments, a source of interest comprises an organism, such as an animal or human. In other embodiments, a source of interest comprises a microorganism, such as a bacterium, virus, protozoan, or fungus. In further embodiments, a source of interest may be a synthetic tissue, organism, cell culture, nucleic acid or other material. In yet further embodiments, a source of interest may be a plant-based organism. In yet another embodiment, a sample may be an environmental sample such as, for example, a water sample, soil sample, archeological sample, or other sample collected from a non-living source. In other embodiments, a sample may be a multi-organism sample (e.g., a mixed organism sample). In still further embodiments, a sample may comprise a cell mixture or a tissue mixture. In other embodiments, a sample may be derived from a multichimeric organism or tissue, transplant tissue, or multichimeric cell cultures. In further embodiments, the sample may include fetal DNA. In yet other embodiments, a sample may be collected from a crime scene or other law enforcement investigation inquiry (e.g., in forensic cases such as for identifying perpetrators, victims or missing persons, etc.). In other embodiments, a sample may be collected from a war or terrorism investigation inquiry or historical study (e.g., for identifying victims or missing persons), etc. In other embodiments, a sample may be collected from an archeological study. In some embodiments, a biological sample is or comprises biological tissue or fluid. In some embodiments, a biological sample may be isolated DNA or other nucleic acids or may comprise bone marrow; blood; blood cells; stem cells, ascites; tissue samples, biopsy samples or or fine needle aspiration samples; cell-containing body fluids; free floating nucleic acids; protein-bound nucleic acids, riboprotein-bound nucleic acids; sputum; saliva; urine; cerebrospinal fluid, peritoneal fluid; pleural fluid; feces; lymph; gynecological fluids; skin swabs; vaginal swabs; pap smear, oral swabs; nasal swabs; washings or lavages such as a ductal lavages or broncheoalveolar lavages; vaginal fluid, aspirates; scrapings; bone marrow specimens; tissue biopsy specimens; fetal tissue or fluids; surgical specimens; feces, other body fluids, secretions, and/or excretions; and/or cells therefrom, *etc.* In some embodiments, a biological sample is or comprises cells obtained from an individual. In some embodiments, obtained cells are or include cells from an individual from whom the sample is obtained. In some embodiments cell-derivatives such as organelles or vesicles or exosomes. In a particular embodiment, a biological sample is a liquid biopsy obtained from a subject. In some embodiments, a sample is a "primary sample" obtained directly from a source of interest by any appropriate means. For example, in some embodiments, a primary biological sample is obtained by methods selected from the group consisting of biopsy (*e.g.*, fine needle aspiration or tissue biopsy), surgery, collection of body fluid (*e.g.*, blood (or plasma or serum separated therefrom), lymph, feces *etc.*), *etc.* In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, *etc*.

*Cancer disease:* In an embodiment, a disease or disorder is a "cancer disease" which is familiar to those experience in the art as being generally characterized by dysregulated growth of abnormal cells, which may metastasize. Cancer diseases detectable using one or more aspects of the present technology comprise, by way of non-limiting examples, prostate cancer (i.e. adenocarcinoma, small cell), ovarian cancer (e.g., ovarian adenocarcinoma, serous carcinoma or embryonal carcinoma, yolk sac tumor, teratoma), liver cancer (e.g., HCC or hepatoma, angiosarcoma), plasma cell tumors (e.g., multiple myeloma, plasmacytic leukemia, plasmacytoma, amyloidosis, Waldenstrom's macroglobulinemia), colorectal cancer (e.g., colonic adenocarcinoma, colonic mucinous adenocarcinoma, carcinoid, lymphoma and rectal adenocarcinoma, rectal squamous carcinoma), leukemia (e.g., acute myeloid leukemia, acute lymphocytic leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia, T-cell leukemia, Sezary syndrome, systemic mastocytosis, hairy cell leukemia, chronic myeloid leukemia blast crisis), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, follicular lymphoma, mantle cell lymphoma, MALT lymphoma, marginal cell lymphoma, Richter's transformation, double hit lymphoma, transplant associated lymphoma, CNS lymphoma, extranodal lymphoma, HIV-associated lymphoma, hairy cell leukemia, variant hairy cell leukemia, endemic lymphoma, Burkitt's lymphoma, transplant-associated lymphoproliferative neoplasms, and lymphocytic lymphoma etc.), cervical cancer (squamous cervical carcinoma, clear cell carcinoma, HPV associated carcinoma, cervical sarcoma etc.) esophageal cancer (esophageal squamous cell carcinoma, adenocarcinoma, certain grades of Barretts esophagus, esophageal adenocarcinoma), melanoma (dermal melanoma, uveal melanoma, acral melanoma, amelanotic melanoma etc.), CNS tumors (e.g., oligodendroglioma, astrocytoma, glioblastoma multiforme, meningioma, schwannoma, craniopharyngioma etc.), pancreatic cancer (e.g., adenocarcinoma, adenosquamous carcinoma, signet ring cell carcinoma, hepatoid carcinoma, colloid carcinoma, islet cell carcinoma, pancreatic neuroendocrine carcinoma etc.), gastrointestinal stromal tumor, sarcoma (e.g., fibrosarcoma, myxosarcoma, liposarcoma, chondrosarcoma, osteogenic sarcoma, angiosarcoma, endothelioma sarcoma, lymphangiosarcoma, lymphangioendothelioma sarcoma, leiomyosarcoma, Ewing's sarcoma, and rhabdomyosarcoma, spindle cell tumor etc.), breast cancer (e.g., inflammatory carcinoma, lobar carcinoma, ductal carcinoma etc.), ER-positive cancer, HER-2 positive cancer, bladder cancer (squamous bladder cancer, small cell bladder cancer, urothelial cancer etc.), head and neck cancer (e.g., squamous cell carcinoma of the head and neck, HPV-associated squamous cell carcinoma, nasopharyngeal carcinoma etc.), lung cancer (e.g., non-small cell lung carcinoma, large cell carcinoma, bronchogenic carcinoma, squamous cell cancer, small cell lung cancer etc.), metastatic cancer, oral cavity cancer, uterine cancer (leiomyosarcoma, leiomyoma etc.), testicular cancer (e.g., seminoma, non-seminoma, and embryonal carcinoma yolk sack tumor etc), skin cancer (e.g., squamous cell carcinoma, and basal cell carcinoma, merkel cell carcinoma, melanoma, cutaneous t-cell lymphoma etc.), thyroid cancer (e.g., papillary carcinoma, medullary carcinoma, anaplastic thyroid cancer etc.), stomach cancer, intra-epithelial cancer, bone cancer, biliary tract cancer, eye cancer, larynx cancer, kidney cancer (e.g., renal cell carcinoma, Wilms tumor etc.), gastric cancer, blastoma (e.g., nephroblastoma, medulloblastoma, hemangioblastoma, neuroblastoma, retinoblastoma etc.), myeloproliferative neoplasms (polycythemia vera, essential thrombocytosis, myelofibrosis, etc.), chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, pheochromocytoma, small cell lung cancer, peritoneal mesothelioma, hyperparathyroid adenoma, adrenal cancer, cancer of unknown primary, cancer of the endocrine system, cancer of the penis, cancer of the urethra, cutaneous or intraocular melanoma, a gynecologic tumor, solid tumors of childhood, or neoplasms of the central nervous system, primary mediastinal germ cell tumor, clonal hematopoiesis of indeterminate potential, smoldering myeloma, monoclonal gammaglobulinopathy of unknown significant, monoclonal B-cell lymphocytosis, low grade cancers, clonal field defects, preneoplastic neoplasms, ureteral cancer, autoimmune-associated cancers (i.e. ulcerative colitis, primary sclerosing cholangitis, celiac disease), cancers associated with an inherited predisposition (i.e. those carrying genetic defects in such as *BRCA1*, *BRCA2*, *TP53*, *PTEN*, *ATM*, etc.) and various genetic syndromes such as MEN1, MEN2 trisomy 21 etc.) and those occurring when exposed to chemicals in utero (i.e. clear cell cancer in female offspring of women exposed to Diethylstilbestrol [DES]), among many others.

*Determine:* Many methodologies described herein include a step of "determining". Those of ordinary skill in the art, reading the present specification, will appreciate that such "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference.

*Duplex Sequencing (DS):* As used herein, "Duplex Sequencing (DS)" is, in its broadest sense, refers to a tag-based error-correction method that achieves exceptional accuracy by comparing the sequence from both strands of individual DNA molecules.

*Expression:* As used herein, "expression" of a nucleic acid sequence refers to one or more of the following events: (1) production of an RNA template from a DNA sequence (*e.g.*, by transcription); (2) processing of an RNA transcript (e.g., by splicing, editing, 5' cap formation, and/or 3' end formation); (3) translation of an RNA into a polypeptide or protein; and/or (4) post-translational modification of a polypeptide or protein.

*Mutation:* As used herein, the term "mutation" refers to alterations to nucleic acid sequence or structure. Mutations to a polynucleotide sequence can include point mutations (e.g., single base mutations), multinucleotide mutations, nucleotide deletions, sequence rearrangements, nucleotide insertions, and duplications of the DNA sequence in the sample, among complex multinucelotide changes. Mutations can occur on both strands of a duplex DNA molecule as complementary base changes (i.e. true mutations), or as a mutation on one strand but not the other strand (i.e. heteroduplex), that has the potential to be either repaired, destroyed or be mis-repaired/converted into a true double stranded mutation. Mutations may represent alterations relative to a control sample from the same or a related source and/or individual. Mutations may represent alterations relative to a reference sequence.

*Non-cancerous disease:* In another embodiment, a disease or disorder is a non-cancerous disease that is caused by, or contributed to by, a genomic mutation or damage. By way of non-limiting examples, such non-cancerous types of diseases or disorders that are detectable using one or more aspects of the present technology comprise diabetes; autoimmune disease or disorders, infertility, neurodegeneration, progeria, cardiovascular disease, any disease associated with treatment for another genetically-mediated disease (i.e. chemotherapy-mediated neuropathy and renal failure associated with chemotherapy such as cisplatin), Alzheimer's/dementia, obesity, heart disease, high blood pressure, arthritis, mental illness, other neurological disorders (neurofibromatosis), and a multifactorial inheritance disorder (e.g., a predisposition triggered by environmental factors).

*Nucleic acid:* As used herein, in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. As will be clear from context, in some embodiments, "*nucleic acid*" refers to an individual nucleic acid residue (e.g., a nucleotide and/or nucleoside); in some embodiments, "*nucleic acid*" refers to an oligonucleotide chain comprising individual nucleic acid residues. In some embodiments, a "*nucleic acid*" is or comprises RNA; in some embodiments, a "*nucleic acid*" is or comprises DNA. In some embodiments, a nucleic acid is, comprises, or consists of one or more natural nucleic acid residues. In some embodiments, a nucleic acid is, comprises, or consists of one or more nucleic acid analogs. In some embodiments, a nucleic acid analog differs from a nucleic acid in that it does not utilize a phosphodiester backbone. For example, in some embodiments, a nucleic acid is, comprises, or consists of one or more "*peptide nucleic acids*", which are known in the art and have peptide bonds instead of phosphodiester bonds in the backbone, are considered within the scope of the present technology. Alternatively, or additionally, in some embodiments, a nucleic acid has one or more phosphorothioate and/or 5'-N-phosphoramidite linkages rather than phosphodiester bonds. In some embodiments, a nucleic acid is, comprises, or consists of one or more natural nucleosides (e.g., adenosine, thymidine, guanosine, cytidine, uridine, deoxyadenosine, deoxythymidine, deoxy guanosine, and deoxycytidine). In some embodiments, a nucleic acid is, comprises, or consists of one or more nucleoside analogs (e.g., 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 0(6)-methylguanine, 2-thiocytidine, methylated bases, intercalated bases, and combinations thereof). In some embodiments, a nucleic acid comprises one or more modified sugars (e.g., 2'-fluororibose, ribose, 2'-deoxyribose, arabinose, and hexose) as compared with those in natural nucleic acids. In some embodiments, a nucleic acid has a nucleotide sequence that encodes a functional gene product such as an RNA or protein. In some embodiments, a nucleic acid includes one or more introns. In some embodiments, nucleic acids are prepared by one or more of isolation from a natural source, enzymatic synthesis by polymerization based on a complementary template (*in vivo* or *in vitro*), reproduction in a recombinant cell or system, and chemical synthesis. In some embodiments, a nucleic acid is at least 2, 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 200, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. In some embodiments, a nucleic acid is partly or wholly single stranded; in some embodiments, a nucleic acid is partly or wholly double-stranded. In some embodiments a nucleic acid may be branched of have secondary structures. In some embodiments a nucleic acid has a nucleotide sequence comprising at least one element that encodes, or is the complement of a sequence that encodes, a polypeptide. In some embodiments, a nucleic acid has enzymatic activity. In some embodiments the nucleic acid serves a mechanical function, for example in a ribonucleoprotein complex or a transfer RNA.

*Polynucleotide damage:* As used herein, the term "polynucleotide damage" or "nucleic acid damage" refers to damage to a subject's deoxyribonucleic acid (DNA) sequence ("DNA damage") or ribonucleic acid (RNA) sequence ("RNA damage") that is directly or indirectly (e.g. a metabolite, or induction of a process that is damaging or mutagenic) caused by an agent or process. Damaged nucleic acid may lead to the onset of a disease or disorder in a subject. Polynucleotide damage may further comprise chemical and/or physical modification of the DNA in a cell. In some embodiments, the damage is or comprises, by way of non-limiting examples, at least one of oxidation, alkylation, deamination, methylation, hydrolysis, hydroxylation, nicking, intra-strand crosslinks, inter-strand cross links, blunt end strand breakage, staggered end double strand breakage, phosphorylation, dephosphorylation, sumoylation, glycosylation, deglycosylation, putrescinylation, carboxylation, halogenation, formylation, single-stranded gaps, damage from heat, damage from desiccation, damage from UV exposure, damage from gamma radiation damage from X-radiation, damage from ionizing radiation, damage from non-ionizing radiation, damage from heavy particle radiation, damage from nuclear decay, damage from beta-radiation, damage from alpha radiation, damage from neutron radiation, damage from proton radiation, damage from antimatter, damage from cosmic radiation, damage from high pH, damage from low pH, damage from reactive oxidative species, damage from free radicals, damage from peroxide, damage from hypochlorite, damage from tissue fixation such formalin or formaldehyde, damage from reactive iron, damage from low ionic conditions, damage from high ionic conditions, damage from unbuffered conditions, damage from nucleases, damage from environmental exposure, damage from fire, damage from mechanical stress, damage from enzymatic degradation, damage from microorganisms, damage from preparative mechanical shearing, damage from preparative enzymatic fragmentation, damage having naturally occurred *in vivo*, damage having occurred during nucleic acid extraction, damage having occurred during sequencing library preparation, damage having been introduced by a polymerase, damage having been introduced during nucleic acid repair, damage having occurred during nucleic acid end-tailing, damage having occurred during nucleic acid ligation, damage having occurred during sequencing, damage having occurred from mechanical handling of DNA, damage having occurred during passage through a nanopore, damage having occurred as part of aging in an organism, damage having occurred as a result if chemical exposure of an individual, damage having occurred by a mutagen, damage having occurred by a carcinogen, damage having occurred by a clastogen, damage having occurred from *in vivo* inflammation damage due to oxygen exposure, damage due to one or more strand breaks, and any combination thereof.

*Reference:* As used herein, the term "reference" describes a standard or control relative to which a comparison is performed. For example, in some embodiments, an agent, animal, individual, population, sample, sequence or value of interest is compared with a reference or control agent, animal, individual, population, sample, sequence or value or representation thereof in a physical or computer database that may be present at a location or accessed remotely via electronic means. In one embodiment, the reference is a reference genome or a reference genome assembly. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control. A "reference sample" refers to a sample from a subject that is distinct from the test subject and isolated in the same way as the sample to which it is compared. The subject of the reference sample may be genetically identical to the test subject or may be different.

*Single Molecule Identifier (SMI):* As used herein, the term "single molecule identifier" or "SMI", (which may be referred to as a "tag" a "barcode", a "molecular bar code", a "Unique Molecular Identifier", or "UMI", among other names) refers to any material (e.g., a nucleotide sequence, a nucleic acid molecule feature) that is capable of substantially distinguishing an individual molecule among a larger heterogeneous population of molecules. In some embodiments, a SMI can be or comprise an exogenously applied SMI. In some embodiments, an exogenously applied SMI may be or comprise a degenerate or semi-degenerate sequence. In some embodiments substantially degenerate SMIs may be known as Random Unique Molecular Identifiers (R-UMIs). In some embodiments an SMI may comprise a code (for example a nucleic acid sequence) from within a pool of known codes. In some embodiments pre-defined SMI codes are known as Defined Unique Molecular Identifiers (D-UMIs). In some embodiments, a SMI can be or comprise an endogenous SMI. In some embodiments, an endogenous SMI may be or comprise information related to specific shear-points of a target sequence, features relating to the terminal ends of individual molecules comprising a target sequence, or a specific sequence at or adjacent to or within a known distance from an end of individual molecules. In some embodiments an SMI may relate to a sequence variation in a nucleic acid molecule cause by random or semi-random damage, chemical modification, enzymatic modification or other modification to the nucleic acid molecule. In some embodiments the modification may be deamination of methylcytosine. In some embodiments the modification may entail sites of nucleic acid nicks. In some embodiments, an SMI may comprise both exogenous and endogenous elements. In some embodiments an SMI may comprise physically adjacent SMI elements. In some embodiments SMI elements may be spatially distinct in a molecule. In some embodiments an SMI may be a non-nucleic acid. In some embodiments an SMI may comprise two or more different types of SMI information. Various embodiments of SMIs are further disclosed in International Patent Publication No. WO2017/100441, which is incorporated by reference herein in its entirety.

*Strand Defining Element (SDE):* As used herein, the term "Strand Defining Element" or "SDE", refers to any material which allows for the identification of a specific strand of a double-stranded nucleic acid material and thus differentiation from the other/complementary strand (e.g., any material that renders the amplification products of each of the two single stranded nucleic acids resulting from a target double-stranded nucleic acid substantially distinguishable from each other after sequencing or other nucleic acid interrogation). In some embodiments, a SDE may be or comprise one or more segments of substantially non-complementary sequence within an adapter sequence. In particular embodiments, a segment of substantially non-complementary sequence within an adapter sequence can be provided by an adapter molecule comprising a Y-shape or a "loop" shape. In other embodiments, a segment of substantially non-complementary sequence within an adapter sequence may form an unpaired "bubble" in the middle of adjacent complementary sequences within an adapter sequence. In other embodiments an SDE may encompass a nucleic acid modification. In some embodiments an SDE may comprise physical separation of paired strands into physically separated reaction compartments. In some embodiments an SDE may comprise a chemical modification. In some embodiments an SDE may comprise a modified nucleic acid. In some embodiments an SDE may relate to a sequence variation in a nucleic acid molecule caused by random or semi-random damage, chemical modification, enzymatic modification or other modification to the nucleic acid molecule. In some embodiments the modification may be deamination of methylcytosine. In some embodiments the modification may entail sites of nucleic acid nicks. Various embodiments of SDEs are further disclosed in International Patent Publication No. WO2017/100441, which is incorporated by reference herein in its entirety.

*Subject:* As used herein, the term "subject" refers an organism, typically a mammal, such as a human (in some embodiments including prenatal human forms), a non-human animal (e.g., mammals and non-mammals including, but not limited to, non-human primates, mice, rats, hamsters, otters, wildebeests, horses, sheep, dogs, cows, pigs, chickens, amphibians, reptiles, sea-life, other model organisms such as worms, flies, zebrafish etc.), and transgenic animals (e.g., transgenic rodents), etc. In some embodiments, a subject is suffering from a relevant disease, disorder or condition. In some embodiments, a subject is susceptible to a disease, disorder, or condition. In some embodiments, a subject displays one or more symptoms or characteristics of a disease, disorder or condition. In some embodiments, a subject does not display any symptom or characteristic of a disease, disorder, or condition. In some embodiments, a subject has one or more features characteristic of susceptibility to or risk of a disease, disorder, or condition. In some embodiments, a subject is an individual to whom diagnosis and/or therapy is and/or has been administered. In still other embodiments, a subject refers to any living biological sources or other nucleic acid material, for example, organisms, cells, and/or tissues, such as for *in vivo* studies, e.g.: fungi, protozoans, bacteria, archaebacteria, viruses, isolated cells in culture, cells that have been intentionally (e.g., stem cell transplant, organ transplant) or unintentionally (i.e. fetal or maternal microchimerism) or isolated nucleic acids or organelles (i.e. mitochondria, chloroplasts, free viral genomes, free plasmids, aptamers, ribozymes or derivatives or precursors of nucleic acids (i.e. oligonucleotides, dinucleotide triphosphates, etc.). In further embodiments, a subject refers to any living, or at one time living biological sources or other nucleic acid materials obtained in a forensic investigation or application.

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

## II. Selected Embodiments of Duplex Sequencing Methods and Associated Adapters and Reagents

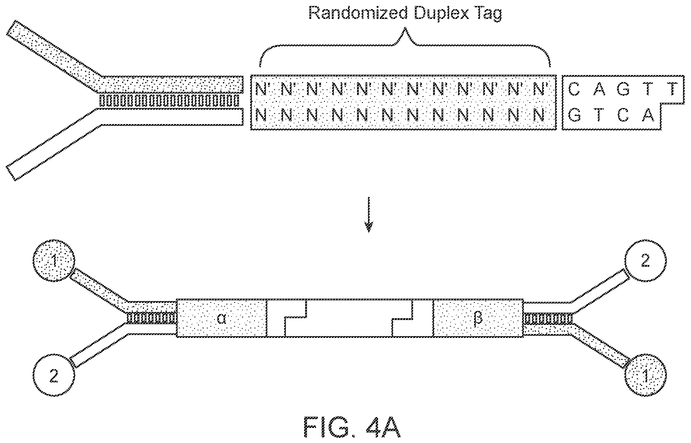

Duplex Sequencing is a method for producing error-corrected DNA sequences from double-stranded nucleic acid molecules, and which was originally described in International Patent Publication No. WO 2013/142389 and in U.S. Patent No. 9,752,188, both of which are incorporated by reference in their entireties. As illustrated in FIGS. **4A-4C,** and in certain aspects of the technology, Duplex Sequencing can be used to independently sequence both strands of individual DNA molecules in such a way that the derivative sequence reads can be recognized as having originated from the same double-stranded nucleic acid parent molecule during massively parallel sequencing, but also differentiated from each other as distinguishable entities following sequencing. The resulting sequence reads from each strand are then compared for the purpose of obtaining an error-corrected sequence of the original double-stranded nucleic acid molecule.

In certain embodiments, methods incorporating Duplex Sequencing may include ligation of one or more sequencing adapters to a target double-stranded nucleic acid molecule, comprising a first strand target nucleic acid sequence and a second strand target nucleic sequence, to produce a double-stranded target nucleic acid complex (e.g. FIG. **4A**).

In various embodiments, a resulting target nucleic acid complex can include at least one SMI sequence, which may entail an exogenously applied degenerate or semi-degenerate sequence (e.g., randomized duplex tag shown in FIG. **4A,** sequences identified as $\alpha$ and $\beta$ in FIG. **4A**), endogenous information related to the specific shear-points of the target double-stranded nucleic acid molecule, or a combination thereof. The SMI can render the target-nucleic acid molecule substantially distinguishable from the plurality of other molecules in a population being sequenced either alone or in combination with distinguishing elements of the nucleic acid fragments to which they were ligated. The SMI element's substantially distinguishable feature can be independently carried by each of the single strands that form the double-stranded nucleic acid molecule such that the derivative amplification products of each strand can be recognized as having come from the same original substantially unique double-stranded nucleic acid molecule after sequencing. In other embodiments the SMI may include additional information and/or may be used in other methods for which such molecule distinguishing functionality is useful, such as those described in the above-referenced publications. In another embodiment, the SMI element may be incorporated after adapter ligation. In some embodiments the SMI is double-stranded, while in other embodiments the SMI is single-stranded (e.g., the SMI can be on the single-stranded portion(s) of the adapters). In other embodiments, the SMI is a combination of single-stranded and double-stranded SMI sequences.

In some embodiments, each double-stranded target nucleic acid sequence complex can further include an element (e.g., an SDE) that renders the amplification products of the two single-stranded nucleic acids that form the target double-stranded nucleic acid molecule substantially distinguishable from each other after sequencing. In one embodiment, the SDE may comprise asymmetric primer sites comprised within the sequencing adapters, or, in other arrangements, sequence asymmetries may be introduced into the adapter molecules not within the primer sequences, such that at least one position in the nucleotide sequences of the first strand target nucleic acid sequence complex and the second stand of the target nucleic acid sequence complex are different from each other following amplification and sequencing. In other embodiments, the SMI may comprise another biochemical asymmetry between the two strands that differs from the canonical nucleotide sequences A, T, C, G or U, but is converted into at least one canonical nucleotide sequence difference in the two amplified and sequenced molecules. In yet another embodiment, the SDE may be a means of physically separating the two strands before amplification, such that the derivative amplification products from the first strand target nucleic acid sequence and the second strand target nucleic acid sequence are maintained in substantial physical isolation from one and other for the purposes of maintaining a distinction between the two. Other such arrangements or methodologies for providing an SDE function that allows for distinguishing the first and second strands may be utilized, such as those described in the above-referenced publications, or other methods that serves the functional purpose described.

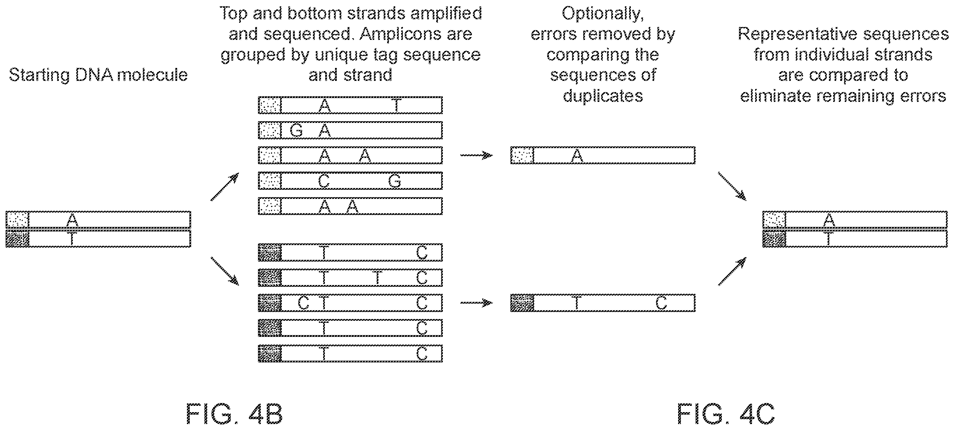

After generating the double-stranded target nucleic acid complex comprising at least one SMI and at least one SDE, or where one or both of these elements will be subsequently introduced, the complex can be subjected to DNA amplification, such as with PCR, or any other biochemical method of DNA amplification, such that one or more copies of the first strand target nucleic acid sequence and one or more copies of the second strand target nucleic acid sequence are produced (e.g., FIG. **4B**). The one or more amplification copies of the first strand target nucleic acid molecule and the one or more amplification copies of the second target nucleic acid molecule can then be subjected to DNA sequencing, preferably using a "Next-Generation" massively parallel DNA sequencing platform (e.g., FIG. **4B**).

The sequence reads produced from either the first strand target nucleic acid molecule and the second strand target nucleic acid molecule derived from the original double-stranded target nucleic acid molecule can be identified based on sharing a related substantially unique SMI and distinguished from the opposite strand target nucleic acid molecule by virtue of an SDE. In some embodiments the SMI may be a sequence based on a mathematically-based error correction code (for example, a Hamming code), whereby certain amplification errors, sequencing errors or SMI synthesis errors can be tolerated for the purpose of relating the sequences of the SMI sequences on complementary strands of an original Duplex (e.g., a double-stranded nucleic acid molecule). For example, with a double stranded exogenous SMI where the SMI comprises 15 base pairs of fully degenerate sequence of canonical DNA bases, an estimated $4^{15} = 1,073,741,824$ SMI variants will exist in a population of the fully degenerate SMIs. If two SMIs are recovered from reads of sequencing data that differ by only one nucleotide within the SMI sequence out of a population of 10,000 sampled SMIs, it can be mathematically calculated the probability of this occurring by random chance and a decision made whether it is more probable that the single base pair difference reflects one of the aforementioned types of errors and the SMI sequences could be determined to have in fact derived from the same original duplex molecule. In some embodiments where the SMI is, at least in part, an exogenously applied sequence where the sequence variants are not fully degenerate to each other and are, at least in part, known sequences, the identity of the known sequences can in some embodiments be designed in such a way that one or more errors of the aforementioned types will not convert the identity of one known SMI sequence to that of another SMI sequence, such that the probability of one SMI being misinterpreted as that of another SMI is reduced. In some embodiments this SMI design strategy comprises a Hamming Code approach or derivative thereof. Once identified, one or more sequence reads produced from the first strand target nucleic acid molecule are compared with one or more sequence reads produced from the second strand target nucleic acid molecule to produce an error-corrected target nucleic acid molecule sequence (e.g., FIG. **4C**). For example, nucleotide positions where the bases from both the first and second strand target nucleic acid sequences agree are deemed to be true sequences, whereas nucleotide positions that disagree between the two strands are recognized as potential sites of technical errors that may be discounted, eliminated, corrected or otherwise identified. An error-corrected sequence of the original double-stranded target nucleic acid molecule can thus be produced (shown in FIG. **4C**). In some embodiments and following separately grouping of each of the sequencing reads produced from the first strand target nucleic acid molecule and the second strand target nucleic acid molecule, a single-strand consensus sequence can be generated for each of the first and second strands. The single-stranded consensus sequences from the first strand target nucleic acid molecule and the second strand target nucleic acid molecule can then be compared to produce an error-corrected target nucleic acid molecule sequence (e.g., FIG. **4C**).

Alternatively, in some embodiments, sites of sequence disagreement between the two strands can be recognized as potential sites of biologically-derived mismatches in the original double stranded target nucleic acid molecule. Alternatively, in some embodiments, sites of sequence disagreement between the two strands can be recognized as potential sites of DNA synthesis-derived mismatches in the original double stranded target nucleic acid molecule. Alternatively, in some embodiments, sites of sequence disagreement between the two strands can be recognized as potential sites where a damaged or modified nucleotide base was present on one or both strands and was converted to a mismatch by an enzymatic process (for example a DNA polymerase, a DNA glycosylase or another nucleic acid modifying enzyme or chemical process). In some embodiments, this latter finding can be used to infer the presence of nucleic acid damage or nucleotide modification prior to the enzymatic process or chemical treatment.

In some embodiments, and in accordance with aspects of the present technology, sequencing reads generated from the Duplex Sequencing steps discussed herein can be further filtered to eliminate sequencing reads from DNA-damaged molecules (e.g., damaged during storage, shipping, during or following tissue or blood extraction, during or following library preparation, etc.). For example, DNA repair or modification enzymes, such as Uracil-DNA Glycosylase (UDG), Formamidopyrimidine DNA glycosylase (FPG), and 8-oxoguanine DNA glycosylase (OGG1), can be utilized to eliminate or correct DNA damage (e.g., in vitro DNA damage or in vivo damage). These DNA repair enzymes, for example, are glycoslyases that remove damaged bases from DNA. For example, UDG removes uracil that results from cytosine deamination (caused by spontaneous hydrolysis of cytosine) and FPG removes 8-oxo-guanine (e.g., a common DNA lesion that results from reactive oxygen species). FPG also has lyase activity that can generate a 1 base gap at abasic sites. Such abasic sites will generally subsequently fail to amplify by PCR, for example, because the polymerase fails to copy the template. Accordingly, the use of such DNA damage repair/elimination enzymes can effectively remove damaged DNA that doesn't have a true mutation but might otherwise be undetected as an error following sequencing and duplex sequence analysis. Although an error due to a damaged base can often be corrected by Duplex Sequencing in rare cases a complementary error could theoretically occur at the same position on both strands, thus, reducing error-increasing damage can reduce the probability of artifacts. Furthermore, during library preparation certain fragments of DNA to be sequenced may be single-stranded from their source or from processing steps (for example, mechanical DNA shearing). These regions are typically converted to double stranded DNA during an "end repair" step known in the art, whereby a DNA polymerase and nucleoside substrates are added to a DNA sample to extend 5' recessed ends. A mutagenic site of DNA damage in the single-stranded portion of the DNA being copied (i.e. single-stranded 5' overhang at one or both ends of the DNA duplex or internal single-stranded nicks or gaps) can cause an error during the fill-in reaction that could render a single-stranded mutation, synthesis error or site of nucleic acid damage into a double-stranded form that could be misinterpreted in the final duplex consensus sequence as a true mutation whereby the true mutation was present in the original double stranded nucleic acid molecule, when, in fact, it was not. This scenario, termed "pseudo-duplex", can be reduced or prevented by use of such damage destroying/repair enzymes. In other embodiments this occurrence can be reduced or eliminated through use of strategies to destroy or prevent single-stranded portions of the original duplex molecule to form (e.g. use of certain enzymes being used to fragment the original double stranded nucleic acid material rather than mechanical shearing or certain other enzymes that may leave nicks or gaps). In other embodiments use of processes to eliminate single-stranded portions of original double-stranded nucleic acids (e.g. single-stand specific nucleases such as S1 nuclease or mung bean nuclease) can be utilized for a similar purpose.

In further embodiments, sequencing reads generated from the Duplex Sequencing steps discussed herein can be further filtered to eliminate false mutations by trimming ends of the reads most prone to pseudoduplex artifacts. For example, DNA fragmentation can generate single strand portions at the terminal ends of double-stranded molecule. These single-stranded portions can be filled in (e.g., by Klenow or T4 polymerase) during end repair. In some instances, polymerases make copy mistakes in these end repaired regions leading to the generation of "pseudoduplex molecules." These artifacts of library preparation can incorrectly appear to be true mutations once sequenced. These errors, as a result of end repair mechanisms, can be eliminated or reduced from analysis post-sequencing by trimming the ends of the sequencing reads to exclude any mutations that may have occurred in higher risk regions, thereby reducing the number of false mutations. In one embodiment, such trimming of sequencing reads can be accomplished automatically (e.g., a normal process step). In another embodiment, a mutant frequency can be assessed for fragment end regions and if a threshold level of mutations is observed in the fragment end regions, sequencing read trimming can be performed before generating a double-strand consensus sequence read of the DNA fragments.

By way of specific example, in some embodiments, provided herein are methods of generating an error-corrected sequence read of a double-stranded target nucleic acid material, including the step of ligating a double-stranded target nucleic acid material to at least one adapter sequence, to form an adapter-target nucleic acid material complex, wherein the at least one adapter sequence comprises (a) a degenerate or semi-degenerate single molecule identifier (SMI) sequence that uniquely labels each molecule of the double-stranded target nucleic acid material, and (b) a first nucleotide adapter sequence that tags a first strand of the adapter-target nucleic acid material complex, and a second nucleotide adapter sequence that is at least partially non-complimentary to the first nucleotide sequence that tags a second strand of the adapter-target nucleic acid material complex such that each strand of the adapter-target nucleic acid material complex has a distinctly identifiable nucleotide sequence relative to its complementary strand. The method can next include the steps of amplifying each strand of the adapter-target nucleic acid material complex to produce a plurality of first strand adapter-target nucleic acid complex amplicons and a plurality of second strand adapter-target nucleic acid complex amplicons. The method can further include the steps of amplifying both the first and strands to provide a first nucleic acid product and a second nucleic acid product. The method may also include the steps of sequencing each of the first nucleic acid product and second nucleic acid product to produce a plurality of first strand sequence reads and plurality of second strand sequence reads, and confirming the presence of at least one first strand sequence read and at least one second strand sequence read. The method may further include comparing the at least one first strand sequence read with the at least one second strand sequence read, and generating an error-corrected sequence read of the double-stranded target nucleic acid material by discounting nucleotide positions that do not agree, or alternatively removing compared first and second strand sequence reads having one or more nucleotide positions where the compared first and second strand sequence reads are non-complementary.

By way of an additional specific example, in some embodiments, provided herein are methods of identifying a DNA variant from a sample including the steps of ligating both strands of a nucleic acid material (e.g., a double-stranded target DNA molecule) to at least one asymmetric adapter molecule to form an adapter-target nucleic acid material complex having a first nucleotide sequence associated with a first strand of a double-stranded target DNA molecule (e.g., a top strand) and a second nucleotide sequence that is at least partially non-complementary to the first nucleotide sequence associated with a second strand of the double-stranded target DNA molecule (e.g., a bottom strand), and amplifying each strand of the adapter-target nucleic acid material, resulting in each strand generating a distinct yet related set of amplified adapter-target nucleic acid products. The method can further include the steps of sequencing each of a plurality of first strand adapter-target nucleic acid products and a plurality of second strand adapter-target nucleic acid products, confirming the presence of at least one amplified sequence read from each strand of the adapter-target nucleic acid material complex, and comparing the at least one amplified sequence read obtained from the first strand with the at least one amplified sequence read obtained from the second strand to form a consensus sequence read of the nucleic acid material (e.g., a double-stranded target DNA molecule) having only nucleotide bases at which the sequence of both strands of the nucleic acid material (e.g., a double-stranded target DNA molecule) are in agreement, such that a variant occurring at a particular position in the consensus sequence read (e.g., as compared to a reference sequence) is identified as a true DNA variant.

In some embodiments, provided herein are methods of generating a high accuracy consensus sequence from a double-stranded nucleic acid material, including the steps of tagging individual duplex DNA molecules with an adapter molecule to form tagged DNA material, wherein each adapter molecule comprises (a) a degenerate or semi-degenerate single molecule identifier (SMI) that uniquely labels the duplex DNA molecule, and (b) first and second non-complementary nucleotide adapter sequences that distinguishes an original top strand from an original bottom strand of each individual DNA molecule within the tagged DNA material, for each tagged DNA molecule, and generating a set of duplicates of the original top strand of the tagged DNA molecule and a set of duplicates of the original bottom strand of the tagged DNA molecule to form amplified DNA material. The method can further include the steps of creating a first single strand consensus sequence (SSCS) from the duplicates of the original top strand and a second single strand consensus sequence (SSCS) from the duplicates of the original bottom strand, comparing the first SSCS of the original top strand to the second SSCS of the original bottom strand, and generating a high-accuracy consensus sequence having only nucleotide bases at which the sequence of both the first SSCS of the original top strand and the second SSCS of the original bottom strand are complimentary.

In further embodiments, provided herein are methods of detecting and/or quantifying a donor source of nucleic acid from a mixture including the steps of ligating both strands of each double-stranded target DNA molecule in a mixture to at least one asymmetric adapter molecule to form a plurality of adapter-target DNA complexes, wherein each adapter-target DNA complex has a first nucleotide sequence associated with a first strand of a double-stranded target DNA molecule and a second nucleotide sequence that is at least partially non-complementary to the first nucleotide sequence associated with a second strand of the double-stranded target DNA molecule, and for each adapter target DNA complex: amplifying each strand of the adapter-target DNA complex, resulting in each strand generating a distinct yet related set of amplified adapter-target DNA amplicons. The method can further include the steps of sequencing each of a plurality of first strand adapter-target DNA amplicons and a plurality of second strand adapter-target DNA amplicons, confirming the presence of at least one sequence read from each strand of the adapter-target DNA complex, and comparing the at least one sequence read obtained from the first strand with the at least one sequence read obtained from the second strand to detect and/or quantify nucleotide bases at which the sequence read of one strand of the double-stranded DNA molecule is in disagreement (e.g., non-complimentary) with the sequence read of the other strand of the double-stranded DNA molecule, such that site(s) of DNA damage can be detected and/or quantified. In some embodiments, the method can further include the steps of creating a first single strand consensus sequence (SSCS) from the first strand adapter-target DNA amplicons and a second single strand consensus sequence (SSCS) from the second strand adapter-target DNA amplicons, comparing the first SSCS of the original first strand to the second SSCS of the original second strand, and identifying nucleotide bases at which the sequence of the first SSCS and the second SSCS are non-complementary to detect and/or quantify a donor source of nucleic acid from the mixture.

*Single Molecule Identifier Sequences (SMIs)*

In accordance with various embodiments, provided methods and compositions include one or more SMI sequences on each strand of a nucleic acid material. The SMI can be independently carried by each of the single strands that result from a double-stranded nucleic acid molecule such that the derivative amplification products of each strand can be recognized as having come from the same original substantially unique double-stranded nucleic acid molecule after sequencing. In some embodiments, the SMI may include additional information and/or may be used in other methods for which such molecule distinguishing functionality is useful, as will be recognized by one of skill in the art. In some embodiments, an SMI element may be incorporated before, substantially simultaneously, or after adapter sequence ligation to a nucleic acid material.

In some embodiments, an SMI sequence may include at least one degenerate or semi-degenerate nucleic acid. In other embodiments, an SMI sequence may be non-degenerate. In some embodiments, the SMI can be the sequence associated with or near a fragment end of the nucleic acid molecule (e.g., randomly or semi-randomly sheared ends of ligated nucleic acid material). In some embodiments, an exogenous sequence may be considered in conjunction with the sequence corresponding to randomly or semi-randomly sheared ends of ligated nucleic acid material (e.g., DNA) to obtain an SMI sequence capable of distinguishing, for example, single DNA molecules from one another. In some embodiments, a SMI sequence is a portion of an adapter sequence that is ligated to a double-strand nucleic acid molecule. In certain embodiments, the adapter sequence comprising a SMI sequence is double-stranded such that each strand of the double-stranded nucleic acid molecule includes an SMI following ligation to the adapter sequence. In another embodiment, the SMI sequence is single-stranded before or after ligation to a double-stranded nucleic acid molecule and a complimentary SMI sequence can be generated by extending the opposite strand with a DNA polymerase to yield a complementary double-stranded SMI sequence. In other embodiments, an SMI sequence is in a single-stranded portion of the adapter (e.g., an arm of an adapter having a Y-shape). In such embodiments, the SMI can facilitate grouping of families of sequence reads derived from an original strand of a double-stranded nucleic acid molecule, and in some instances can confer relationship between original first and second strands of a double-stranded nucleic acid molecule (e.g., all or part of the SMIs maybe relatable via look up table). In embodiments where the first and second strands are labeled with different SMIs, the sequence reads from the two original strands may be related using one or more of an endogenous SMI (e.g., a fragment-specific feature such as sequence associated with or near a fragment end of the nucleic acid molecule), or with use of an additional molecular tag shared by the two original strands (e.g., a barcode in a double-stranded portion of the adapter, or a combination thereof. In some embodiments, each SMI sequence may include between about 1 to about 30 nucleic acids (e.g., 1, 2, 3, 4, 5, 8, 10, 12, 14, 16, 18, 20, or more degenerate or semi-degenerate nucleic acids).

In some embodiments, a SMI is capable of being ligated to one or both of a nucleic acid material and an adapter sequence. In some embodiments, a SMI may be ligated to at least one of a T-overhang, an A-overhang, a CG-overhang, a dehydroxylated base, and a blunt end of a nucleic acid material.

In some embodiments, a sequence of a SMI may be considered in conjunction with (or designed in accordance with) the sequence corresponding to, for example, randomly or semi-randomly sheared ends of a nucleic acid material (e.g., a ligated nucleic acid material), to obtain a SMI sequence capable of distinguishing single nucleic acid molecules from one another.

In some embodiments, at least one SMI may be an endogenous SMI (e.g., an SMI related to a shear point (e.g., a fragment end), for example, using the shear point itself or using a defined number of nucleotides in the nucleic acid material immediately adjacent to the shear point [e.g., 2, 3, 4, 5, 6, 7, 8, 9, 10 nucleotides from the shear point]). In some embodiments, at least one SMI may be an exogenous SMI (e.g., an SMI comprising a sequence that is not found on a target nucleic acid material).

In some embodiments, a SMI may be or comprise an imaging moiety (e.g., a fluorescent or otherwise optically detectable moiety). In some embodiments, such SMIs allow for detection and/or quantitation without the need for an amplification step.

In some embodiments a SMI element may comprise two or more distinct SMI elements that are located at different locations on the adapter-target nucleic acid complex.

Various embodiments of SMIs are further disclosed in International Patent Publication No. WO2017/100441, which is incorporated by reference herein in its entirety.

*Strand-Defining Element (SDE)*

In some embodiments, each strand of a double-stranded nucleic acid material may further include an element that renders the amplification products of the two single-stranded nucleic acids that form the target double-stranded nucleic acid material substantially distinguishable from each other after sequencing. In some embodiments, a SDE may be or comprise asymmetric primer sites comprised within a sequencing adapter, or, in other arrangements, sequence asymmetries may be introduced into the adapter sequences and not within the primer sequences, such that at least one position in the nucleotide sequences of a first strand target nucleic acid sequence complex and a second stand of the target nucleic acid sequence complex are different from each other following amplification and sequencing. In other embodiments, the SDE may comprise another biochemical asymmetry between the two strands that differs from the canonical nucleotide sequences A, T, C, G or U, but is converted into at least one canonical nucleotide sequence difference in the two amplified and sequenced molecules. In yet another embodiment, the SDE may be or comprise a means of physically separating the two strands before amplification, such that derivative amplification products from the first strand target nucleic acid sequence and the second strand target nucleic acid sequence are maintained in substantial physical isolation from one another for the purposes of maintaining a distinction between the two derivative amplification products. Other such arrangements or methodologies for providing an SDE function that allows for distinguishing the first and second strands may be utilized.

In some embodiments, a SDE may be capable of forming a loop (e.g., a hairpin loop). In some embodiments, a loop may comprise at least one endonuclease recognition site. In some embodiments the target nucleic acid complex may contain an endonuclease recognition site that facilitates a cleavage event within the loop. In some embodiments a loop may comprise a non-canonical nucleotide sequence. In some embodiments the contained non-canonical nucleotide may be recognizable by one or more enzyme that facilitates strand cleavage. In some embodiments the contained non-canonical nucleotide may be targeted by one or more chemical process facilitates strand cleavage in the loop. In some embodiments the loop may contain a modified nucleic acid linker that may be targeted by one or more enzymatic, chemical or physical process that facilitates strand cleavage in the loop. In some embodiments this modified linker is a photocleavable linker.

A variety of other molecular tools could serve as SMIs and SDEs. Other than shear points and DNA-based tags, single-molecule compartmentalization methods that keep paired strands in physical proximity or other non-nucleic acid tagging methods could serve the strand-relating function. Similarly, asymmetric chemical labelling of the adapter strands in a way that they can be physically separated can serve an SDE role. A recently described variation of Duplex Sequencing uses bisulfite conversion to transform naturally occurring strand asymmetries in the form of cytosine methylation into sequence differences that distinguish the two strands. Although this implementation limits the types of mutations that can be detected, the concept of capitalizing on native asymmetry is noteworthy in the context of emerging sequencing technologies that can directly detect modified nucleotides. Various embodiments of SDEs are further disclosed in International Patent Publication No. WO2017/100441, which is incorporated by reference in its entirety.

*Adapters and Adapter Sequences*

In various arrangements, adapter molecules that comprise SMIs (e.g., molecular barcodes), SDEs, primer sites, flow cell sequences and/or other features are contemplated for use with many of the embodiments disclosed herein. In some embodiments, provided adapters may be or comprise one or more sequences complimentary or at least partially complimentary to PCR primers (e.g., primer sites) that have at least one of the following properties: 1) high target specificity; 2) capable of being multiplexed; and 3) exhibit robust and minimally biased amplification.

In some embodiments, adapter molecules can be "Y"-shaped, "U"-shaped, "hairpin" shaped, have a bubble (e.g., a portion of sequence that is non-complimentary), or other features. In other embodiments, adapter molecules can comprise a "Y"-shape, a "U"-shaped, a "hairpin" shaped, or a bubble. Certain adapters may comprise modified or non-standard nucleotides, restriction sites, or other features for manipulation of structure or function in vitro. Adapter molecules may ligate to a variety of nucleic acid material having a terminal end. For example, adapter molecules can be suited to ligate to a T-overhang, an A-overhang, a CG-overhang, a multiple nucleotide overhang, a dehydroxylated base, a blunt end of a nucleic acid material and the end of a molecule were the 5' of the target is dephosphorylated or otherwise blocked from traditional ligation. In other embodiments the adapter molecule can contain a dephosphorylated or otherwise ligation-preventing modification on the 5' strand at the ligation site. In the latter two embodiments such strategies may be useful for preventing dimerization of library fragments or adapter molecules.

An adapter sequence can mean a single-strand sequence, a double-strand sequence, a complimentary sequence, a non-complimentary sequence, a partial complimentary sequence, an asymmetric sequence, a primer binding sequence, a flow-cell sequence, a ligation sequence or other sequence provided by an adapter molecule. In particular embodiments, an adapter sequence can mean a sequence used for amplification by way of compliment to an oligonucleotide.

In some embodiments, provided methods and compositions include at least one adapter sequence (e.g., two adapter sequences, one on each of the 5' and 3' ends of a nucleic acid material). In some embodiments, provided methods and compositions may comprise 2 or more adapter sequences (e.g., 3, 4, 5, 6, 7, 8, 9, 10 or more). In some embodiments, at least two of the adapter sequences differ from one another (e.g., by sequence). In some embodiments, each adapter sequence differs from each other adapter sequence (e.g., by sequence). In some embodiments, at least one adapter sequence is at least partially non-complementary to at least a portion of at least one other adapter sequence (e.g., is non-complementary by at least one nucleotide).

In some embodiments, an adapter sequence comprises at least one non-standard nucleotide. In some embodiments, a non-standard nucleotide is selected from an abasic site, a uracil, tetrahydrofuran, 8-oxo-7,8-dihydro-2'deoxyadenosine (8-oxo-A), 8-oxo-7,8-dihydro-2'-deoxyguanosine (8-oxo-G), deoxyinosine, 5'nitroindole, 5-Hydroxymethyl-2' -deoxycytidine, iso-cytosine, 5 '-methyl-isocytosine, or isoguanosine, a methylated nucleotide, an RNA nucleotide, a ribose nucleotide, an 8-oxo-guanine, a photocleavable linker, a biotinylated nucleotide, a desthiobiotin nucleotide, a thiol modified nucleotide, an acrydite modified nucleotide an iso-dC, an iso dG, a 2'-O-methyl nucleotide, an inosine nucleotide Locked Nucleic Acid, a peptide nucleic acid, a 5 methyl dC, a 5-bromo deoxyuridine, a 2,6-Diaminopurine, 2-Aminopurine nucleotide, an abasic nucleotide, a 5-Nitroindole nucleotide, an adenylated nucleotide, an azide nucleotide, a digoxigenin nucleotide, an I-linker, an 5' Hexynyl modified nucleotide, an 5-Octadiynyl dU, photocleavable spacer, a non-photocleavable spacer, a click chemistry compatible modified nucleotide, and any combination thereof.

In some embodiments, an adapter sequence comprises a moiety having a magnetic property (i.e., a magnetic moiety). In some embodiments this magnetic property is paramagnetic. In some embodiments where an adapter sequence comprises a magnetic moiety (e.g., a nucleic acid material ligated to an adapter sequence comprising a magnetic moiety), when a magnetic field is applied, an adapter sequence comprising a magnetic moiety is substantially separated from adapter sequences that do not comprise a magnetic moiety (e.g., a nucleic acid material ligated to an adapter sequence that does not comprise a magnetic moiety).

In some embodiments, at least one adapter sequence is located 5' to a SMI. In some embodiments, at least one adapter sequence is located 3' to a SMI.

In some embodiments, an adapter sequence may be linked to at least one of a SMI and a nucleic acid material via one or more linker domains. In some embodiments, a linker domain may be comprised of nucleotides. In some embodiments, a linker domain may include at least one modified nucleotide or non-nucleotide molecules (for example, as described elsewhere in this disclosure). In some embodiments, a linker domain may be or comprise a loop.

In some embodiments, an adapter sequence on either or both ends of each strand of a double-stranded nucleic acid material may further include one or more elements that provide a SDE. In some embodiments, a SDE may be or comprise asymmetric primer sites comprised within the adapter sequences.

In some embodiments, an adapter sequence may be or comprise at least one SDE and at least one ligation domain (i.e., a domain amendable to the activity of at least one ligase, for example, a domain suitable to ligating to a nucleic acid material through the activity of a ligase). In some embodiments, from 5' to 3', an adapter sequence may be or comprise a primer binding site, a SDE, and a ligation domain.

Various methods for synthesizing Duplex Sequencing adapters have been previously described in, e.g., U.S. Patent No. 9,752,188, International Patent Publication No. WO2017/100441, and International Patent Application No. PCT/US18/59908 (filed November 8, 2018), all of which are incorporated by reference herein in their entireties.

*Primers*

In some embodiments, one or more PCR primers that have at least one of the following properties: 1) high target specificity; 2) capable of being multiplexed; and 3) exhibit robust and minimally biased amplification are contemplated for use in various embodiments in accordance with aspects of the present technology. A number of prior studies and commercial products have designed primer mixtures satisfying certain of these criteria for conventional PCR-CE. However, it has been noted that these primer mixtures are not always optimal for use with MPS. Indeed, developing highly multiplexed primer mixtures can be a challenging and time-consuming process. Conveniently, both Illumina and Promega have recently developed multiplex compatible primer mixtures for the Illumina platform that show robust and efficient amplification of a variety of standard and non-standard STR and SNP loci. Because these kits use PCR to amplify their target regions prior to sequencing, the 5'-end of each read in paired-end sequencing data corresponds to the 5'-end of the PCR primers used to amplify the DNA. In some embodiments, provided methods and compositions include primers designed to ensure uniform amplification, which may entail varying reaction concentrations, melting temperatures, and minimizing secondary structure and intra/inter-primer interactions. Many techniques have been described for highly multiplexed primer optimization for MPS applications, such as, for example, techniques are often known as ampliseq methods, which are described in the art.

*Amplification*

Provided methods and compositions, in various embodiments, make use of, or are of use in, at least one amplification step wherein a nucleic acid material (or portion thereof, for example, a specific target region or locus) is amplified to form an amplified nucleic acid material (e.g., some number of amplicon products).

In some embodiments, amplifying a nucleic acid material includes a step of amplifying nucleic acid material derived from each of a first and second nucleic acid strand from an original double-stranded nucleic acid material using at least one single-stranded oligonucleotide at least partially complementary to a sequence present in a first adapter sequence such that a SMI sequence is at least partially maintained. An amplification step further includes employing a second single-stranded oligonucleotide to amplify each strand of interest, and such second single-stranded oligonucleotide can be (a) at least partially complementary to a target sequence of interest, or (b) at least partially complementary to a sequence present in a second adapter sequence such that the at least one single-stranded oligonucleotide and a second single-stranded oligonucleotide are oriented in a manner to effectively amplify the nucleic acid material.

In some embodiments, amplifying nucleic acid material in a sample can include amplifying nucleic acid material in "tubes" (e.g., PCR tubes), in emulsion droplets, microchambers, and other examples described above or other known vessels.

In some embodiments, at least one amplifying step includes at least one primer that is or comprises at least one non-standard nucleotide. In some embodiments, a non-standard nucleotide is selected from a uracil, a methylated nucleotide, an RNA nucleotide, a ribose nucleotide, an 8-oxo-guanine, a biotinylated nucleotide, a locked nucleic acid, a peptide nucleic acid, a high-Tm nucleic acid variant, an allele discriminating nucleic acid variant, any other nucleotide or linker variant described elsewhere herein and any combination thereof.

While any application-appropriate amplification reaction is contemplated as compatible with some embodiments, by way of specific example, in some embodiments, an amplification step may be or comprise a polymerase chain reaction (PCR), rolling circle amplification (RCA), multiple displacement amplification (MDA), isothermal amplification, polony amplification within an emulsion, bridge amplification on a surface, the surface of a bead or within a hydrogel, and any combination thereof.

In some embodiments, amplifying a nucleic acid material includes use of single-stranded oligonucleotides at least partially complementary to regions of the adapter sequences on the 5' and 3' ends of each strand of the nucleic acid material. In some embodiments, amplifying a nucleic acid material includes use of at least one single-stranded oligonucleotide at least partially complementary to a target region or a target sequence of interest (e.g., a genomic sequence, a mitochondrial sequence, a plasmid sequence, a synthetically produced target nucleic acid, etc.) and a single-stranded oligonucleotide at least partially complementary to a region of the adapter sequence (e.g., a primer site).

In general, robust amplification, for example PCR amplification, can be highly dependent on the reaction conditions. Multiplex PCR, for example, can be sensitive to buffer composition, monovalent or divalent cation concentration, detergent concentration, crowding agent (i.e. PEG, glycerol, etc.) concentration, primer concentrations, primer Tms, primer designs, primer GC content, primer modified nucleotide properties, and cycling conditions (i.e. temperature and extension times and rate of temperature changes). Optimization of buffer conditions can be a difficult and time-consuming process. In some embodiments, an amplification reaction may use at least one of a buffer, primer pool concentration, and PCR conditions in accordance with a previously known amplification protocol. In some embodiments, a new amplification protocol may be created, and/or an amplification reaction optimization may be used. By way of specific example, in some embodiments, a PCR optimization kit may be used, such as a PCR Optimization Kit from Promega®, which contains a number of pre-formulated buffers that are partially optimized for a variety of PCR applications, such as multiplex, real-time, GC-rich, and inhibitor-resistant amplifications. These pre-formulated buffers can be rapidly supplemented with different $Mg^{2+}$ and primer concentrations, as well as primer pool ratios. In addition, in some embodiments, a variety of cycling conditions (e.g., thermal cycling) may be assessed and/or used. In assessing whether or not a particular embodiment is appropriate for a particular desired application, one or more of specificity, allele coverage ratio for heterozygous loci, interlocus balance, and depth, among other aspects may be assessed. Measurements of amplification success may include DNA sequencing of the products, evaluation of products by gel or capillary electrophoresis or HPLC or other size separation methods followed by fragment visualization, melt curve analysis using double-stranded nucleic acid binding dyes or fluorescent probes, mass spectrometry or other methods known in the art.

In accordance with various embodiments, any of a variety of factors may influence the length of a particular amplification step (e.g., the number of cycles in a PCR reaction, etc.). For example, in some embodiments, a provided nucleic acid material may be compromised or otherwise suboptimal (e.g. degraded and/or contaminated). In such case, a longer amplification step may be helpful in ensuring a desired product is amplified to an acceptable degree. In some embodiments an amplification step may provide an average of 3 to 10 sequenced PCR copies from each starting DNA molecule, though in other embodiments, only a single copy of each of a first strand and second strand are required. Without wishing to be held to a particular theory, it is possible that too many or too few PCR copies could result in reduced assay efficiency and, ultimately, reduced depth. Generally, the number of nucleic acid (e.g., DNA) fragments used in an amplification (e.g., PCR) reaction is a primary adjustable variable that can dictate the number of reads that share the same SMI/barcode sequence.

*Nucleic Acid Material*

Types

In accordance with various embodiments, any of a variety of nucleic acid material may be used. In some embodiments, nucleic acid material may comprise at least one modification to a polynucleotide within the canonical sugar-phosphate backbone. In some embodiments, nucleic acid material may comprise at least one modification within any base in the nucleic acid material. For example, by way of non-limiting example, in some embodiments, the nucleic acid material is or comprises at least one of double-stranded DNA, single-stranded DNA, double-stranded RNA, single-stranded RNA, peptide nucleic acids (PNAs), locked nucleic acids (LNAs).

Modifications

In accordance with various embodiments, nucleic acid material may receive one or more modifications prior to, substantially simultaneously, or subsequent to, any particular step, depending upon the application for which a particular provided method or composition is used.

In some embodiments, a modification may be or comprise repair of at least a portion of the nucleic acid material. While any application-appropriate manner of nucleic acid repair is contemplated as compatible with some embodiments, certain exemplary methods and compositions therefore are described below and in the Examples.

By way of non-limiting example, in some embodiments, DNA repair enzymes, such as Uracil-DNA Glycosylase (UDG), Formamidopyrimidine DNA glycosylase (FPG), and 8-oxoguanine DNA glycosylase (OGG1), can be utilized to correct DNA damage (e.g., in vitro DNA damage). As discussed above, these DNA repair enzymes, for example, are glycoslyases that remove damaged bases from DNA. For example, UDG removes uracil that results from cytosine deamination (caused by spontaneous hydrolysis of cytosine) and FPG removes 8-oxo-guanine (e.g., most common DNA lesion that results from reactive oxygen species). FPG also has lyase activity that can generate 1 base gap at abasic sites. Such abasic sites will subsequently fail to amplify by PCR, for example, because the polymerase fails copy the template. Accordingly, the use of such DNA damage repair enzymes can effectively remove damaged DNA that doesn't have a true mutation, but might otherwise be undetected as an error following sequencing and duplex sequence analysis.

As discussed above, in further embodiments, sequencing reads generated from the processing steps discussed herein can be further filtered to eliminate false mutations by trimming ends of the reads most prone to artifacts. For example, DNA fragmentation can generate single-strand portions at the terminal ends of double-stranded molecules. These single-stranded portions can be filled in (e.g., by Klenow) during end repair. In some instances, polymerases make copy mistakes in these end-repaired regions leading to the generation of "pseudoduplex molecules." These artifacts can appear to be true mutations once sequenced. These errors, as a result of end repair mechanisms, can be eliminated from analysis post-sequencing by trimming the ends of the sequencing reads to exclude any mutations that may have occurred, thereby reducing the number of false mutations. In some embodiments, such trimming of sequencing reads can be accomplished automatically (e.g., a normal process step). In some embodiments, a mutant frequency can be assessed for fragment end regions and if a threshold level of mutations is observed in the fragment end regions, sequencing read trimming can be performed before generating a double-strand consensus sequence read of the DNA fragments.

The high degree of error correction provided by the strand-comparison technology of Duplex Sequencing reduces sequencing errors of double-stranded nucleic acid molecules by multiple orders of magnitude as compared with standard next-generation sequencing methods. This reduction in errors improves the accuracy of sequencing in nearly all types of sequences but can be particularly well suited to biochemically challenging sequences that are well known in the art to be particularly error prone. One non-limiting example of such type of sequence is homopolymers or other microsatellites/short-tandem repeats. Another non-limiting example of error prone sequences that benefit from Duplex Sequencing error correction are molecules that have been damaged, for example, by heating, radiation, mechanical stress, or a variety of chemical exposures which creates chemical adducts that are error prone during copying by one or more nucleotide polymerases. In further embodiments, Duplex Sequencing can also be used for the accurate detection of minority sequence variants among a population of double-stranded nucleic acid molecules. One non-limiting example of this application is detection of a small number of DNA molecules derived from a cancer, among a larger number of unmutated molecules from non-cancerous tissues within a subject. Another non-limiting application for rare variant detection by Duplex Sequencing is forensic detection of the DNA from one individual intermixed at low abundance with the DNA of another individual of a different genotype.

III. Selected Embodiments of Methods for Resolving Nucleic Acid Mixtures and Mixed Cell Populations

The problem of identifying and measuring genotypes in a mixed sample occurs in diverse fields, including forensics and cell-based therapies (e.g., stem cell transplants). In accordance with aspects of the present technology, Duplex Sequencing can be used use deconvolve and identify source-specific genotypes present in nucleic mixtures. In particular embodiments, Duplex Sequencing is used to identify microhaplotypes present on individual nucleic acid molecules present in biological sample mixtures. In some embodiments, the microhaplotypes are used to deconvolve complex mixtures of multiple genotypes.

Microhaplotypes are small genomic loci comprising two or more non-redundant genomic DNA SNPs within a relatively short distance from each other (e.g., <200 nucleotides, <250 nucleotides, < 300 nucleotides, < 350 nucleotides or longer) that are generally defined as groups of polymorphic loci that can be comprised within the same read or read pair or a sequencing read. Genotyping can be achieved using next generation DNA sequencing (NGS), sanger sequencing, massively parallel sequencing, nanopore sequencing, single molecule sequencing, sequencing by hybridization or other related methods. The length of a region is not purely defined by a length of nucleotides but, rather, a sequence that can be genotyped as a "phased" unit on whatever genotyping platform is used. With many contemporary NGS platforms such as, for example, manufactured by Illumina, Inc. (San Diego, CA, USA) or Thermo Fisher Scientific, Inc. (Waltham, MA, USA), read lengths/paired read lengths are on the order of dozens to hundreds of nucleotides. Such lengths are practical sizes for microhaplotypes with these platforms. For longer sequence read technologies, such as sequencers manufactured by, for example, Pacific Biosciences of California, Inc. (Menlo Park, CA, USA) and Oxford Nanopore Technologies, Ltd. (Oxford, UK), the practically usable length of microhaplotypes is considerably longer. For the examples below, microhaplotypes on the order of dozens to several hundred nucleotides in length are shown for the sake of clarity and practicality, but this should not be construed as a general limitation. Microhaplotypes can have from 3 to 14 or more distinct alleles or allelic combinations. Such multi-allelic loci can be especially informative in the context of many-component mixtures. Duplex Sequencing can resolve rare variants that are concealed by the error rates of standard next-generation sequencing (NGS) and single-strand consensus sequencing methods in a manner that allows for the detection of microhaplotypes even when particular genotypes are present at very low levels within a biological mixture. A given microhaplotype may have as few as zero "informative" individual polymorphisms for a given mixture (i.e. there are no differences between the microhaplotypes between the DNA molecules in the mixture from different individuals), or several individual polymorphisms (e.g., at least about 2, 3, 4, 5, 6, 7, 8, 9, 10, 11. 12, 15, 20, 24, or more). The number of composite microhaplotype genotypes may be several (e.g., greater than 10, greater than 20, greater than 30, etc.) but, similarly may or may not be informative for a given mixture if the constituent subjects in the mixture are not sufficiently genetically different.

In some embodiments, aspects of the present technology are used to detect and quantify genotypes present within a biological sample at a level of about 1/100,000 parts in a mixture. Other aspects of the present technology can accurately quantify mixing proportions from multiple known genotypes (e.g., about 5, about 10, about 12, about 15, about 20, about 25, about 30, about 35, about 40, about 45, about 50, etc.). In other embodiments, aspects of the present technology can be used deconvolve mixtures of at least about 5 (e.g., about 2, about 3, about 4, about 5, about 6, etc.) unknown genotypes (e.g., no empirical or *a priori* knowledge of numbers or which genetic sources are present in a biological sample. Still further aspects of the present technology can be used to partially recover genotypes of extremely low-abundance sources in a mixture. For example, such embodiments are useful in forensic applications, microchimerism analysis (e.g., fetal microchimerism), measurement of engrafted cells in a host (e.g. after stem cell transplant), and others. Additional embodiments are directed to identification of subjects from a database in complex mixtures (e.g., up to at least about 8 individual genotypes).

IV. Embodiments of Systems and Computing Environments for Deconvolution of Complex Mixtures of Genotypes

*Suitable Computing Environments*

The following discussion provide a general description of a suitable computing environment in which aspects of the disclosure can be implemented. Although not required, aspects and embodiments of the disclosure will be described in the general context of computer-executable instructions, such as routines executed by a general-purpose computer, e.g., a server or personal computer. Those skilled in the relevant art will appreciate that the disclosure can be practiced with other computer system configurations, including Internet appliances, hand-held devices, wearable computers, cellular or mobile phones, multi-processor systems, microprocessor-based or programmable consumer electronics, set-top boxes, network PCs, mini-computers, mainframe computers and the like. The disclosure can be embodied in a special purpose computer or data processor that is specifically programmed, configured or constructed to perform one or more of the computer-executable instructions explained in detail below. Indeed, the term "computer", as used generally herein, refers to any of the above devices, as well as any data processor.

The disclosure can also be practiced in distributed computing environments, where tasks or modules are performed by remote processing devices, which are linked through a communications network, such as a Local Area Network ("LAN"), Wide Area Network ("WAN") or the Internet. In a distributed computing environment, program modules or sub-routines may be located in both local and remote memory storage devices. Aspects of the disclosure described below may be stored or distributed on computer-readable media, including magnetic and optically readable and removable computer discs, stored as firmware in chips (e.g., EEPROM chips), as well as distributed electronically over the Internet or over other networks (including wireless networks). Those skilled in the relevant art will recognize that portions of the disclosure may reside on a server computer, while corresponding portions reside on a client computer. Data structures and transmission of data particular to aspects of the disclosure are also encompassed within the scope of the disclosure.

Embodiments of computers, such as a personal computer or workstation, can comprise one or more processors coupled to one or more user input devices and data storage devices. A computer can also be coupled to at least one output device such as a display device and one or more optional additional output devices (e.g., printer, plotter, speakers, tactile or olfactory output devices, etc.). The computer may be coupled to external computers, such as via an optional network connection, a wireless transceiver, or both.

Various input devices may include a keyboard and/or a pointing device such as a mouse. Other input devices are possible such as a microphone, joystick, pen, touch screen, scanner, digital camera, video camera, and the like. Further input devices can include sequencing machine(s) (e.g., massively parallel sequencer), fluoroscopes, and other laboratory equipment, etc. Suitable data storage devices may include any type of computer-readable media that can store data accessible by the computer, such as magnetic hard and floppy disk drives, optical disk drives, magnetic cassettes, tape drives, flash memory cards, digital video disks (DVDs), Bernoulli cartridges, RAMs, ROMs, smart cards, etc. Indeed, any medium for storing or transmitting computer-readable instructions and data may be employed, including a connection port to or node on a network such as a local area network (LAN), wide area network (WAN) or the Internet.

Aspects of the disclosure may be practiced in a variety of other computing environments. For example, a distributed computing environment with a network interface can include one or more user computers in a system where they may include a browser program module that permits the computer to access and exchange data with the Internet, including web sites within the World Wide Web portion of the Internet. User computers may include other program modules such as an operating system, one or more application programs (e.g., word processing or spread sheet applications), and the like. The computers may be general-purpose devices that can be programmed to run various types of applications, or they may be single-purpose devices optimized or limited to a particular function or class of functions. More importantly, while shown with network browsers, any application program for providing a graphical user interface to users may be employed, as described in detail below; the use of a web browser and web interface are only used as a familiar example here.

At least one server computer, coupled to the Internet or World Wide Web ("Web"), can perform much or all of the functions for receiving, routing and storing of electronic messages, such as web pages, data streams, audio signals, and electronic images that are described herein. While the Internet is shown, a private network, such as an intranet may indeed be preferred in some applications. The network may have a client-server architecture, in which a computer is dedicated to serving other client computers, or it may have other architectures such as a peer-to-peer, in which one or more computers serve simultaneously as servers and clients. A database or databases, coupled to the server computer(s), can store much of the web pages and content exchanged between the user computers. The server computer(s), including the database(s), may employ security measures to inhibit malicious attacks on the system, and to preserve integrity of the messages and data stored therein (e.g., firewall systems, secure socket layers (SSL), password protection schemes, encryption, and the like).

A suitable server computer may include a server engine, a web page management component, a content management component and a database management component, among other features. The server engine performs basic processing and operating system level tasks. The web page management component handles creation and display or routing of web pages. Users may access the server computer by means of a URL associated therewith. The content management component handles most of the functions in the embodiments described herein. The database management component includes storage and retrieval tasks with respect to the database, queries to the database, read and write functions to the database and storage of data such as video, graphics and audio signals.

Many of the functional units described herein have been labeled as modules, in order to more particularly emphasize their implementation independence. For example, modules may be implemented in software for execution by various types of processors. An identified module of executable code may, for instance, comprise one or more physical or logical blocks of computer instructions which may, for instance, be organized as an object, procedure, or function. The identified blocks of computer instructions need not be physically located together but may comprise disparate instructions stored in different locations which, when joined logically together, comprise the module and achieve the stated purpose for the module.

A module may also be implemented as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices or the like.

A module of executable code may be a single instruction, or many instructions, and may even be distributed over several different code segments, among different programs, and across several memory devices. Similarly, operational data may be identified and illustrated herein within modules and may be embodied in any suitable form and organized within any suitable type of data structure. The operational data may be collected as a single data set or may be distributed over different locations including over different storage devices, and may exist, at least partially, merely as electronic signals on a system or network.

*System for Deconvolution of Nucleic Acid Mixtures*

The present invention further comprises a system (e.g. a networked computer system, a high throughput automated system, etc.) for processing a biological sample comprising a nucleic acid mixture, and transmitting the sequencing data via a wired or wireless network to a server to determine the sample's error-corrected sequence reads (e.g., duplex sequence reads, duplex consensus sequence, etc.), genotype identification, quantification of individual/attributable genotypes, etc.

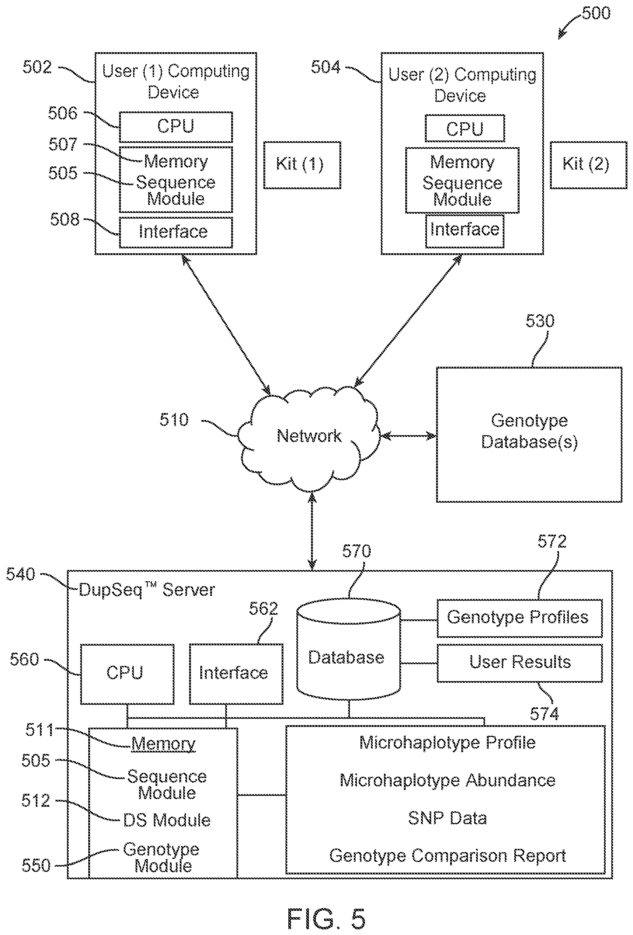

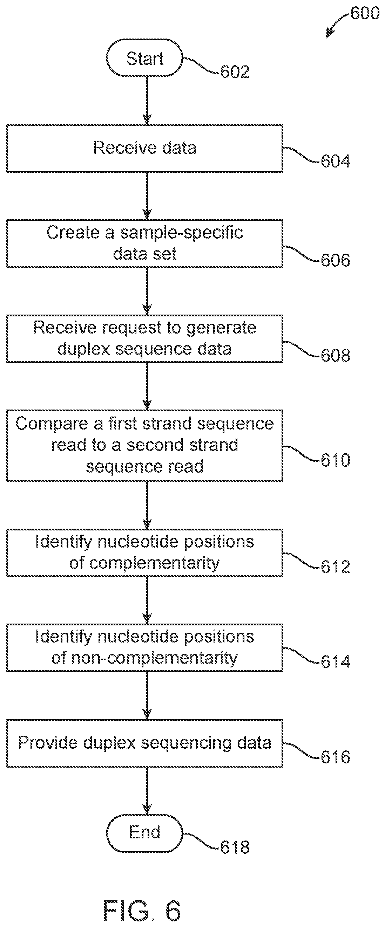

As described in additional detail below, and with respect to the embodiment illustrated in FIG. 5, a computerized system for deconvolution of nucleic acids in a mixture comprises: (1) a server (e.g., a remote server, or locally stored server); (2) a plurality of user electronic computing devices able to generate and/or transmit sequencing data; (3) optionally, a database with known genotypes and associated information (optional); and (4) a wired or wireless network for transmitting electronic communications between the electronic computing devices, database, and the server. The server further comprises: (a) a database storing deconvolution record results, and records of genotype profiles (e.g. microhaplotype profiles etc.); (b) one or more processors communicatively coupled to a memory; and one or more non-transitory computer-readable storage devices or medium comprising instructions for processor(s), wherein said processors are configured to execute said instructions to perform operations comprising one or more of the steps described in FIGS. 6-8.

In one embodiment, the present technology further comprises, a non-transitory computer-readable storage media comprising instructions that, when executed by one or more processors, performs methods for determining the presence of one or more genotypes in a mixture, the quantification of each identified genotype in the mixture, the identity of a subject/individual from a database who's genetic material is present in the mixture, quantify a mixing proportion from multiple known genotypes, deconvolve mixtures of multiple unknown genotypes, and the like. In particular embodiments, the methods can include one or more of the steps described in FIGS. 6-8.

Additional aspects of the present technology are directed to computerized methods for determining the presence of one or more genotypes in a mixture, the quantification of each identified genotype in the mixture, the identity of a subject/individual from a database who's genetic material is present in the mixture, quantify a mixing proportion from multiple known genotypes, deconvolve mixtures of multiple unknown genotypes, and the like. In particular embodiments, the methods can include one or more of the steps described in FIGS. 6-8.

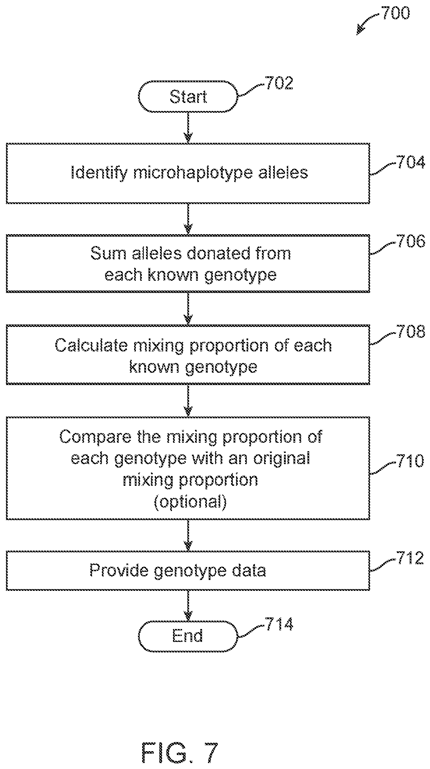

FIG. **5** is a block diagram of a computer system 500 with a computer program product **550** installed thereon and for use with the methods disclosed herein to deconvolve nucleic acid mixtures from biological samples. Although FIG. **5** illustrates various computing system components, it is contemplated that other or different components known to those of ordinary skill in the art, such as those discussed above, can provide a suitable computing environment in which aspects of the disclosure can be implemented. FIG. **6** is a flow diagram illustrating a routine for providing Duplex Sequencing consensus sequence data in accordance with an embodiment of the present technology. FIGS. **7-8** are flow diagrams illustrating various routines for identifying and/or quantifying genotypes from nucleic acid mixtures. In accordance with aspects of the present technology, methods described with respect to FIGS. **7-8** can provide sample data including, for example, genotypes present in a sample, including the number of independent biological sources represented within the sample, and quantification of each biological source present in a biological mixture, and information derived from comparison of sample data to data sets of known genotypes (including databases comprising individual subject's genotypes).

As illustrated in FIG. **5,** the computer system **500** can comprise a plurality of user computing devices **502, 504;** a wired or wireless network **510** and a server ("DupSeq™" server) **540** comprising processors to analyze microhaplotypes and deconvolve nucleic acid mixtures into individual genotypes. In embodiments, user computing devices **502, 504** can be used to generate and/or transmit sequencing data. In one embodiment, users of computing devices **502, 504** may be those performing other aspects of the present technology such as Duplex Sequencing method steps of biological samples for deconvolution of nucleic acid mixtures comprising more than one biological source of genetic material. In one example, users of computing devices **502, 504** perform certain Duplex Sequencing method steps with a kit (**1, 2**) comprising reagents and/or adapters, in accordance with an embodiment of the present technology, to interrogate biological samples.

As illustrated, each user computing device **502, 504** includes at least one central processing unit **506,** a memory **507** and a user and network interface **508.** In an embodiment, the user devices **502, 504** comprise a desktop, laptop, or a tablet computer.

Although two user computing devices **502, 504** are depicted, it is contemplated that any number of user computing devices may be included or connected to other components of the system **500.** Additionally, computing devices **502, 504** may also be representative of a plurality of devices and software used by User (1) and User (2) to amplify and sequence the samples. For example, a computing device may a sequencing machine (e.g., Illumina HiSeq™, Ion Torrent™ PGM, ABI SOLiD™ sequencer, PacBio RS, Helicos Heliscope™, etc.), a real-time PCR machine (e.g., ABI 7900, Fluidigm BioMark™, etc.), a microarray instrument, etc.

In addition to the above described components, the system **500** may further comprise a database **530** for storing genotype profiles and associated information. For example, the database **530,** which can be accessible by the server **540,** can comprise records or collections of microhaplotypes, genotypes of known subjects, and mixing proportions of starting material (e.g., mixtures of cells). In a particular example, the database **530** can be a third-party database comprising genotype profiles **532.** For example, various forensic databases comprising genotypes of known individuals can be queried for particular applications. In another embodiment, the database can be a standalone database **530** (private or not private) hosted separately from server **540,** or a database can be hosted on the server **540,** such as database **570,** that comprises empirically-derived genotype profiles **572.** In some embodiments, as the system **500** is used to generate new genotype profiles, the data generated from use of the system **500** and associated methods (e.g., methods described herein and, for example, in FIGS. **6-8**), can be uploaded to the database **530** and/or **570** so additional genotype profiles **532, 572** can be created for future comparison activities.

The server **540** can be configured to receive, compute and analyze sequencing data (e.g., raw sequencing files) and related information from user computing devices **502, 504** via the network **510.** Sample-specific raw sequencing data can be computed locally using a computer program product/module (Sequence Module **505**) installed on devices **502, 504,** or accessible from the server **540** via the network **510,** or using other sequencing software well known in the art. The raw sequence data can then be transmitted via the network **510** to the server **540** and user results **574** can be stored in database **570.** The server **540** also comprises program product/module "DS Module" **512** configured to receive the raw sequencing data from the database **570** and configured to computationally generate error corrected double-stranded sequence reads using, for example, Duplex Sequencing techniques disclosed herein. While DS Module **512** is shown on server **540,** one of ordinary skill in the art would recognize that DS Module **512** can alternatively, be hosted at operated at devices **502, 504** or on another server (not shown).

The server **540** can comprise at least one central processing unit (CPU) **560,** a user and a network interface **562** (or server-dedicated computing device with interface connected to the server), a database **570,** such as described above, with a plurality of computer files/records to store genotype profiles of known and unknown biological sources **572,** and files/records to store results (e.g., raw sequencing data, Duplex Sequencing data, microhaplotype analysis, genotype analysis, etc.) for tested samples **574.** Server **540** further comprises a computer memory **511** having stored thereon the Genotype Computer Program Product (Genotype Module) **550,** in accordance with aspects of the present technology.

Computer program product/module **550** is embodied in a non-transitory computer readable medium that, when executed on a computer (e.g. server **540**), performs steps of the methods disclosed herein for detecting and identifying microhaplotypes, resolving mixtures into individual genotypes, and/or quantifying the same. Another aspect of the present disclosure comprises the computer program product/module 550 comprising a non-transitory computer-usable medium having computer-readable program codes or instructions embodied thereon for enabling a processor to carry out genotype analysis (e.g. compute microhaplotypes, quantify identified microhaplotypes, resolve mixtures into contributing biological sources, genotype comparison reports, etc.). These computer program instructions may be loaded onto a computer or other programmable apparatus to produce a machine, such that the instructions which execute on the computer or other programmable apparatus create means for implementing the functions or steps described herein. These computer program instructions may also be stored in a computer-readable memory or medium that can direct a computer or other programmable apparatus to function in a particular manner, such that the instructions stored in the computer-readable memory or medium produce an article of manufacture including instruction means which implement the analysis. The computer program instructions may also be loaded onto a computer or other programmable apparatus to cause a series of operational steps to be performed on the computer or other programmable apparatus to produce a computer implemented process such that the instructions which execute on the computer or other programmable apparatus provide steps for implementing the functions or steps described above.

Furthermore, computer program product/module **550** may be implemented in any suitable language and/or browsers. For example, it may be implemented with Python, Java, Scala, C language and preferably using object-oriented high-level programming languages such as Visual Basic, SmallTalk, C++, and the like. The application can be written to suit environments such as the Microsoft Windows™ environment including Windows™ 98, Windows™ 2000, Windows™ NT, and the like. In addition, the application can also be written for the Macintosh™, SUN™, UNIX or LINUX environment. In addition, the functional steps can also be implemented using a universal or platform-independent programming language. Examples of such multi-platform programming languages include, but are not limited to, hypertext markup language (HTML), JAVA™, JavaScript™, Flash programming language, common gateway interface/structured query language (CGI/SQL), practical extraction report language (PERL), AppleScript™ and other system script languages, programming language/structured query language (PL/SQL), and the like. Java™- or JavaScript™-enabled browsers such as HotJava™, Microsoft™ Explorer™, or Firefox™ can be used. When active content web pages are used, they may include Java™ applets or ActiveX™ controls or other active content technologies.

The system invokes a number of routines. While some of the routines are described herein, one skilled in the art is capable of identifying other routines the system could perform. Moreover, the routines described herein can be altered in various ways. As examples, the order of illustrated logic may be rearranged, substeps may be performed in parallel, illustrated logic may be omitted, other logic may be included, etc.

FIG. **6** is a flow diagram illustrating routine **600** for providing Duplex Sequencing Data for double-stranded nucleic acid molecules in a sample (e.g., a sample from a biological mixture). The routine **600** can be invoked by a computing device, such as a client computer or a server computer coupled to a computer network. In one embodiment the computing device includes sequence data generator and/or a sequence module. As an example, the computing device may invoke the routine **600** after an operator engages a user interface in communication with the computing device.

The routine **600** begins at block **602** and the sequence module receives raw sequence data from a user computing device (block **604**) and creates a sample-specific data set comprising a plurality of raw sequence reads derived from a plurality of nucleic acid molecules in the sample (block **606**). In some embodiments, the server can store the sample-specific data set in a database for later processing. Next, the DS module receives a request to for generating Duplex Consensus Sequencing data from the raw sequence data in the sample-specific data set (block **608**). The DS module groups sequence reads from families representing an original double-stranded nucleic acid molecule (e.g., based on SMI sequences) and compares representative sequences from individual strands to each other (block **610**). In one embodiment, the representative sequences can be one or more than one sequence read from each original nucleic acid molecule. In another embodiment, the representative sequences can be single-strand consensus sequences (SSCSs) generated from alignment and error-correction within representative strands. In such embodiments, a SSCS from a first strand can be compared to a SSCS from a second strand.

At block **612,** the DS module identifies nucleotide positions of complementarity between the compared representative strands. For example, the DS module identifies nucleotide positions along the compared (e.g., aligned) sequence reads where the nucleotide base calls are in agreement. Additionally, the DS module identifies positions of non-complementarity between the compared representative strands (block **614**). Accordingly, the DS module can identify nucleotide positions along the compared (e.g., aligned) sequence reads where the nucleotide base calls are in disagreement.

Next, the DS module can provide Duplex Sequencing Data for double-stranded nucleic acid molecules in a sample (block **616**). Such data can be in the form of duplex consensus sequences for each of the processed sequence reads. Duplex consensus sequences can include, in one embodiment, only nucleotide positions where the representative sequences from each strand of an original nucleic acid molecule are in agreement. Accordingly, in one embodiment, positions of disagreement can be eliminated or otherwise discounted such that the duplex consensus sequence is a high accuracy sequence read that has been error-corrected. In another embodiment, Duplex Sequencing Data can include reporting information on nucleotide positions of disagreement in order that such positions can be further analyzed (e.g., in instances where DNA damage can be assessed). The routine **600** may then continue at block **618,** where it ends.

FIG. **7** is a flow diagram illustrating a routine **700** for detecting, identifying and quantifying microhaplotypes present in nucleic acid mixtures to determine known source genotypes a sample. The routine can be invoked by the computing device of FIG. **5.** The routine **700** begins at block **702** and the genotype module analyzes the Duplex Sequencing Data from FIG. **6** (e.g., following block **616**) to identify microhaplotype allele combinations present within individual DNA molecules (block **704**) and sums the total counts of each allele donated from each known source genotype (block **706**). Next, the genotype module calculates the mixing proportion of each genotype present in the mixture using, for example, a regression-based model (block **708**). As such, a microhaplotype analysis can provide information regarding the original biological source and the relative proportion of each source contributing to the nucleic acid mixture.

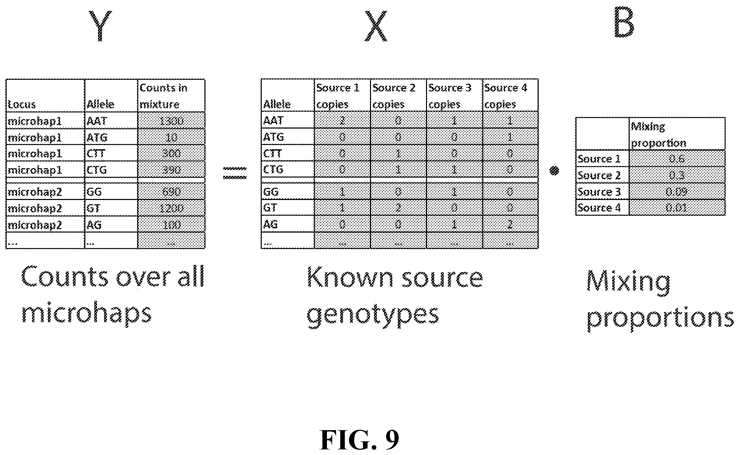

The genotype module can also optionally compare a mixing proportion of each genotype with an original mixing proportion (block **710**) (e.g., in cases of cells from different biological source grown together) to evaluate selective pressures on a biological source mixture (e.g., mixture of cells in culture). Next, the genotype module can provide genotype data (block **712**) that can be stored in the sample-specific data set in the database. The routine **700** may then continue at block **714,** where it ends. FIG. **9** illustrates one example of genotype data (e.g., counts over all microhaplotypes, known source genotypes, mixing proportions) that can be determined using the routine 2100 and which can be stored in the database.

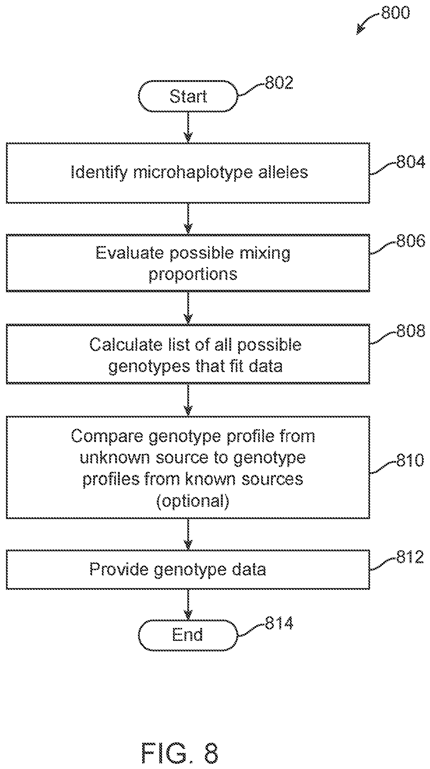

FIG. **8** is a flow diagram illustrating a routine **800** for deconvolving nucleic acid mixtures of unknown genotypes in a sample. The routine can be invoked by the computing device of FIG. **5.** The routine **800** begins at block **802** and the genotype module analyzes the Duplex Sequencing Data from FIG. **6** (e.g., following block **616**) to identify microhaplotype allele combinations present within individual DNA molecules (block **804**). In some embodiments, SNP allele combinations can be identified when, for example, long-read sequencing technologies are utilized. Next, the genotype module evaluates all possible mixing proportions against all possible genotypes present at each genetic locus (block **806**). The genotype module then calculates, for each genetic locus, a list of all possible genotypes that adequately fit the sequence data (e.g., SNP data, microhaplotype data) and all possible mixing proportions evaluated (block **808**). As such, a microhaplotype/SNP analysis can provide genetic information regarding the original biological sources, information regarding the number of original biological sources present, and the relative proportion of each source contributing to the nucleic acid mixture.

The genotype module can also optionally compare genotype profiles from the unknown sources to a database comprising genotype profiles of known sources to identify a specific biological source contributor to the complex nucleic acid mixture (block **810**) (e.g., in forensic cases such as for identifying perpetrators, victims or missing persons). In some embodiments, the genotype module can also optionally determine the presence of genotypes from a database within a mixed sample by evaluating whether the mixture data can be adequately fit if one or more known genotypes are included in the mixture.

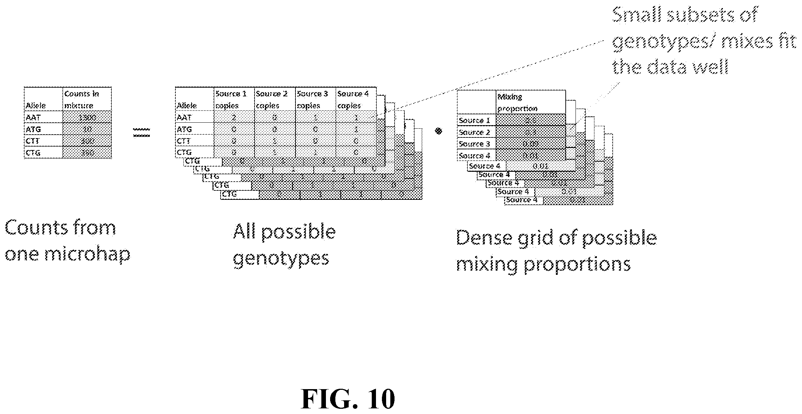

Next, the genotype module can provide genotype data (block **812**) that can be stored in the sample-specific data set in the database. The routine **800** may then continue at block **814,** where it ends. FIG. **10** illustrates one example of genotype data (e.g., counts from microhaplotypes, possible genotype profiles, grids of possible mixing proportions) that can be determined using the routine 2100 and which can be stored in the database.

V. Experimental Examples

The following section provides some illustrative examples of methods for resolving nucleic acid mixtures using Duplex Sequencing and associated reagents.

*Example 1*

Cord blood expansion: expanding CD34+ cord blood stem cells for use in allogeneic transplants. Cord blood samples from multiple donors (e.g., 8 donors) were pooled and notch ligand-expanded together in culture. In this example, Duplex Sequencing was used to assess whether the relative proportions of inputted CD34+ stem cells from each donor were maintained during the expansion process.

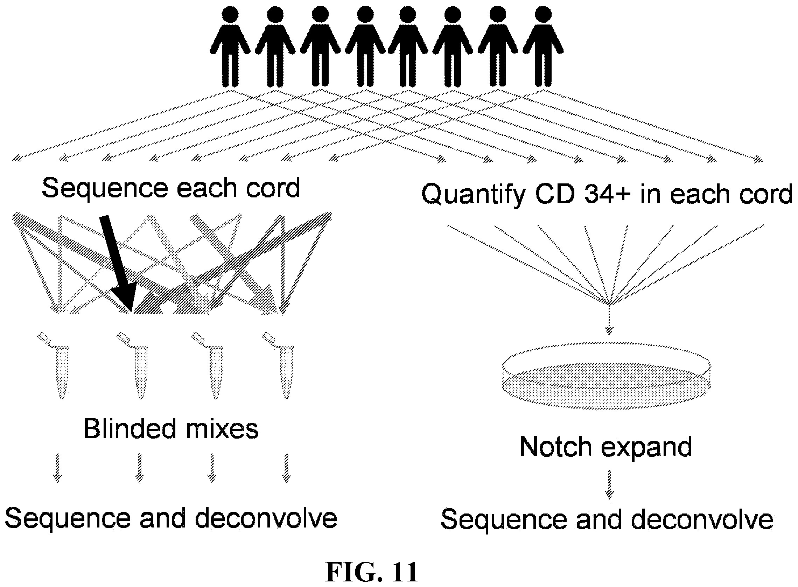

In this example, cord blood from the 8 individual donors was CD34+ enriched and flow-quantified. As illustrated in FIG. **11,** an aliquot of each cord blood sample was DNA-extracted and individually sequenced. Four blinded test mixtures were prepared (left-hand side of FIG. **11**), sequenced using Duplex Sequencing protocols as previously described herein and in U.S. Patent No. 9,752,188. Following sequencing, the mixtures were analyzed and deconvolved.

In a second aspect of this example shown in the right-hand side of FIG. **11,** viable cells from the original cord blood samples were pooled (variable CD34+ count each) and notch ligand expanded. Following expansion, DNA was extracted from the pooled cells and sequenced.

Sequencing results were subsequently analyzed to determine if the sequenced mixtures recapitulate the expected mixes of the four blinded test mixtures (left-hand side of FIG. **11**), and to determine if the relative percentage (based on representative DNA quantity of each unique genotype) of each cord sample after expansion mirrors the original input CD34+ counts.

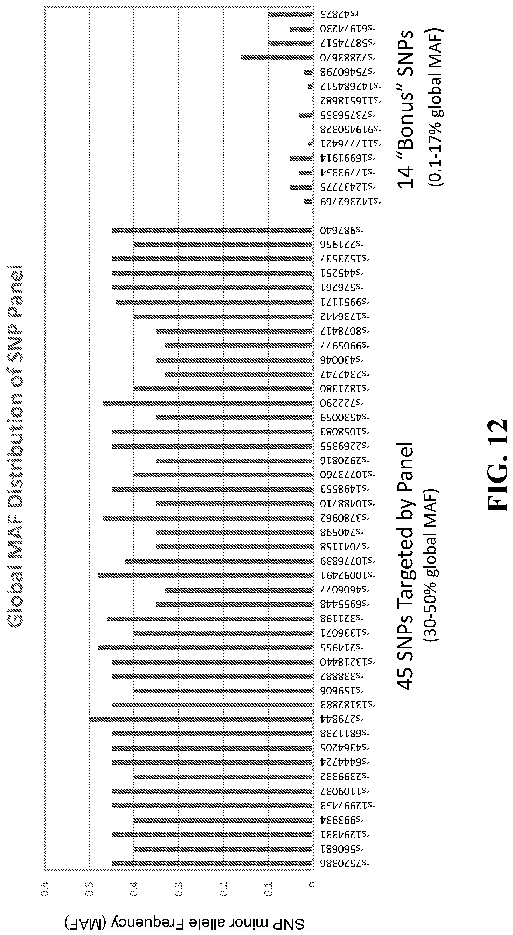

Duplex Sequencing was performed using a panel of probes to genotype 45 high MAF SNP sites and 16 low MAF SNP sites scattered across the entire human genome. As illustrated in FIG. **12,** illustrates the global distribution of the SNP panel used in this example.

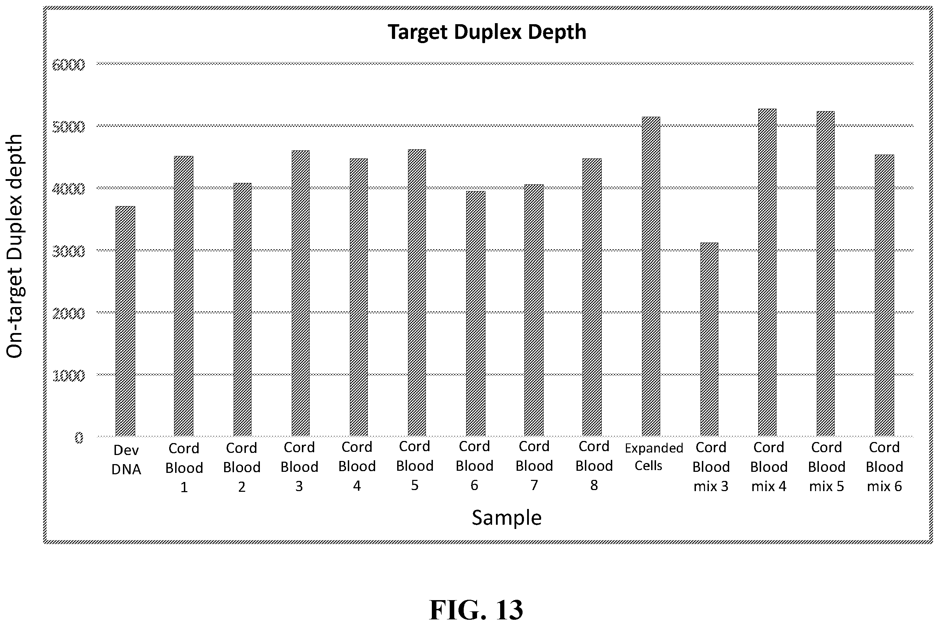

Within the mixtures that were generated in this example, it was known that the lowest variant allele frequency (VAF) SNP was 0.6%. As such, sequencing was performed to an approximate 3,000x depth such that at 0.6% VAF, there was a likelihood of determining approximately 18 SNP events from a homozygous individual or approximately 9 SNP events from a heterozygous individual. Sequencing was carried out on 250ng of DNA from each of 4 cord blood mixtures, the cord blood expansion mixture and cord blood samples from each of the 8 individual donors, to the approximate 3000x unique molecular depth using Duplex Sequencing methods. All sequencing and genotype determinations were performed blinded. FIG. **13** is a bar graph showing the on-target Duplex Sequencing depth for each sample.

Analysis included examination of 59 SNP sites for indication of cross-contamination in a DNA standard that was prepared concurrently to experimental samples. No contamination molecules were found (among 222,025 polymorphic site base pairs sequenced). The 59 SNP sites were examined in the 8 cord blood samples and no evidence of human DNA cross-contamination in the cord blood samples was found.

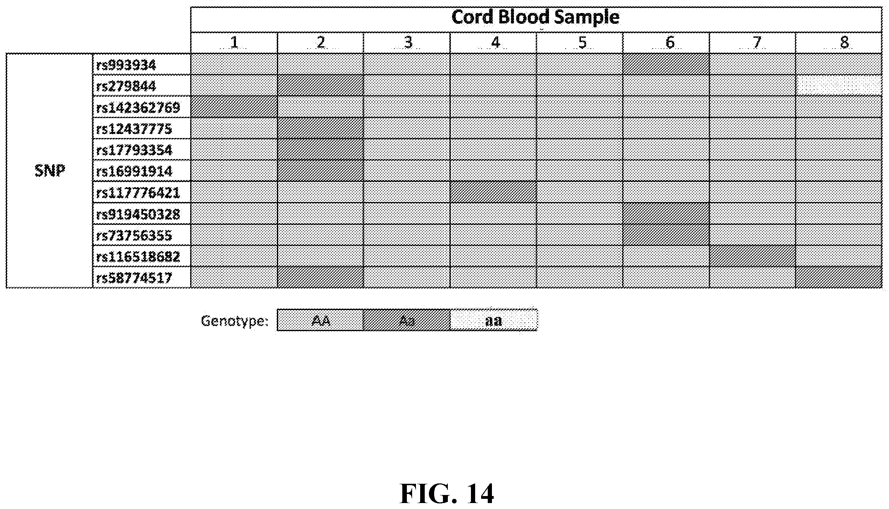

FIG. **14** shows a panel identifying 11 specific SNP alleles used to differentiate the donor genotypes. Ten of these SNPs were within the low-MAF subset shown in FIG. **12.** Analysis of the individual donor samples, shown in FIG. **14,** shows that 9 SNP variants were unique to an individual sample, and two additional SNP sites were present in only two of the donor samples. Five of the original cord blood samples from the donors could be uniquely identified by one or more specific alleles, and the 3 remaining cord blood samples could be identified by inference of shared higher frequency SNPs.

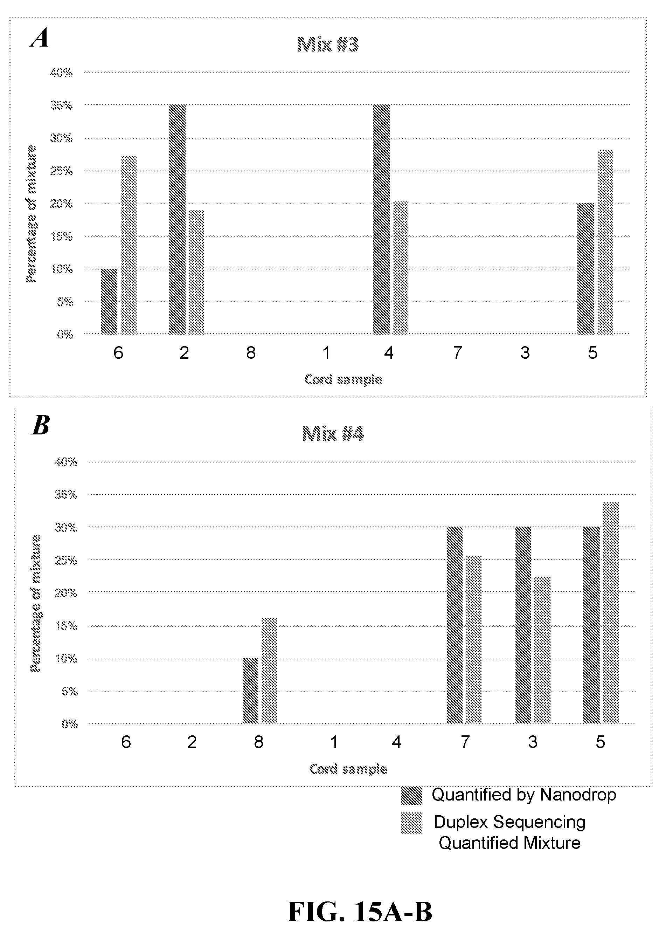

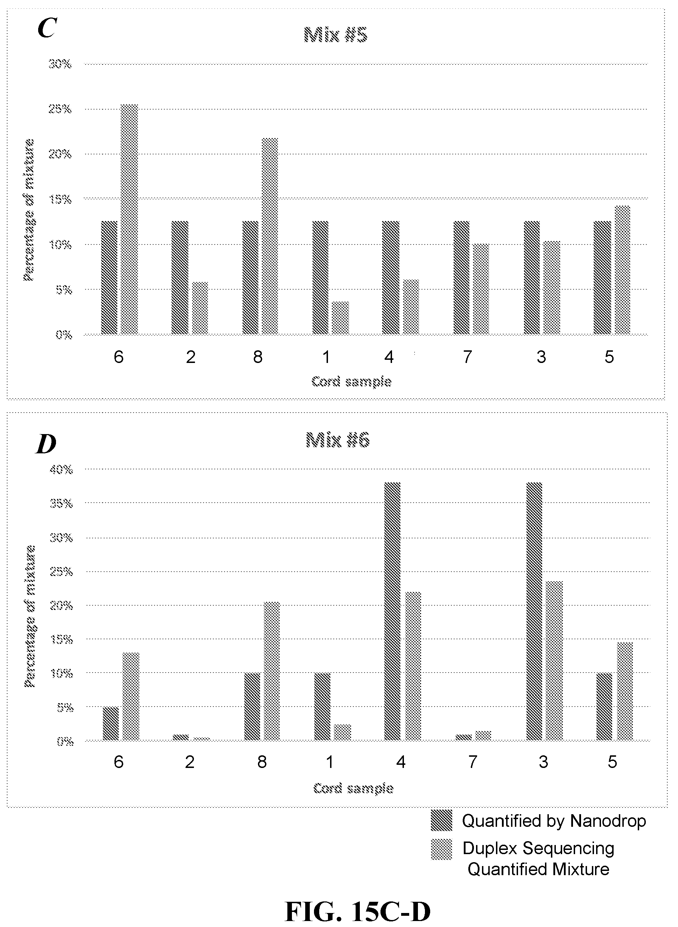

Referring to FIGS. **15A-15D,** Duplex Sequencing methods yielded complete sensitivity and specificity for detecting each individual cord blood source in each of the mixtures. For example, in reference to FIG. **15D,** one of the cord blood mixtures (i.e., Cord Blood Mixture #6) contained two individual cord blood samples (#2, #7) representing 1% each of the total mixture. These cord blood samples were detected and accurately quantified at the 1% of total representation using Duplex Sequencing methodology.

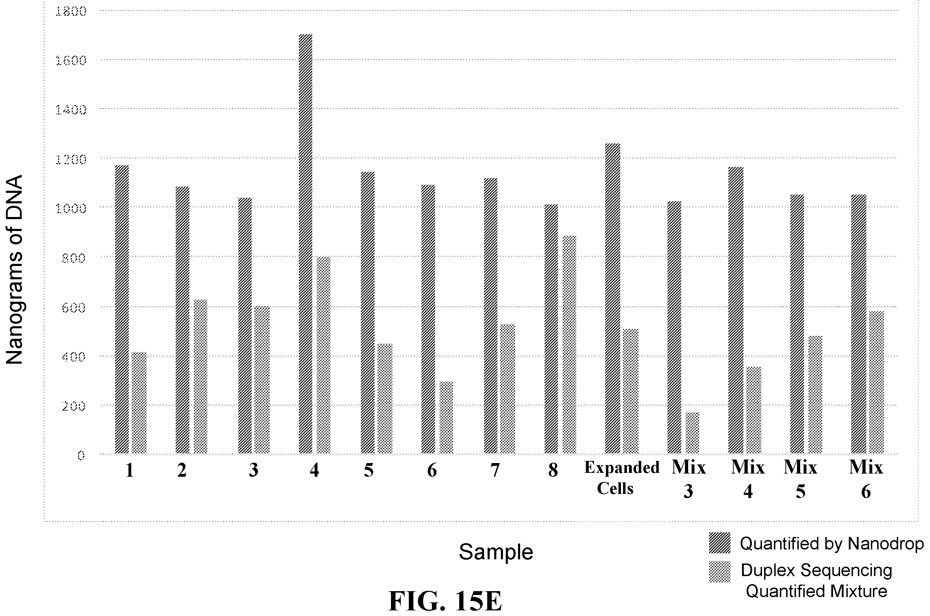

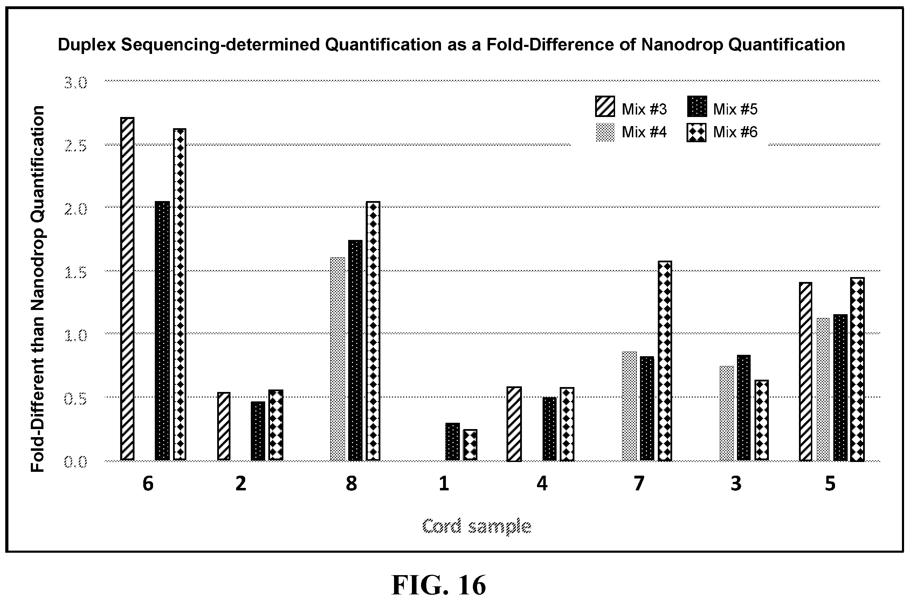

Referring to FIGS. **15A-15D,** it was noted that there was a significant amount of variation from the expected percentages (e.g., Nanodrop spectrophotometer measured quantifications of the amount of each sample that was used to produce the mixtures). Without being bound by theory, it is believed that the shown discrepancies in DNA quantification shown in FIGS. **15A-15B** between the Duplex Sequencing approach and the Nanodrop quantification approach are due to confounding factors attributable to the Nanodrop approach (e.g., the presence of non-destroyed RNA, etc.). This was substantiated when looking at quantification of all samples (e.g., individual cord blood samples, DNA extracted from expanded cells, and from test mixtures) with both Nanodrop and by Qubit fluorometer measurement (see FIG. **15E**). As shown, Nanodrop measurements (dark grey bar) appeared to overrepresent the sample quantification measurement as compared to Qubit (light grey bar). As further evidence of quantification measurement discrepancy between Duplex Sequencing quantification approach and the Nanodrop quantification approach, FIG. **16** illustrates the fold-difference in quantification for each individual cord blood sample within each mixture. As shown, the fold difference for each of these samples were similar, further suggesting that quantification errors prior to generating the mixtures explains the discrepant results in FIGS. **15A-15D.** Regardless, the individual sources of the cord blood samples used to generate the mixtures were accurately identified as well as their representative contributions to the mixture (FIGS. **15A-15D**).

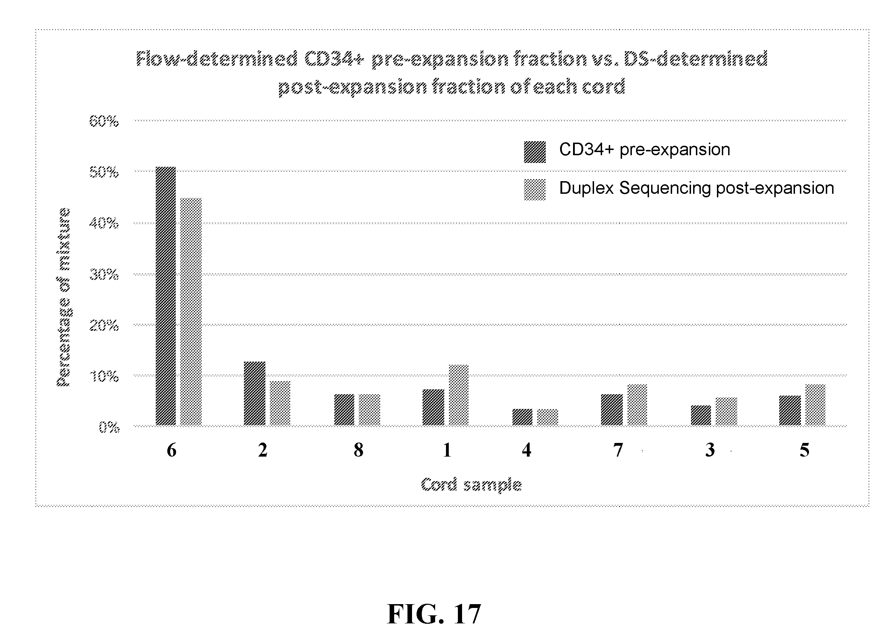

FIG. **17** is a bar graph depicting the flow-cytometry determined CD34+ fraction of cells prior to expansion and the CD34$^+$ fraction of cells (as determined by Duplex Sequencing) following expansion for each individual blood cord sample. As shown, there is a strong correlation between the pre-expansion CD34+ cell count and the post-expansion cell count. These results suggest that the cells from each cord blood sample present in this expansion proliferated proportionally. Further, these data show that the cells proliferating are CD34+ cells and are not other differentiated cells that also undergo Notch expansion.

In this example, it has been demonstrated that Duplex Sequencing methodology can be used to deconvolve a biological mixture (e.g., a mixture of cord blood samples from 8 individuals) using polymorphic markers. Using fairly moderate sequencing depth (e.g., 3000x), Duplex Sequencing methodology was able to confidently detect each cord ample with total sensitivity and specificity in each synthetic mixture tested. Without being bound by theory, it is believed that discrepancies in the relative abundance of each cord sample in the synthetic mixtures tested are the result of errors in DNA quantification when making the mixtures and not a deficiency of the Duplex Sequencing process.

It was further demonstrated in this example, that Duplex Sequencing methodology used to analyze the post-expansion cord-blood mixture yielded very similar results to the relative percentages of CD34+ cells from each cord blood sample prior to Notch-expansion, suggesting that, at least in the present experiment, CD34+ cells from each cord blood sample proliferated relatively proportionately to each other and relative fractions of each are represented at the same fraction of total as the original mixture.

In this example, Duplex Sequencing demonstrates to be a successful method for deconvolving mixtures of nucleic acid material and identify presence as well as abundance of the original DNA sources. Accordingly, Duplex Sequencing methodology provides a rapid, quantitative, and automatable way of cost-effectively assessing the abundance of multi-individual cultures of therapeutic human cells.

Based on the patterns of SNP genotypes determined for each cord this approach is able to narrow in on a range of expected HLA haplotypes from the SNP haplotypes. For example, prediction of ancestry probability (e.g., Maori vs Inuit vs. Northern European ancestry) can be assessed.

In general, this example demonstrates resolution of 8 sources however, a panel can be designed to allow whatever specification is needed. It is expected that Duplex Sequencing can get to multiple dozen constituents with a cost-effective panel if there are known genotypes ahead of time. In other embodiments, deconvolution of multiple sources without *a priori* knowledge is also possible with Duplex Sequencing approach as described herein.

The targeted depth of 3000x unique molecular depth, in this example was chosen as there were no particularly rare events. In examples where rare events are known or suspected, sequencing depth can increase (e.g. about 10,000x,15,000x, 20,000x, 25,000x, 30,000x, 35,000x, 40,000x, 45,000x, 50,000x, 75,000x, 100,000x, 200,000x, 500,000x, 1,000,000x or more). Variants can be detected as a ratio (variant SNP per total SNP BP sequenced); with depth sufficient as long as achieve more than minimum targeted to achieve particular statistical confidences.

The ability to sort into different cell compartments and quantify chimerisms in cord blood mixtures may provide information on whether one cord vs another differentiates differently into one lineage vs another. The ability to assess relative chimerisms cell-free DNA in plasma allows Duplex Sequencing method to track the relative die-off of one cord over another on short time scale (half-life of cfDNA is ~2.5 hours in plasma). Similarly, such methods applied to cell free-DNA could assess the relative proportion of DNA from a transplanted genome, such as with as solid organ transplant such as kidney, heart or lung, as may occur with transplant rejection.

*Example 2*

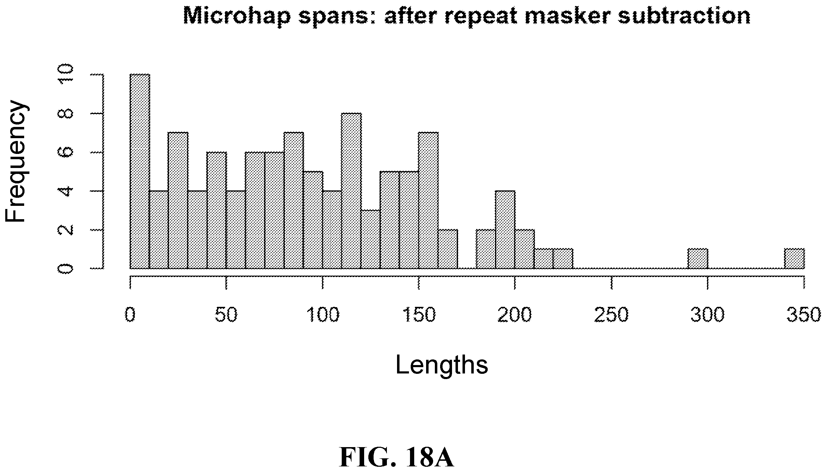

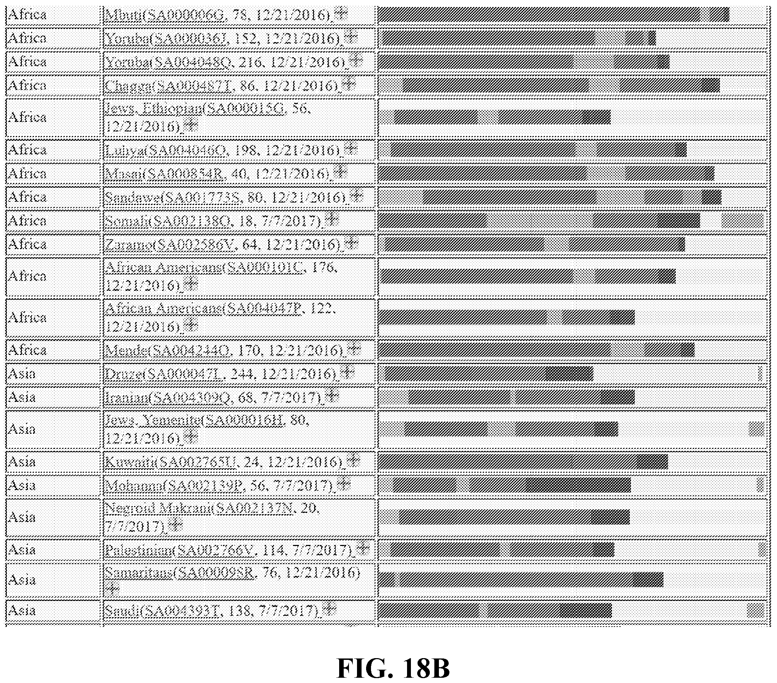

Duplex Sequencing of microhaplotype genomic sites for mixture deconvolution. Microhaplotypes are loci of two or more genomic DNA SNPs within a relatively short distance from each other (e.g., <200 nucleotides, <250 nucleotides, < 300 nucleotides, < 350 nucleotides or longer) with three or more allelic combinations (e.g., about 3, 4, 5, 6, 7, 8, 9, 10, 11, 12, 13, 14 or more distinct alleles). A given microhaplotype may have as few as zero "informative" individual polymorphisms for a given mixture (i.e. there are no differences between the microhaplotypes between the DNA molecules in the mixture from different individuals), or several individual polymorphisms (e.g., at least about 2, 3, 4, 5, 6, 7, 8, 9, 10, 11. 12, 15, 20, 24, or more). As shown in FIG. **18A,** the majority of microhaplotypes in a particular panel of identified useful microhaplotypes are under 200 nucleotides in length. FIG. **18B** is an example of allele frequency for one microhaplotype in varied populations around the world.

In this example, DNA mixtures were probed and sequenced over microhaplotype regions to deconvolve the mixtures into the one or more original DNA sources for purposes of, for example, identification and to determine abundance of each source. By using a probe panel for assessing microhaplotype markers, more alleles per probe/sequencing read can be assessed which yields more power in the sequencing data for source identification. In a particular example, a probe panel looking at ~100 genomic regions containing microhaplotypes can utilize unique combination of ~ 300 alleles to identify source(s). A probe panel can be used to perform paired end sequencing or single end sequencing using Duplex Sequencing.

In an example of where donor genotypes are known, private alleles can be used to identify a particular donor. For example, if an allele is unique to a particular donor, the donor's proportion is equal to the proportion of sequencing reads at that locus containing the minor allele. This methodology can be used for assessing low abundance samples because only one read of the private allele would be necessary to identify the donor.

In another example, Duplex Sequencing and subsequent deconvolution of the sequencing data can be used to identify genotype (e.g., source) donors. For example, given enough alleles probed, a combination of the alleles can be used to generate a unique genotype fingerprint (e.g., genetic signature) to identify and quantify the donor(s).

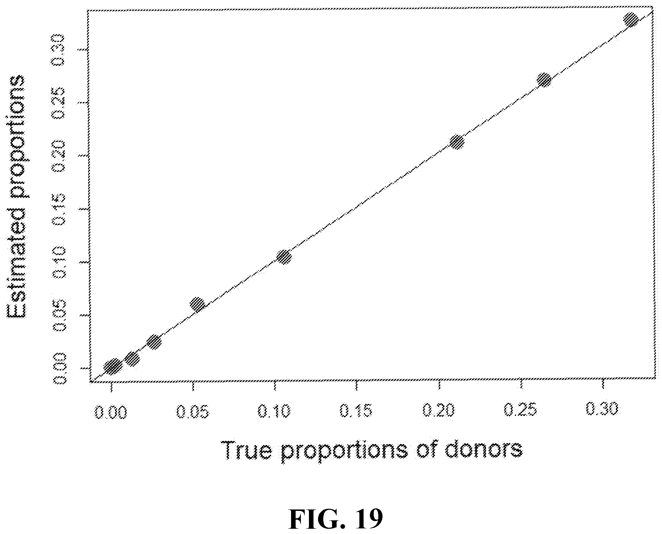

In a simulated example, 9 genotypes are in a mixture with some rare genotypes mixed with some abundant genotypes. A probe panel for sequencing 100 microhaplotypes representing 266 distinct alleles will be used to sequence the DNA mixture at a depth of approximately 3000-fold. In this hypothetical example, using mixture estimation, private alleles arising from simulated microhaplotypes detected each donor. In the simulation, after removing the use of private alleles, the data could still be used to conclusively detect all but the 3 lowest-abundance donors (0.03%, 0.3%, and 1.3% of simulated mixture). Results of the simulated deconvolution of Duplex Sequencing data is shown in FIG. **19.**

*Example 3*

This example describes an embodiment of Duplex Sequencing of microhaplotype genomic sites for mixture deconvolution. In this example, a patient sample with minor alleles at 4 loci observed at 5%, 10%, 20%, and 40%. In this example, these allele frequencies can't be explained by just the patient's DNA. At least 3 genomes are present. A good fit for this scenario would be donor 1 at 10%, donor 2 at 40% and patient 3 at 50%. In deconvolution, this strategy seeks to find mixing proportions that best explain the observed allele frequencies, given whatever donor genotypes lead to the best fit. In some examples, machine learning methods can facilitate this analysis.

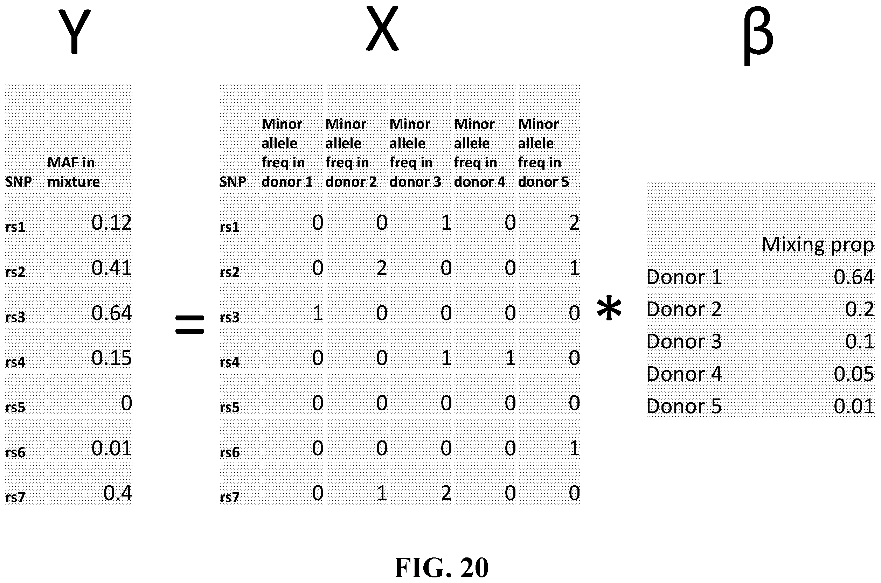

One example of a deconvolution model for resolving Duplex Sequencing data can use linear regression, generalized linear models, or extension thereof. FIG. **20** illustrates one example of a linear regression model for determining donor sources within a mixture using 7 SNPs each with a known genotype in the donors. The mixing proportion for each donor ($\beta$) is determined.

*Example 4*

This example describes another embodiment of Duplex Sequencing of microhaplotype genomic sites for mixture deconvolution. In this example, Duplex Sequencing Data derived from a sample with a mixture of nucleic acid molecules from known biological sources is used to estimate a mixing proportion of the mixed sources. Aspects of the analysis can be performed using various embodiments of the computing system as described herein (e.g., with reference to FIG. **5**).

In a first step, a routine can be invoked by a computing device, such a computing device that has a genotype module, and such routine can call the vector of counts of each allele of each assayed locus (Y). An example of the vector Y is shown in Table 1. In this particular example, each locus has two alleles and a no-call, or "N" value, however, this routine can accommodate any number of alleles at any given locus. Accordingly, in this example, when microhaplotype data is used, each locus will have at least 3 alleles, plus various no-call alleles where one or more SNPs in the microhaplotype are not given a call by the genotype module (e.g., the routine).

**Table 1**

| Locus | Allele | Locus-Allele | Allele counts ("Y") | Total depth at locus |
|---|---|---|---|---|
| 1 | a | 1a | 1500 | 1850 |
| 1 | b | 1b | 200 | 1850 |
| 1 | N | 1N | 150 | 1850 |
| 2 | a | 2a | 200 | 1300 |
| 2 | b | 2b | 1000 | 1300 |
| 2 | N | 2N | 100 | 1300 |
| 3 | a | 3a | 1230 | 1420 |
| 3 | b | 3b | 140 | 1420 |
| 3 | N | 3N | 50 | 1420 |
| 4 | a | 4a | 800 | 1800 |
| 4 | b | 4b | 850 | 1800 |
| 4 | N | 4N | 150 | 1800 |

Example allele count data. The vector Y is highlighted (grey column); accompanying data and IDs are in other columns.

In a second step, the routine defines the diagonal matrix $\lambda$ with rows and columns corresponding to the elements of Y. The diagonal elements of $\lambda$ equal the total counts of all alleles at the locus they correspond to. For example, the value of $\lambda$ at element [1,1] should equal the total counts at the locus in the first position of Y. An example of the matrix $\lambda$ is shown in Table 2.

**Table 2**

| | 1a | 1b | 1N | 2a | 2b | 2N | 3a | 3b | 3N | 4a | 4b | 4N |
|---|---|---|---|---|---|---|---|---|---|---|---|---|
| 1a | 1850 | 0 | 0 | 0 | 0 | 0 | 0 | 0 | 0 | 0 | 0 | 0 |
| 1b | 0 | 1850 | 0 | 0 | 0 | 0 | 0 | 0 | 0 | 0 | 0 | 0 |
| 1N | 0 | 0 | 1850 | 0 | 0 | 0 | 0 | 0 | 0 | 0 | 0 | 0 |
| 2a | 0 | 0 | 0 | 1300 | 0 | 0 | 0 | 0 | 0 | 0 | 0 | 0 |
| 2b | 0 | 0 | 0 | 0 | 1300 | 0 | 0 | 0 | 0 | 0 | 0 | 0 |
| 2N | 0 | 0 | 0 | 0 | 0 | 1300 | 0 | 0 | 0 | 0 | 0 | 0 |
| 3a | 0 | 0 | 0 | 0 | 0 | 0 | 1420 | 0 | 0 | 0 | 0 | 0 |
| 3b | 0 | 0 | 0 | 0 | 0 | 0 | 0 | 1420 | 0 | 0 | 0 | 0 |
| 3N | 0 | 0 | 0 | 0 | 0 | 0 | 0 | 0 | 1420 | 0 | 0 | 0 |
| 4a | 0 | 0 | 0 | 0 | 0 | 0 | 0 | 0 | 0 | 1800 | 0 | 0 |
| 4b | 0 | 0 | 0 | 0 | 0 | 0 | 0 | 0 | 0 | 0 | 1800 | 0 |
| 4N | 0 | 0 | 0 | 0 | 0 | 0 | 0 | 0 | 0 | 0 | 0 | 1800 |

Example of the matrix $\lambda$. The matrix is highlighted in grey; row and column names are shown for alignment with other data vectors and matrices.

In a third step, the routine writes the source genotypes as a matrix G0 with rows aligning to the elements of Y and columns corresponding to individual sources. The routine defines G0 such that the [i,j] element of G0 will correspond to the genotype of the $i^{th}$ allele in Y in the $j^{th}$ source, populating it with 0, 0.5 or 1 depending on whether source j has 0, 1 or 2 copies of the allele. An example of the matrix G0 is shown in Table 3.

**Table 3**

| Locus | Allele | Locus+Allele | Source 1 genotype frequency | Source 2 genotype frequency | Source 3 genotype frequency | Source 4 genotype frequency |
|---|---|---|---|---|---|---|
| 1 | a | 1a | 1 | 0.5 | 1 | 0 |
| 1 | b | 1b | 0 | 0.5 | 0 | 1 |
| 2 | a | 2a | 0.5 | 1 | 1 | 1 |
| 2 | b | 2b | 0.5 | 0 | 0 | 0 |
| 3 | a | 3a | 1 | 1 | 0.5 | 1 |
| 3 | b | 3b | 0 | 0 | 0.5 | 0 |
| 4 | a | 4a | 1 | 1 | 0 | 1 |
| 4 | b | 4b | 0 | 0 | 1 | 0 |

Example of G0, the matrix of source genotypes. G0 is highlighted in grey; other columns show IDs for alignment with other data vectors and matrices.

In a fourth step, the routine accounts for no-call, or "N" reads in the data. "N" reads at a locus occur from various causes, and the probability of an N can vary both with locus and allele. Calibration experiments on samples with known genotypes can estimate the locus-and-allele-specific probabilities of an N being recorded.

In effect, the no-call phenomenon leads to a distortion of the patient genotype data. If a patient is homozygous for allele A at a locus, that patient is not truly expected to contribute 100% counts of allele A: he will also contribute some counts with value "N".

To account for no-call or "N" reads, the routine creates a modified genotype matrix that accounts for the allele-specific probabilities of alleles being read as no-call, or "N", by the sequencer and/or the genotype module (e.g., the routine). Table 4 shows how this G matrix can be calculated from G0 and from the locus-and-allele-specific rates of no-call/N reads.

**Table 4**

| Locus | Allele | Locus-Allele | Rate of change to Ns | Source 1 genotype frequency | Source 2 genotype frequency | Source 3 genotype frequency | Source 4 genotype frequency |
|---|---|---|---|---|---|---|---|
| 1 | a | 1a | 0.2 | 0.8 | 0.4 | 0.8 | 0 |
| 1 | b | 1b | 0.1 | 0 | 0.45 | 0 | 0.9 |
| 1 | N | 1N | | 0.2 | 0.15 | 0.2 | 0.1 |
| 2 | a | 2a | 0.1 | 0.45 | 0.9 | 0.9 | 0.9 |
| 2 | b | 2b | 0.1 | 0.45 | 0 | 0 | 0 |
| 2 | N | 2N | | 0.1 | 0.1 | 0.1 | 0.1 |
| 3 | a | 3a | 0.15 | 0.85 | 0.85 | 0.425 | 0.85 |
| 3 | b | 3b | 0.05 | 0 | 0 | 0.475 | 0 |
| 3 | N | 3N | | 0.15 | 0.15 | 0.1 | 0.15 |
| 4 | a | 4a | 0.05 | 0.95 | 0.95 | 0 | 0.95 |
| 4 | b | 4b | 0.12 | 0 | 0 | 0.88 | 0 |
| 4 | N | 4N | | 0.05 | 0.05 | 0.12 | 0.05 |

Example of G, the matrix of source genotypes after accounting for no-call, or "N" reads. G is highlighted in grey; other columns show IDs for alignment with other data vectors and matrices. G can be calculated from G0 and from the column "Rate of change to Ns".

In another embodiment, the routine can be configured to discard all uncalled, or "N", alleles from the data vector Y, use this partial Y for calculation of $\lambda$, and use G0 in place of G in the following steps.

Once the data matrices are determined, the routine can estimate the source mixing proportions.

In this example, the routine calls β the vector of mixing proportions of the sources in the mixed sample. An example of β is shown in Table 5. The routine may first estimate the elements of β and secondly place confidence intervals around these estimates.

**Table 5**

|  | Mixing proportion |
| --- | --- |
| Source 1 | β1 |
| Source 2 | β2 |
| Source 3 | β3 |
| Source 4 | β4 |

Example of β, the vector of unknown mixing proportions. β itself is highlighted in grey; other values show IDs for alignment to other vectors and matrices.

Next, the routine can model the data as follows:

$$E(Y) = \lambda G\beta,$$

Where Y, λ, G and β are as defined above, and juxtaposition of two vectors or matrices denotes the dot product operator as is standard in linear algebra notation. This formula has the simple interpretation for a given allele of a given locus, the expected number of reads is equal to the sequencing depth at that locus times the mixing proportion of that allele in the sample. (The mixing proportion of an allele in the sample, or more accurately, the allele's expected proportion of reads at that locus, is equal to its element in the vector Gβ.)

This model comprises a generalized linear regression (GLM) approach for estimating β and for calculating confidence intervals for its elements. Define:

$$X = \lambda G\beta,$$

and a GLM setup arises naturally. The GLM's mean model is given by E(Y) = Xβ (i.e. the GLM has an identity link). The GLM's family can be one of several choices.

1. A Poisson family (i.e. the use of Poisson regression of Y on X).

2. A negative binomial family (i.e. the use of Negative Binomial regression of Y on X) can, in some embodiments, be more flexible to noisy data than a Poisson family.

3. A gaussian family (i.e. the use of ordinary linear regression of Y on X) may also be used in some applications.

In all the above setups, the GLM approach returns both estimates and confidence intervals for the elements of β.

Example 4 as described herein comprise one approach to resolving nucleic acid mixtures of known genotypes. One or ordinary skill in the art will recognize that other approaches may also be used. Non-limiting examples of other approaches can include multinomial or binomial regression. In another embodiment, a regression of GLM could be used to predict the frequency of each allele (calculated by the counts of the allele divided by the total counts at the locus) from the genotypes matrix. In yet another embodiment, constraints could be placed on the elements of beta (β). For example, any combination of the following constraints could be applied alongside any embodiment of the regression or GLM approach.

1. Beta (β) could be constrained to be non-negative

2. Beta (β) could be constrained so its elements are all less than or equal to 1.

3. Beta (β) could be constrained so its elements sum to exactly 1.

Because regression methods like Poisson, binomial and multinomial regression all derive approximate standard errors based on asymptotic theory (e.g. using wald, score or likelihood-ratio-based standard errors), these methods can occasionally fail to declare greater-than-zero abundance with statistical significance for very low-abundance sources in a mixture. To correct this error, when a source has a "private" allele not shared by any other sources, and that private allele is observed in the mixed sample, then the source can be declared present at non-zero abundance with high statistical confidence. A confidence interval for the source's abundance can be calculated using any of the diverse methods for calculating confidence intervals on binomial proportions. For example, Wilson-method confidence intervals can be used.

*Example 5*

This example describes another embodiment of Duplex Sequencing of microhaplotype genomic sites for mixture deconvolution. In this example, Duplex Sequencing Data derived from a sample with a mixture of nucleic acid molecules from unknown biological sources is used to identify source genotypes and estimate a mixing proportion of the mixed sources. Aspects of the analysis can be performed using various embodiments of the computing system as described herein (e.g., with reference to FIG. 5).

The data for this example can be formatted as in the above Example 4, with Y, $\lambda$, G0, G and $\beta$ defined as above and as show in Table 1 - Table 5. In the present example, however, the only data we observe is Y and $\lambda$, which is calculated directly from Y. Thus, the unknown genotypes problem differs from the known genotypes problem in that we must estimate G0 alongside $\beta$ in order to evaluate how well any choice of $\beta$ fits the data.

In the known genotypes problem (Example 4), the routine estimated $\beta$ and placed confidence intervals around that estimate, effectively delineating the subspace of all possible $\beta$ vectors that could adequately fit the data. In the present example addressing the unknown genotypes problem, the routine is configured to describe the characteristics of $\beta$ vectors that could plausibly explain the data. For example, the routine determines whether the observed data could have arisen from a sample with only one non-zero element of $\beta$ (i.e. a single source), or whether only a mixture of sources could result in the observed data. In another embodiment, the routine determines whether the observed data could have arisen from a relatively even mixture of sources or whether the data can only be explained if one source is dominant or one or more sources have very low abundance.

Thus, this method evaluates all $\beta$ vectors under consideration, with a goal of returning a summary of the kinds of $\beta$ vectors that could adequately explain the data.

In this example, an approach is described:

1. Set up the data as follows:

1a. Choose K, the maximum number of sources you will consider to possibly occur in a mixture. K gives the length of $\beta$. $\beta$ vectors corresponding to less than K sources will have 0s in some positions.

1b. Define a grid of $\beta$'s to consider, which takes the form of a list of $\beta$ vectors of length K. In one embodiment, the routine is configured to define a set of $\beta$ element values as {0, 0.001, 0.01, 0.02, 0.05, 0.1, 0.2, 0.3, 0.4, 0.5, 0.6, 0.7, 0.8, 0.9, 0.95, 0.99, 0.999, 1}. The routine can then create a $\beta$ list by taking all possible K-length samples from this list with decreasing values, e.g. if K = 3, the routine takes {1,1,1}, {1,1,.999}, {1,1,.99} ... (because the source genotypes are unknown, two choices of $\beta$ with the same values in a different order explain the data equally well). To achieve a list of legal $\beta$'s (i.e. $\beta$'s that sum to 1), the routine is configured to rescale each $\beta$ in the list to sum to 1. The resulting list achieves a detailed grid over the space of possible $\beta$'s.

1c. For each locus, define a list of possible genotypes, formatted as in the G0 matrix described in Example 4. Possible genotypes are all matrices that meet the following criteria:

i. K columns and J rows, where J is the number of unique alleles observed for the locus.

ii. Each element is either 0, 0.5, or 1.

iii. Each column of the matrix sums to 1 (which implies each source is diploid at the locus).

iv. If desired, the routine is configured to modify each G0 matrix in the list in order to account for no-call/ "N" values, using the same approach described in Example 4. Henceforth the matrices in this list of matrices is referred to as "G" matrices, consistent with earlier notation.

2. Evaluate the $\beta$'s fit to the data Y. The routine is configured to associate each $\beta$ in the list with a log-likelihood using the following algorithm.

2a. For each $\beta$ and for each locus, search the list of genotype matrices for the matrix G that leads to the largest log-likelihood for the values of Y from that locus. Log-likelihood can be calculated using any of the following models: 1. with Y being a Poisson random vector with mean vector $\lambda G\beta$, 2. with Y being a Negative Binomial random vector with mean vector $\lambda G\beta$ and size parameter equal to a pre-specified value of a value fit to the data, or 3. with Y being a Log-normal random vector with mean vector log $(\lambda G\beta)$ and variance parameter equal to a pre-specified value of a value fit to the data. In other embodiments, other parametric distributions may be used.

For each $\beta$ in the list, the output of step 2a is a choice of best-fitting G at each locus and an accompanying set of log-likelihoods achieved by $\beta$ with these best-fitting G's.

2b. Calculate the log-likelihood of each $\beta$ as the sum of its log-likelihoods across the loci.

2c. Choose a threshold for acceptably high log-likelihoods. In one embodiment, the choice of threshold is a judgment call depending on the user's discretion. The following approaches all offer reasonable ways to score $\beta$'s on a scale where thresholds have natural interpretations; all of them are calculated from the log-likelihood: 1. The Bayesian Information Criterion (BIC) can be used. 2. The Akaike Information Criterion (AIC) can be used. 3. The chi-squared statistic equal to -2 times the log likelihood ratio between two competing fits can be used.

3. Report all $\beta$'s from the list with log-likelihoods exceeding the chosen threshold.

Recovering source genotypes from mixtures of unknown genotypes.

In addition to estimating the mixing proportions in a sample of unknown genotypes, to the routine can be configured to recover the genotypes of the sources in the mixture. The following algorithm (e.g., that can be invoked by one or more computing systems described herein) describes one method for doing so:

1. Derive a set of valid $\beta$'s using the above algorithm/routine.
2. For each locus:

2a. Calculate all possible genotype matrices as in step 1c of the above algorithm/routine.

2b. Evaluate each possible genotype matrix against all valid $\beta$'s, recording the genotype's highest log-likelihood achieved over all valid $\beta$'s.

2c. Choose a log-likelihood threshold, and report all genotype matrices that exceed that threshold when evaluated along with at least one valid $\beta$.

2d. In some cases, the set of possible genotype matrices is in near-unanimous agreement about a particular source's allele(s) (e.g. all possible genotype matrices include a homozygous allele A for the most-abundant source). In this event, the source's genotype can be called at that allele.

In other cases, the possible genotype matrices may be definite about one half of a source's genotype at a locus. For example, all possible genotype matrices show source 1 as having a copy of allele A, but the matrices disagree on the other half of the source diploid genotype at that locus. In this case, the routine can be configured to call the source as having at least one copy of allele A.

In one embodiment, and to improve computational efficiency, $\beta$'s with very poor log-likelihoods at a single locus can be discarded from further consideration, thus omitting the need to evaluate it across further loci.

If one or more sources with known genotypes occur in a mixture of otherwise unknown genotypes, this method can also be adapted as follows: 1. If there are M sources with known genotypes, associate them with the first M elements of each $\beta$ and with the first M columns of each G. 2. Generate the list of candidate $\beta$s in the same way as described above, but only require that the last K-M columns have decreasing values. 3. When finding the best G matrix to maximize a given $\beta$'s log likelihood at a given locus, fill in the first M columns with the M known genotypes. 4. Run the rest of the algorithm unchanged.

In some embodiments, this method can be run on isolated SNP data. In other embodiments, this method can be run on data from microhaplotypes, which are short regions of the genome containing multiple (2 or more) SNPs and thereby presenting 3 or more allelic combinations. In still further embodiments, various routines can be run using data from a long-read sequencing technology that returns reads spanning >1 SNP.

To improve computational efficiency, and in one embodiment, the method can first be run on SNP-level data to quickly eliminate a large subset of candidate $\beta$'s. The algorithm can then be re-run on microhaplotype-level data using only the $\beta$'s passing this first step.

In some cases where there are large number of source genotypes and larger numbers of alleles, the matrix of possible genotypes may be so large as to be computationally intractable, inefficient, or unnecessary for the particular application. In such cases, for each possible mixing proportion vector β under consideration, a best-fitting genotypes matrix G may be sought through optimization algorithms.

*Example 6*

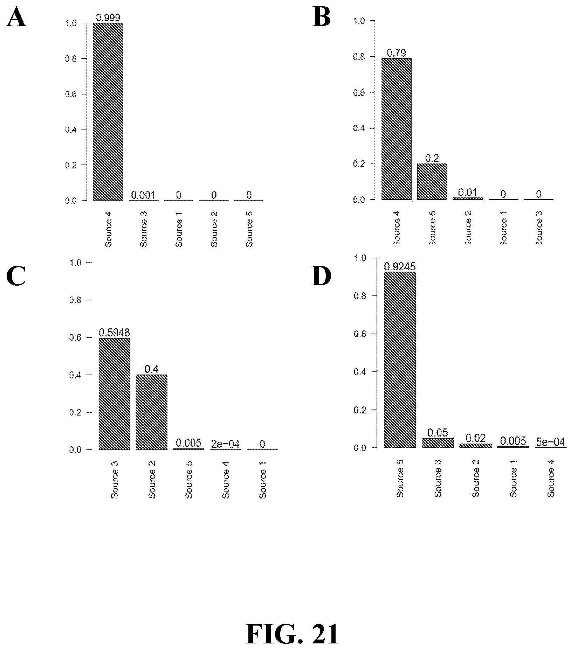

Performance evaluation in synthetic mixes of nucleic acid molecules derived from more than one source: purified DNA from differing biological sources were pre-mixed in specified mixing proportions in four independent samples (e.g., each sample having a different mixing proportion of each source material) and such mixing proportions were then blinded. FIG. **21,** panels A-D are bar graphs depicting the true mixing proportions of sources 1-5.

Sequencing results were subsequently analyzed to determine if the sequenced mixtures recapitulate the expected mixes of the four blinded test mixtures (shown FIG. **21,** panels A-D), and to determine if the sensitively of detection of low frequency/rare alleles present in the mixed samples. Duplex Sequencing was performed using a panel of probes to genotype 17 microhaplotype sites scattered across the entire human genome.

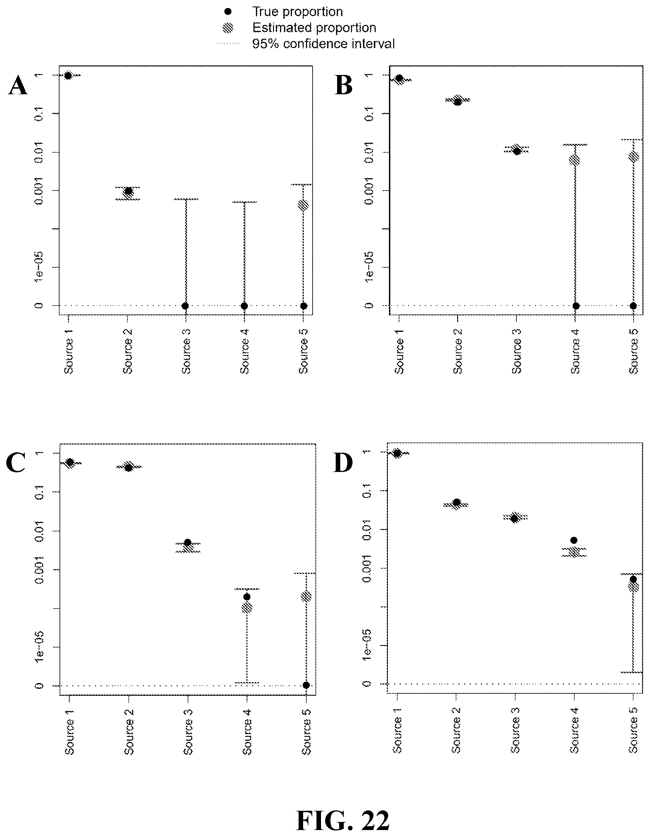

Referring to FIG. **22,** panels A-D (corresponding to FIG. **21,** panels A-D, respectively), Duplex Sequencing methods yielded complete sensitivity and specificity for detecting each individual source in each of the mixtures when the genotypes for each of the five potential sources was known in advance. For example, in reference to FIG. **21,** panel D and FIG. **22,** panel D, one of blinded mixtures (i.e., comprising a mixture of all 5 sources) contained a source representing 0.5% (source 1) and a source representing 0.05% (source 4) of the total mixture. These sources were detected and accurately quantified using Duplex Sequencing methodology (FIG. **22,** panel D).

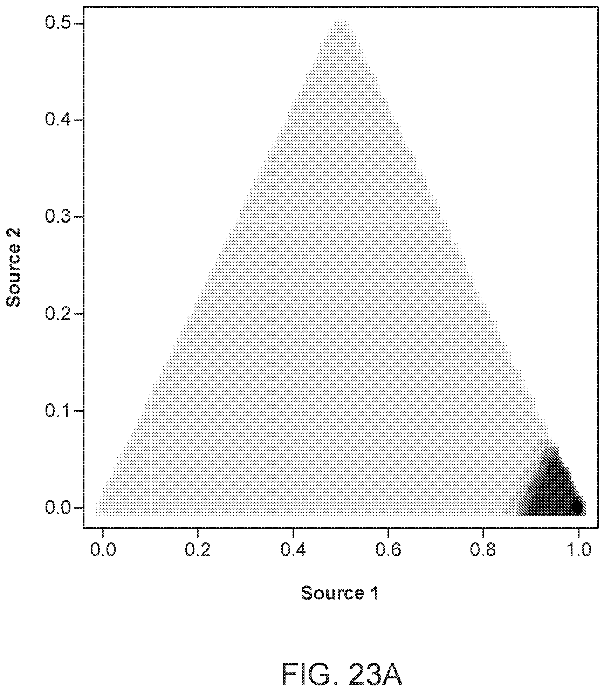

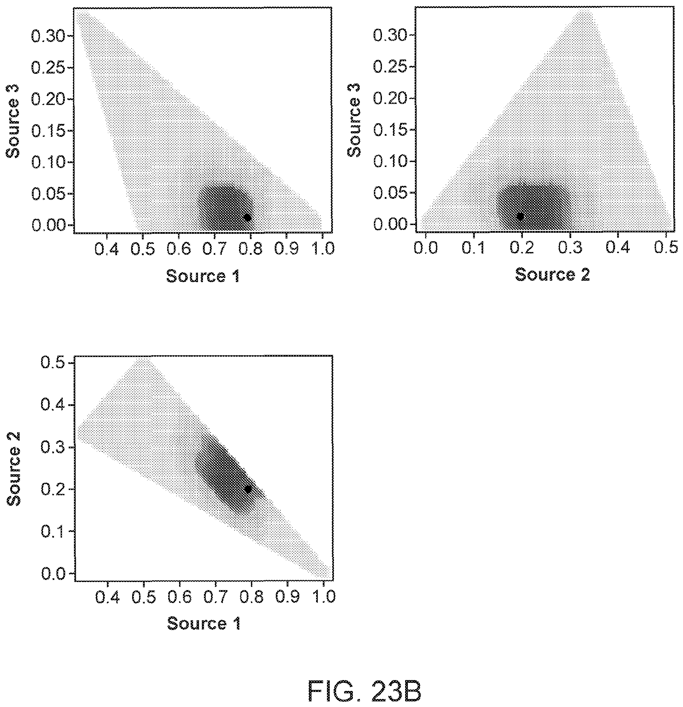

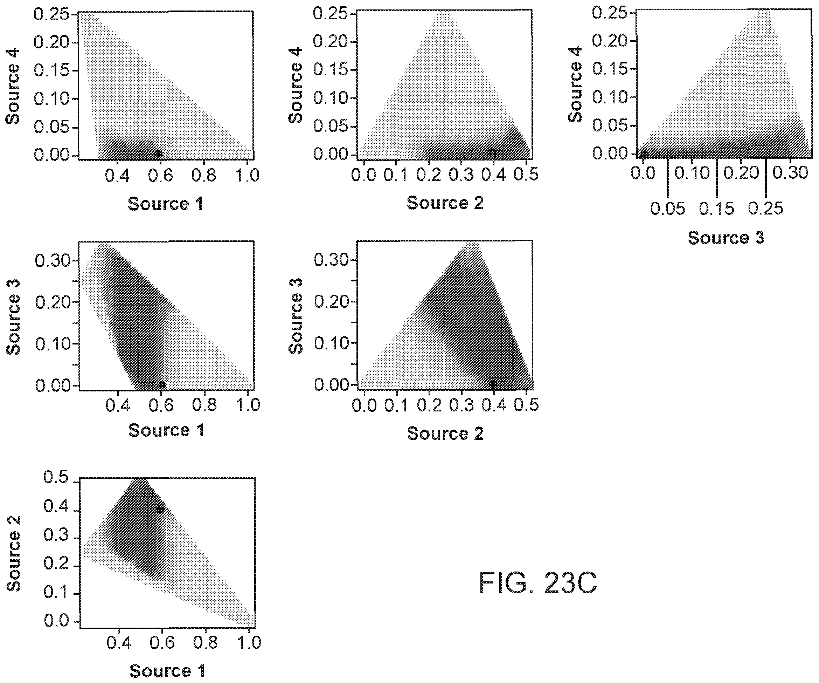

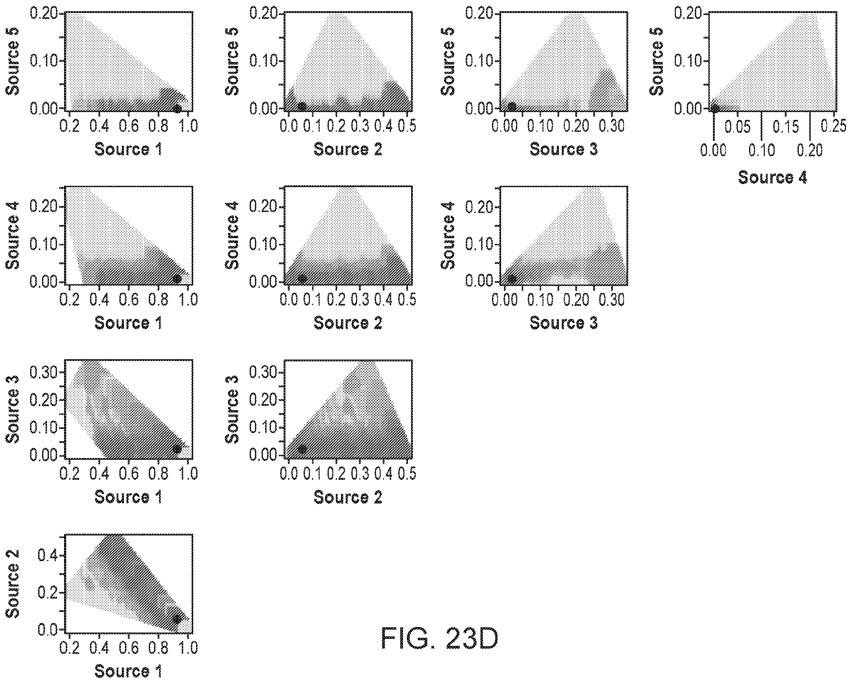

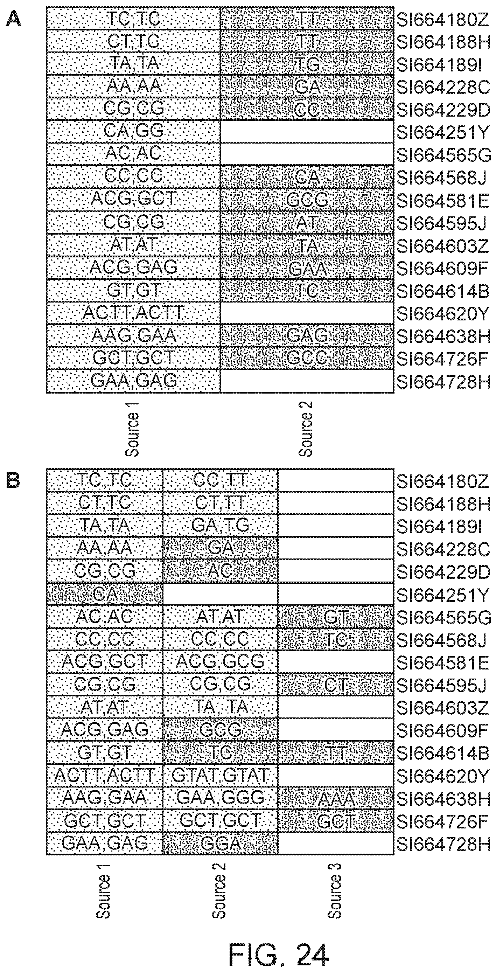

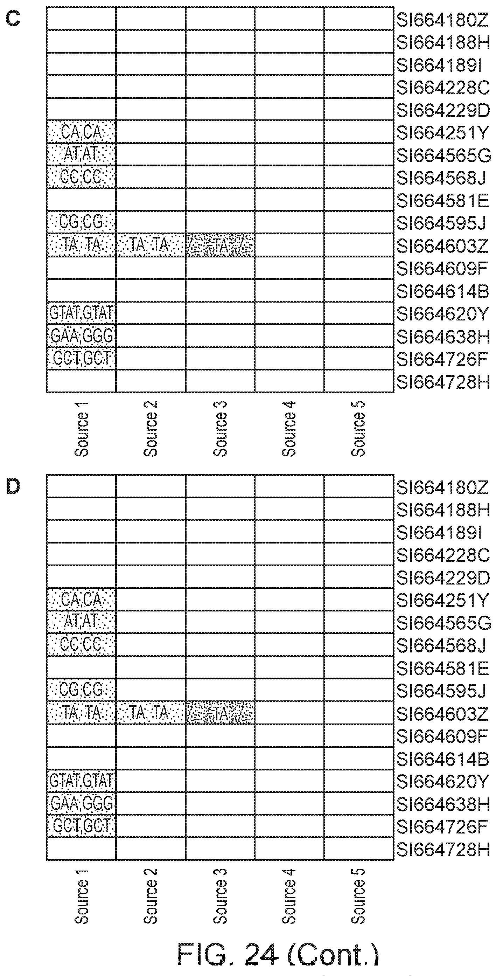

Referring to FIGS **23A-23D** (corresponding to FIG. **21,** panels A-D, respectively), Duplex Sequencing methods demonstrated the ability to estimate mixed proportions when the genotypes and potential number of sources for each sample was unknown in advance. For example, FIGS. **23A-23D** are heat map graphs demonstrating the likelihood and abundance of each source determined in the sample using Duplex sequencing. As demonstrated in FIG. **24,** panels A-D (corresponding to FIGS. **23A-23D,** respectively), microhaplotype alleles could be determined for multiple sources even when the genotypes of the sources were not previously known (see, e.g., FIG. **24,** panel B which demonstrates reliable prediction of genotypes of three sources from the mixture of FIG. **21,** panel B).

In this example, it has been demonstrated that Duplex Sequencing methodology can be used to deconvolve a biological mixture (e.g., a mixture of nucleic acid samples from 5 individuals/sources) using microhaplotypes. It was demonstrated that Duplex Sequencing methods provide sensitivity down to very low frequency alleles and can detect rare variants in complex mixtures.

In this example, Duplex Sequencing demonstrates to be a successful method for deconvolving mixtures of nucleic acid material and identify presence as well as abundance of the original DNA sources. Accordingly, Duplex Sequencing methodology provides a rapid, quantitative, and automatable way of cost-effectively identifying and assessing the abundance of multi-individual samples.

*Example 7*

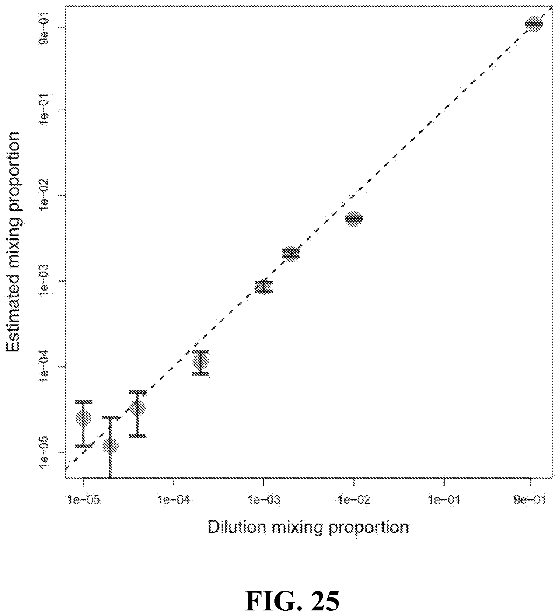

Performance evaluation to determine sensitivity to detect genotypes present at 1/100,000 abundance. In this example, an 8-sample mixture was sequenced using Duplex Sequencing methods to estimate mixing proportions and with using a small SNV panel. FIG. **25** is a scatter plot comparing actual mixing proportions of the samples against the estimates of abundance of the samples in the mixture. Points show the estimated vs. expected mixing proportions of each sample for all 8 samples, and lines show 95% confidence intervals. The dotted line shows the identity. As shown, Duplex sequencing demonstrates sensitivity and accuracy down to a 1/100,000 abundance.

*Example 8*

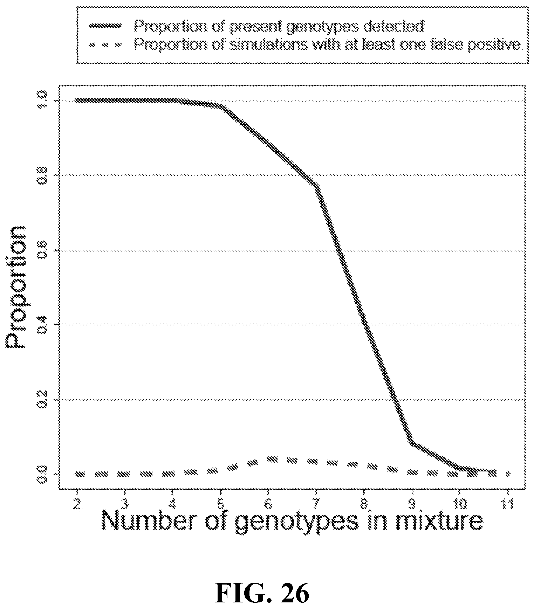

Performance evaluation to determine ability to detect genotypes present in a database. In this example, sample mixtures were sequenced using Duplex Sequencing methods to estimate mixing proportions and genotypes of original sources. FIG. **26** is a line graph plotting proportions of genotypes present in the mixture that are detected (solid line) and proportion of simulated mixtures that generate at least one false positive (dashed line). In this example, microhaplotype data from 1000 simulated mixtures of 2-10 sources was queried against a genotype database of 1000 sources and 100 microhaplotypes with 4 alleles each. Each of the 2-10 different sources were from the queried database. Results of this example, yielded identification of most subjects in mixtures of up to 8 sources with a false positive rate of 1-5%.

*Example 9*

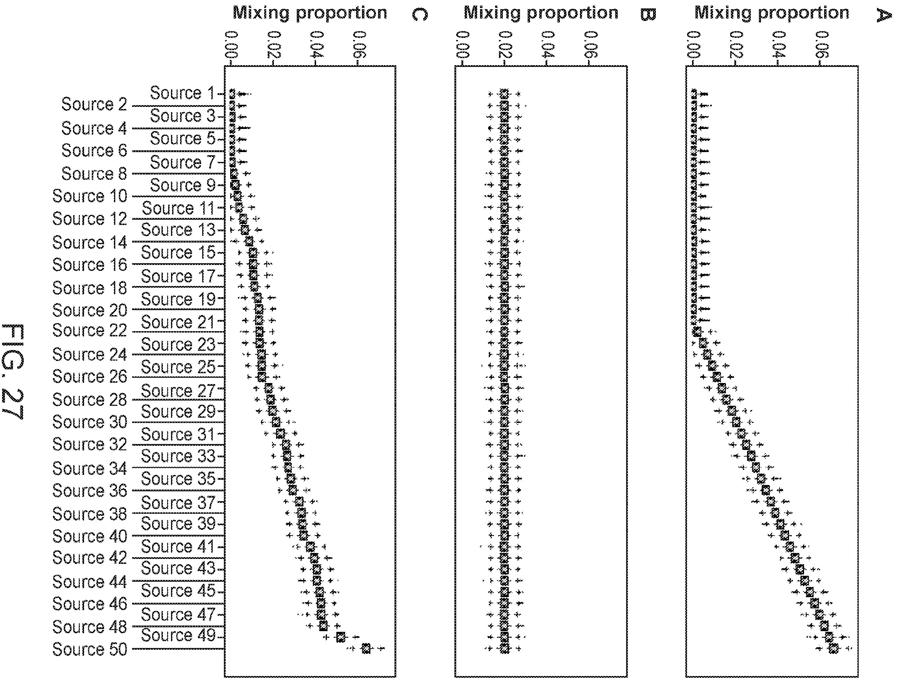

Performance evaluation to determine accurate quantification in mixtures comprising multiple genotypes. In this example, sample mixtures comprising 50 different genotypes were sequenced using Duplex Sequencing methods to estimate mixing proportions of the different genotypes. FIG. **27,** panels A-C are line plots showing estimated versus true mixing proportions in 3 different simulated mixtures of 50 genotypes. Black dots are true proportions; grey boxes/whiskers and points are inner quartiles and extreme outliers of estimates in 1000 simulations. FIG. **27,** panels A-C demonstrate that at least 50 different genotypes in a complex mixture can be accurately distinguished and quantified using Duplex Sequencing.

VI. Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments. All references cited herein are incorporated by reference as if fully set forth herein.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

The present application and invention further includes the subject matter of the following numbered clauses:

1. A method for detecting and/or quantifying a donor source of nucleic acid from a mixture, comprising:

providing the mixture comprising target double-stranded DNA molecules from one or more donor sources, wherein the target double-stranded DNA molecules contain one or more genetic polymorphisms;
generating an error-corrected sequence read for each of a plurality of the target double-stranded DNA molecules in the mixture, comprising:

ligating adapter molecules to the plurality of target double-stranded DNA fragments to generate a plurality of adapter-DNA molecules;
generating a set of copies of an original first strand of the adapter-DNA molecule and a set of copies of an original second strand of the adapter-DNA molecule;
sequencing one or more copies of the original first and second strands to provide a first strand sequence and a second strand sequence; and
comparing the first strand sequence and the second strand sequence to identify one or more correspondences between the first and second strand sequences; and

identifying a donor source of nucleic acid present in the mixture of nucleic acid by deconvolving the error-corrected sequence reads into individual genotypes.

2. A method for detecting and/or quantifying a donor source of nucleic acid from a mixture, comprising:

generating duplex sequencing data from raw sequencing data, wherein the raw sequencing data is generated from a mixture comprising target double-stranded DNA molecules from one or more donor sources, and wherein the target double-stranded DNA molecules contain one or more genetic polymorphisms; and
identifying a donor source of nucleic acid present in the mixture of nucleic acid by deconvolving the error-corrected sequence reads into individual genotypes.

3. The method of clause 1 or clause 2, wherein one or more of the donor sources have known genotypes.

4. The method of clause 1 or clause 2, wherein one or more of the donor sources have unknown genotypes.

5. The method of clause 1 or clause 2, wherein the mixture comprises one or more unknown individual genotypes, and wherein deconvolving the error-corrected sequence reads into individual genotypes comprises:

identifying microhaplotype allele combinations present within individual target double-stranded DNA molecules that map to one or more genetic loci in a reference sequence;
evaluating all possible mixing proportions against all possible genotypes present at each genetic locus within the one or more genetic loci; and
determining a list of all possible individual genotypes that adequately fit the identified microhaplotype allele combinations and all possible mixing proportions evaluated.

6. The method of clause 1 or clause 2, wherein the mixture comprises one or more known individual genotypes, and wherein deconvolving the error-corrected sequence reads into individual genotypes comprises:

identifying microhaplotype allele combinations present within individual target double-stranded DNA molecules in the mixture;
summing total counts of each allele donated from each known individual genotype; and
determining a mixing proportion of each known genotype present in the mixture.

7. The method of any of clauses 1-6, further comprising comparing one or more individual genotypes to a database comprising a plurality of known genotypes to identify the one or more donor sources.

8. The method of any one of clauses 1-7, wherein the mixture comprises more than one donor source, and wherein the method further comprises determining the proportion of each donor source from the more than one donor sources present in the mixture by calculating the proportion of each genetic polymorphism or the proportion of a substantially unique combination of genetic polymorphisms present in the error-corrected sequence reads.

9. The method of any one of clauses 1-3 and 6-8, wherein the target double-stranded DNA molecules were extracted from one or more cord blood samples.

10. The method of any one of clauses 1-8, wherein the target double-stranded DNA molecules were extracted from a forensic sample.

11. The method of any one of clauses 1-3 and 6-8, wherein the target double-stranded DNA molecules were extracted from a patient with a stem cell or organ transplant.

12. The method of any one of clauses 1-8, wherein the target double-stranded DNA molecules were extracted from a patient, and wherein identifying the one or more donor sources present in the mixture includes measuring a level of microchimerism in the patient.

13. The method of any one of clauses 1-8, wherein the target double-stranded DNA molecules were extracted from a tumor sample.

14. The method of any one of clauses 1-13, further comprising quantifying a relative abundance of each individual genotype present in the mixture.

15. The method of any one of clauses 1-14, wherein the one or more genetic polymorphisms comprise a microhaplotype.

16. The method of any one of clauses 1 and 3-15, wherein generating an error-corrected sequence read for each of a plurality of the target double-stranded DNA molecules in the mixture further comprises selectively enriching one or more targeted genomic regions prior to sequencing.

17. The method of clause 16, wherein the one or more targeted genomic regions comprises a microhaplotype site in the genome.

18. The method of any one of clause 2-15, wherein the target double-stranded DNA molecules in the mixture are selectively enriched for one or more targeted genomic regions prior to generating raw sequencing data.

19. The method of clause 18, wherein the one or more targeted genomic regions comprises a microhaplotype site in the genome.

20. A system for detecting and/or quantifying a donor source of nucleic acid from a mixture, comprising:

a computer network for transmitting information relating to sequencing data and genotype data, wherein the information includes one or more of raw sequencing data, duplex sequencing data, sample information, and genotype information;
a client computer associated with one or more user computing devices and in communication with the computer network;
a database connected to the computer network for storing a plurality of genotype profiles and user results records;
a duplex sequencing module in communication with the computer network and configured to receive raw sequencing data and requests from the client computer for generating duplex sequencing data, group sequence reads from families representing an original double-stranded nucleic acid molecule and compare representative sequences from individual strands to each other to generate duplex sequencing data; and
a genotype module in communication with the computer network and configured to identify microhaplotype alleles and calculate relative abundance of the donor source to generate genotype data.

21. The system of clause 20, wherein the genotype profiles comprise microhaplotype and/or single nucleotide polymorphism (SNP) information from a plurality of known donor sources.

22. A non-transitory computer-readable storage medium comprising instructions that, when executed by one or more processors, performs a method of any one of clauses 2-15 and 18-19.

23. The non-transitory computer-readable storage medium of clause 22, further comprising instructions for computing a mixing proportion of each identified donor source.

24. A computer system for performing a method of any one of clauses 2-15 and 18-19 for detecting and/or quantifying a donor source of nucleic acid from a mixture, the system comprising: at least one computer with a processor, memory, database, and a non-transitory computer readable storage medium comprising instructions for the processor(s), wherein said processor(s) are configured to execute said instructions to perform operations comprising the methods of any one of clauses 2-15 and 18-19.

25. A non-transitory computer-readable medium whose contents cause at least one computer to perform a method for providing duplex sequencing data for double-stranded nucleic acid molecules in a sample comprising a mixture of donor source material, the method comprising:

receiving raw sequence data from a user computing device;
creating a sample-specific data set comprising a plurality of raw sequence reads derived from a plurality of nucleic acid molecules in the sample;
grouping sequence reads from families representing an original double-stranded nucleic acid molecule, wherein the grouping is based on a shared single molecule identifier sequence;
comparing a first strand sequence read and a second strand sequence read from an original double-stranded nucleic acid molecule to identify one or more correspondences between the first and second strand sequences reads;
providing duplex sequencing data for the double-stranded nucleic acid molecules in the sample; and
identifying microhaplotype allele combinations present within individual double-stranded nucleic acid molecules in the sample to identify one or more donor sources in the mixture.

26. The computer-readable medium of clause 25, further comprising computing a mixing proportion of each identified donor source.

27. The computer-readable medium of clause 25 or clause 26, further comprising identifying nucleotide positions of non-complementarity between the compared first and second sequence reads, wherein the method further comprises, in positions of non-complementarity, identifying and eliminating or discounting process errors.

28. A non-transitory computer-readable medium whose contents cause at least one computer to perform a method for detecting, identifying and quantifying microhaplotypes present in nucleic acid mixtures to determine known source genotypes a sample, the method comprising:

identifying microhaplotype allele combinations present within individual DNA molecules in a mixture;
summing total counts of each allele donated from each known source genotype; and
determining a mixing proportion of each genotype present in the mixture.

29. The computer-readable medium of clause 28, wherein calculating a mixing proportion includes using a regression-based model.

30. The computer-readable medium of clause 28 or clause 29, further comprising comparing the determined mixing proportion of each genotype with an original mixing proportion.

31. A non-transitory computer-readable medium whose contents cause at least one computer to perform a method for deconvolving nucleic acid mixtures of unknown genotypes in a sample, the method comprising:

identifying microhaplotype allele combinations present within individual DNA molecules in a mixture;
evaluating all possible mixing proportions against all possible genotypes present at each genetic locus; and
determining a list of all possible genotypes that adequately fit the identified microhaplotype allele combinations and all possible mixing proportions evaluated.

32. The computer-readable medium of clause 31, further comprising comparing the possible genotypes from the unknown genotypes in the sample to a database comprising genotype profiles of known sources to identify a donor source.

**Claims**

1. A method for detecting and quantifying a donor source of nucleic acid from a mixture, comprising:

providing the mixture comprising target double-stranded DNA molecules from more than one donor sources, wherein the target double-stranded DNA molecules contain one or more genetic polymorphisms;
generating an error-corrected sequence read for each of a plurality of the target double-stranded DNA molecules in the mixture, comprising: ligating adapter molecules to the plurality of target double-stranded DNA

fragments to generate a plurality of adapter-DNA molecules;
generating a set of copies of an original first strand of the adapter-DNA molecule and a set of copies of an original second strand of the adapter-DNA molecule;
sequencing one or more copies of the original first and second strands to provide a first strand sequence and a second strand sequence; and
comparing the first strand sequence and the second strand sequence to identify one or more correspondences between the first and second strand sequences; and

identifying a donor source of nucleic acid present in the mixture of nucleic acid by deconvolving the error-corrected sequence reads into individual genotypes.

2. A method for detecting and quantifying a donor source of nucleic acid from a mixture, comprising:

generating duplex sequencing data from raw sequencing data, wherein the raw sequencing data is generated from a mixture comprising target double-stranded DNA molecules from more than one donor source, and wherein the target double-stranded DNA molecules contain one or more genetic polymorphisms; and
identifying a donor source of nucleic acid present in the mixture of nucleic acid by deconvolving the error-corrected sequence reads into individual genotypes.

3. The method of claim 1 or claim 2, wherein one or more of the donor sources have known genotypes.

4. The method of claim 1 or claim 2, wherein one or more of the donor sources have unknown genotypes.

5. The method of claim 1 or claim 2, wherein the mixture comprises one or more unknown individual genotypes, and wherein deconvolving the error-corrected sequence reads into individual genotypes comprises:

   identifying microhaplotype allele combinations present within individual target double-stranded DNA molecules that map to one or more genetic loci in a reference sequence;
   evaluating all possible mixing proportions against all possible genotypes present at each genetic locus within the one or more genetic loci; and
   determining a list of all possible individual genotypes that adequately fit the identified microhaplotype allele combinations and all possible mixing proportions evaluated.

6. The method of claim 1 or claim 2, wherein the mixture comprises one or more known individual genotypes, and wherein deconvolving the error-corrected sequence reads into individual genotypes comprises:

   identifying microhaplotype allele combinations present within individual target double-stranded DNA molecules in the mixture;
   summing total counts of each allele donated from each known individual genotype; and
   determining a mixing proportion of each known genotype present in the mixture.

7. The method of any of claims 1-6, further comprising comparing one or more individual genotypes to a database comprising a plurality of known genotypes to identify one or more donor sources.

8. The method of any one of claims 1-7, wherein the method further comprises determining the proportion of each donor source from the more than one donor sources present in the mixture by calculating the proportion of each genetic polymorphism or the proportion of a substantially unique combination of genetic polymorphisms present in the error-corrected sequence reads.

9. The method of any one of claims 1-3 and 6-8, wherein the target double-stranded DNA molecules were extracted from one or more cord blood samples.

10. The method of any one of claims 1-8, wherein the target double-stranded DNA molecules were extracted from a forensic sample or a tumor sample.

11. The method of any one of claims 1-3 and 6-8, wherein the target double-stranded DNA molecules were extracted from a patient with a stem cell or organ transplant.

12. The method of any one of claims 1-8, wherein the target double-stranded DNA molecules were extracted from a patient, and wherein identifying the one or more donor sources present in the mixture includes measuring a level of microchimerism in the patient.

13. The method of any one of claims 1-12, further comprising quantifying a relative abundance of each individual genotype present in the mixture.

14. The method of any one of claims 1 and 3-13, wherein generating an error-corrected sequence read for each of a plurality of the target double-stranded DNA molecules in the mixture further comprises selectively enriching one or more targeted genomic regions prior to sequencing.

15. The method of any one of claims 2-13, wherein the target double-stranded DNA molecules in the mixture are selectively enriched for one or more targeted genomic regions prior to generating raw sequencing data.

**FIG. 1**
**Prior Art**

FIG. 2
Prior Art

**FIG. 3**
**Prior Art**

FIG. 4A

Top and bottom strands amplified
and sequenced. Amplicons are
grouped by unique tag sequence
and strand

Optionally,
errors removed by
comparing the
sequences of
duplicates

Representative sequences
from individual strands
are compared to
eliminate remaining errors

Starting DNA molecule

FIG. 4B

FIG. 4C

FIG. 5

_600

```
        ┌─────────┐
        │  Start  │────602
        └─────────┘
             │
             ▼
┌─────────────────────────┐
│      Receive data       │────604
└─────────────────────────┘
             │
             ▼
┌─────────────────────────┐
│  Create a sample-specific │
│       data set          │────606
└─────────────────────────┘
             │
             ▼
┌─────────────────────────┐
│  Receive request to generate │
│   duplex sequence data  │────608
└─────────────────────────┘
             │
             ▼
┌─────────────────────────┐
│  Compare a first strand sequence │
│   read to a second strand │
│     sequence read       │────610
└─────────────────────────┘
             │
             ▼
┌─────────────────────────┐
│  Identify nucleotide positions │
│    of complementarity   │────612
└─────────────────────────┘
             │
             ▼
┌─────────────────────────┐
│  Identify nucleotide positions │
│  of non-complementarity │────614
└─────────────────────────┘
             │
             ▼
┌─────────────────────────┐
│  Provide duplex sequencing data │────616
└─────────────────────────┘
             │
             ▼
        ┌─────────┐
        │   End   │────618
        └─────────┘
```

FIG. 6

EP 4 775 690 A2

FIG. 7

47

800

Start — 802

↓

Identify microhaplotype alleles — 804

↓

Evaluate possible mixing proportions — 806

↓

Calculate list of all possible genotypes that fit data — 808

↓

Compare genotype profile from unknown source to genotype profiles from known sources (optional) — 810

↓

Provide genotype data — 812

↓

End — 814

FIG. 8

Y

X

B

| Locus | Allele | Counts in mixture |
|---|---|---|
| microhap1 | AAT | 1300 |
| microhap1 | ATG | 10 |
| microhap1 | CTT | 300 |
| microhap1 | CTG | 390 |
| microhap2 | GG | 690 |
| microhap2 | GT | 1200 |
| microhap2 | AG | 100 |
| ... | ... | |

| Allele | Source 1 copies | Source 2 copies | Source 3 copies | Source 4 copies |
|---|---|---|---|---|
| AAT | 1 | 0 | 1 | 1 |
| ATG | 0 | 0 | 0 | 1 |
| CTT | 0 | 1 | 0 | 0 |
| CTG | 0 | 1 | 1 | 0 |
| GG | 1 | 0 | 1 | 0 |
| GT | 1 | 2 | 0 | 0 |
| AG | 0 | 0 | 1 | 2 |
| ... | ... | ... | ... | ... |

| | Mixing proportion |
|---|---|
| Source 1 | 0.6 |
| Source 2 | 0.3 |
| Source 3 | 0.09 |
| Source 4 | 0.01 |

Counts over all microhaps

Known source genotypes

Mixing proportions

**FIG. 9**

**FIG. 10**

Sequence each cord

Quantify CD 34+ in each cord

Blinded mixes

Notch expand

Sequence and deconvolve

Sequence and deconvolve

FIG. 11

Global MAF Distribution of SNP Panel

SNP minor allele Frequency (MAF)

14 "Bonus" SNPs (0.1-17% global MAF)

45 SNPs Targeted by Panel (30-50% global MAF)

**FIG. 12**

FIG. 13

EP 4 775 690 A2

| SNP | Cord Blood Sample | | | | | | | |
|---|---|---|---|---|---|---|---|---|
| | 1 | 2 | 3 | 4 | 5 | 6 | 7 | 8 |
| rs993934 | | | | | | | | |
| rs279844 | | | | | | | | |
| rs142362769 | | | | | | | | |
| rs12437775 | | | | | | | | |
| rs17793354 | | | | | | | | |
| rs16991914 | | | | | | | | |
| rs117776421 | | | | | | | | |
| rs919450328 | | | | | | | | |
| rs73756355 | | | | | | | | |
| rs116518682 | | | | | | | | |
| rs58774517 | | | | | | | | |

Genotype: AA    Aa    aa

# FIG. 14

**FIG. 15A-B**

**C** Mix #5

**D** Mix #6

Quantified by Nanodrop

Duplex Sequencing
Quantified Mixture

**FIG. 15C-D**

Nanograms of DNA

Sample

Quantified by Nanodrop
Duplex Sequencing
Quantified Mixture

**FIG. 15E**

FIG. 16

EP 4 775 690 A2

**FIG. 17**

Microhap spans: after repeat masker subtraction

FIG. 18A

| Africa | Mbuti(SA000006G, 78, 12/21/2016) ⊞ | |
|---|---|---|
| Africa | Yoruba(SA000036J, 152, 12/21/2016) ⊞ | |
| Africa | Yoruba(SA004048Q, 216, 12/21/2016) ⊞ | |
| Africa | Chagga(SA000487I, 86, 12/21/2016) ⊞ | |
| Africa | Jews, Ethiopian(SA000015G, 56, 12/21/2016) ⊞ | |
| Africa | Luhya(SA004046Q, 198, 12/21/2016) ⊞ | |
| Africa | Masai(SA000854R, 40, 12/21/2016) ⊞ | |
| Africa | Sandawe(SA001773S, 80, 12/21/2016) ⊞ | |
| Africa | Somali(SA002138O, 18, 7/7/2017) ⊞ | |
| Africa | Zaramo(SA002586V, 64, 12/21/2016) ⊞ | |
| Africa | African Americans(SA000101C, 176, 12/21/2016) ⊞ | |
| Africa | African Americans(SA004047P, 122, 12/21/2016) ⊞ | |
| Africa | Mende(SA004244O, 170, 12/21/2016) ⊞ | |
| Asia | Druze(SA000047L, 244, 12/21/2016) ⊞ | |
| Asia | Iranian(SA004309Q, 68, 7/7/2017) ⊞ | |
| Asia | Jews, Yemenite(SA000016H, 80, 12/21/2016) ⊞ | |
| Asia | Kuwaiti(SA002765U, 24, 12/21/2016) ⊞ | |
| Asia | Mohanna(SA002139P, 56, 7/7/2017) ⊞ | |
| Asia | Negroid Makrani(SA002137N, 20, 7/7/2017) ⊞ | |
| Asia | Palestinian(SA002766V, 114, 7/7/2017) ⊞ | |
| Asia | Samaritans(SA00009SR, 76, 12/21/2016) ⊞ | |
| Asia | Saudi(SA004393T, 138, 7/7/2017) ⊞ | |

**FIG. 18B**

**FIG. 19**

EP 4 775 690 A2

# Y

| SNP | MAF in mixture |
|-----|------|
| rs1 | 0.12 |
| rs2 | 0.41 |
| rs3 | 0.64 |
| rs4 | 0.15 |
| rs5 | 0 |
| rs6 | 0.01 |
| rs7 | 0.4 |

=

# X

| SNP | Minor allele freq in donor 1 | Minor allele freq in donor 2 | Minor allele freq in donor 3 | Minor allele freq in donor 4 | Minor allele freq in donor 5 |
|-----|-----|-----|-----|-----|-----|
| rs1 | 0 | 0 | 1 | 0 | 2 |
| rs2 | 0 | 2 | 0 | 0 | 1 |
| rs3 | 1 | 0 | 0 | 0 | 0 |
| rs4 | 0 | 0 | 1 | 1 | 0 |
| rs5 | 0 | 0 | 0 | 0 | 0 |
| rs6 | 0 | 0 | 0 | 0 | 1 |
| rs7 | 0 | 1 | 2 | 0 | 0 |

$*$

# β

| | Mixing prop |
|---|---|
| Donor 1 | 0.64 |
| Donor 2 | 0.2 |
| Donor 3 | 0.1 |
| Donor 4 | 0.05 |
| Donor 5 | 0.01 |

**FIG. 20**

FIG. 21

FIG. 22

FIG. 23A

FIG. 23B

FIG. 23C

FIG. 23D

**A**

| Source 1 | Source 2 | |
|---|---|---|
| TC,TC | TT | SI664180Z |
| CT,TC | TT | SI664188H |
| TA,TA | TG | SI664189I |
| AA,AA | GA | SI664228C |
| CG,CG | CC | SI664229D |
| CA,GG | | SI664251Y |
| AC,AC | | SI664565G |
| CC,CC | CA | SI664568J |
| ACG,GCT | GCG | SI664581E |
| CG,CG | AT | SI664595J |
| AT,AT | TA | SI664603Z |
| ACG,GAG | GAA | SI664609F |
| GT,GT | TC | SI664614B |
| ACTT,ACTT | | SI664620Y |
| AAG,GAA | GAG | SI664638H |
| GCT,GCT | GCC | SI664726F |
| GAA,GAG | | SI664728H |

**B**

| Source 1 | Source 2 | Source 3 | |
|---|---|---|---|
| TC,TC | CC,TT | | SI664180Z |
| CT,TC | CT,TT | | SI664188H |
| TA,TA | GA,TG | | SI664189I |
| AA,AA | GA | | SI664228C |
| CG,CG | AC | | SI664229D |
| CA | | | SI664251Y |
| AC,AC | AT,AT | GT | SI664565G |
| CC,CC | CC,CC | TC | SI664568J |
| ACG,GCT | ACG,GCG | | SI664581E |
| CG,CG | CG,CG | CT | SI664595J |
| AT,AT | TA,TA | | SI664603Z |
| ACG,GAG | GCG | | SI664609F |
| GT,GT | TC | TT | SI664614B |
| ACTT,ACTT | GTAT,GTAT | | SI664620Y |
| AAG,GAA | GAA,GGG | AAA | SI664638H |
| GCT,GCT | GCT,GCT | GCT | SI664726F |
| GAA,GAG | GGA | | SI664728H |

# FIG. 24

**C**

| Source 1 | Source 2 | Source 3 | Source 4 | Source 5 | |
|---|---|---|---|---|---|
| | | | | | SI664180Z |
| | | | | | SI664188H |
| | | | | | SI664189I |
| | | | | | SI664228C |
| | | | | | SI664229D |
| CA,CA | | | | | SI664251Y |
| AT,AT | | | | | SI664565G |
| CC,CC | | | | | SI664568J |
| | | | | | SI664581E |
| CG,CG | | | | | SI664595J |
| TA,TA | TA,TA | TA | | | SI664603Z |
| | | | | | SI664609F |
| | | | | | SI664614B |
| GTAT,GTAT | | | | | SI664620Y |
| GAA,GGG | | | | | SI664638H |
| GCT,GCT | | | | | SI664726F |
| | | | | | SI664728H |

**D**

| Source 1 | Source 2 | Source 3 | Source 4 | Source 5 | |
|---|---|---|---|---|---|
| | | | | | SI664180Z |
| | | | | | SI664188H |
| | | | | | SI664189I |
| | | | | | SI664228C |
| | | | | | SI664229D |
| CA,CA | | | | | SI664251Y |
| AT,AT | | | | | SI664565G |
| CC,CC | | | | | SI664568J |
| | | | | | SI664581E |
| CG,CG | | | | | SI664595J |
| TA,TA | TA,TA | TA | | | SI664603Z |
| | | | | | SI664609F |
| | | | | | SI664614B |
| GTAT,GTAT | | | | | SI664620Y |
| GAA,GGG | | | | | SI664638H |
| GCT,GCT | | | | | SI664726F |
| | | | | | SI664728H |

FIG. 24 (Cont.)

**FIG. 25**

**FIG. 26**

FIG. 27

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62672573
- US 62811517
- WO 2017100441 A
- WO 2013142389 A
- US 9752188 B
- US 1859908 W